# EUROPEAN PATENT SPECIFICATION

(11) **EP 2 225 377 B1**
(45) Date of publication and mention of the grant of the patent: **08.01.2014**
(21) Application number: 08855697.2
(22) Date of filing: 26.11.2008
(51) Int. Cl.: C12N 15/113

(54) **LNA ANTAGONISTS TARGETING THE ANDROGEN RECEPTOR**
LNA-ANTAGONISTEN MIT ANDROGENREZEPTOR ALS ZIEL
ANTAGONISTES BASÉS SUR LES LNA CIBLANT LE RÉCEPTEUR DE L'ANDROGÈNE

(30) Priority: 26.11.2007 US 990125 P
(43) Date of publication of application: 08.09.2010
(73) Proprietor: Santaris Pharma A/S, 2970 Hørsholm (DK)
(72) Inventor: WORM, Jesper, DK-1655 Copenhagen V (DK)
(74) Representative: Wroe, Stephanie
(86) International application number: PCT/DK2008/000417
(87) International publication number: WO 2009/068033

(56) References cited:
- WO-A-2005/027833
- US-A1- 2005 159 376
- BEANE R L ET AL: "Inhibiting gene expression with locked nucleic acids (LNAs) that target chromosomal DNA" BIOCHEMISTRY 20070626 AMERICAN CHEMICAL SOCIETY US, vol. 46, no. 25, 26 June 2007 (2007-06-26), pages 7572-7580, XP002517552
- EDER I E ET AL: "Inhibition of LNCaP prostate cancer cells by means of androgen receptor antisense oligonucleotides" CANCER GENE THERAPY, NORWALK, CT, US, vol. 7, no. 7, 1 January 2000 (2000-01-01), pages 997-1007, XP002313666 ISSN: 0929-1903
- KURRECK J ET AL: "Design of antisense oligonucleotides stabilized by locked nucleic acids" NUCLEIC ACIDS RESEARCH, OXFORD UNIVERSITY PRESS, SURREY, GB, vol. 30, no. 9, 1 May 2002 (2002-05-01), pages 1911-1918, XP002281375 ISSN: 0305-1048

### SEQUENCE LISTING

<110> Santaris Pharma A/S
<120> LNA ANTAGONISTS TARGETING THE ANDROGEN RECEPTOR
<130> 1043WO
<150> US 60/990125
   <151> 2007-11-26
<160> 106
<170> PatentIn version 3.5
<210> 1
   <211> 4314
   <212> DNA
   <213> homo sapiens
<400> 1
   cgagatcccg gggagccagc ttgctgggag agcgggacgg tccggagcaa gcccagaggc      60
   agaggaggcg acagagggaa aaagggccga gctagccgct ccagtgctgt acaggagccg     120
   aagggacgca ccacgccagc cccagcccgg ctccagcgac agccaacgcc tcttgcagcg     180
   cggcggcttc gaagccgccg cccggagctg ccctttcctc ttcggtgaag tttttaaaag     240
   ctgctaaaga ctcggaggaa gcaaggaaag tgcctggtag gactgacggc tgcctttgtc     300
   ctcctcctct ccaccccgcc tccccccacc ctgccttccc cccctccccc gtcttctctc     360
   ccgcagctgc ctcagtcggc tactctcagc caacccccct caccaccctt ctccccaccc    420
   gcccccccgc ccccgtcggc ccagcgctgc cagcccgagt ttgcagagag gtaactccct    480
   ttggctgcga gcgggcgagc tagctgcaca ttgcaaagaa ggctcttagg agccaggcga     540
   ctggggagcg gcttcagcac tgcagccacg acccgcctgg ttaggctgca cgcggagaga     600
   accctctgtt ttcccccact ctctctccac ctcctcctgc cttccccacc ccgagtgcgg     660
   agccagagat caaaagatga aaaggcagtc aggtcttcag tagccaaaaa acaaaacaaa     720
   caaaaacaaa aaagccgaaa taaaagaaaa agataataac tcagttctta tttgcaccta     780
   cttcagtgga cactgaattt ggaaggtgga ggattttgtt tttttctttt aagatctggg     840
   catcttttga atctaccctt caagtattaa gagacagact gtgagcctag cagggcagat     900
   cttgtccacc gtgtgtcttc ttctgcacga gactttgagg ctgtcagagc gctttttgcg     960
   tggttgctcc cgcaagtttc cttctctgga gcttcccgca ggtgggcagc tagctgcagc    1020
   gactaccgca tcatcacagc ctgttgaact cttctgagca agagaagggg aggcggggta    1080
   agggaagtag gtggaagatt cagccaagct caaggatgga agtgcagtta gggctgggaa    1140
   gggtctaccc tcggccgccg tccaagacct accgaggagc tttccagaat ctgttccaga    1200
   gcgtgcgcga agtgatccag aacccgggcc ccaggcaccc agaggccgcg agcgcagcac    1260
   ctcccggcgc cagtttgctg ctgctgcagc agcagcagca gcagcagcag cagcagcagc    1320
   agcagcagca gcagcagcag cagcagcagc agcaagagac tagccccagg cagcagcagc    1380
   agcagcaggg tgaggatggt tctccccaag cccatcgtag aggccccaca ggctacctgg    1440
   tcctggatga ggaacagcaa ccttcacagc cgcagtcggc cctggagtgc caccccgaga    1500
   gaggttgcgt cccagagcct ggagccgccg tggccgccag caaggggctg ccgcagcagc    1560
   tgccagcacc tccggacgag gatgactcag ctgccccatc cacgttgtcc ctgctgggcc    1620
   ccactttccc cggcttaagc agctgctccg ctgaccttaa agacatcctg agcgaggcca    1680
   gcaccatgca actccttcag caacagcagc aggaagcagt atccgaaggc agcagcagcg    1740
   ggagagcgag ggaggcctcg gggctcccca cttcctccaa ggacaattac ttagggggca    1800
   cttcgaccat ttctgacaac gccaaggagt tgtgtaaggc agtgtcggtg tccatgggcc    1860
   tgggtgtgga ggcgttggag catctgagtc caggggaaca gcttcggggg gattgcatgt    1920
   acgccccact tttgggagtt ccacccgctg tgcgtcccac tccttgtgcc ccattggccg    1980
   aatgcaaagg ttctctgcta gacgacagcg caggcaagag cactgaagat actgctgagt    2040
   attccccttt caagggaggt tacaccaaag ggctagaagg cgagagccta ggctgctctg    2100
   gcagcgctgc agcagggagc tccgggacac ttgaactgcc gtctaccctg tctctctaca    2160
   agtccggagc actggacgag gcagctgcgt accagagtcg cgactactac aactttccac    2220
   tggctctggc cggaccgccg ccccctccgc cgcctcccca tccccacgct cgcatcaagc    2280
   tggagaaccc gctggactac ggcagcgcct gggcggctgc ggcggcgcag tgccgctatg    2340
   gggacctggc gagcctgcat ggcgcgggtg cagcgggacc cggttctggg tcaccctcag 2400
   ccgccgcttc ctcatcctgg cacactctct tcacagccga agaaggccag ttgtatggac 2460
   cgtgtggtgg tggtgggggt ggtggcggcg gcggcggcgg cggcggcggc ggcggcggcg 2520
   gcggcggcgg cggcgaggcg ggagctgtag ccccctacgg ctacactcgg ccccctcagg 2580
   ggctggcggg ccaggaaagc gacttcaccg cacctgatgt gtggtaccct ggcggcatgg 2640
   tgagcagagt gccctatccc agtcccactt gtgtcaaaag cgaaatgggc ccctggatgg 2700
   atagctactc cggaccttac ggggacatgc gtttggagac tgccagggac catgttttgc 2760
   ccattgacta ttactttcca ccccagaaga cctgcctgat ctgtggagat gaagcttctg 2820
   ggtgtcacta tggagctctc acatgtggaa gctgcaaggt cttcttcaaa agagccgctg 2880
   aagggaaaca gaagtacctg tgcgccagca gaaatgattg cactattgat aaattccgaa 2940
   ggaaaaattg tccatcttgt cgtcttcgga aatgttatga agcagggatg actctgggag 3000
   cccggaagct gaagaaactt ggtaatctga aactacagga ggaaggagag gcttccagca 3060
   ccaccagccc cactgaggag acaacccaga agctgacagt gtcacacatt gaaggctatg 3120
   aatgtcagcc catctttctg aatgtcctgg aagccattga gccaggtgta gtgtgtgctg 3180
   gacacgacaa caaccagccc gactcctttg cagccttgct ctctagcctc aatgaactgg 3240
   gagagagaca gcttgtacac gtggtcaagt gggccaaggc cttgcctggc ttccgcaact 3300
   tacacgtgga cgaccagatg gctgtcattc agtactcctg gatggggctc atggtgtttg 3360
   ccatgggctg gcgatccttc accaatgtca actccaggat gctctacttc gcccctgatc 3420
   tggttttcaa tgagtaccgc atgcacaagt cccggatgta cagccagtgt gtccgaatga 3480
   ggcacctctc tcaagagttt ggatggctcc aaatcacccc ccaggaattc ctgtgcatga 3540
   aagcactgct actcttcagc attattccag tggatgggct gaaaaatcaa aaattctttg 3600
   atgaacttcg aatgaactac atcaaggaac tcgatcgtat cattgcatgc aaaagaaaaa 3660
   atcccacatc ctgctcaaga cgcttctacc agctcaccaa gctcctggac tccgtgcagc 3720
   ctattgcgag agagctgcat cagttcactt ttgacctgct aatcaagtca cacatggtga 3780
   gcgtggactt tccggaaatg atggcagaga tcatctctgt gcaagtgccc aagatccttt 3840
   ctgggaaagt caagcccatc tatttccaca cccagtgaag cattggaaac cctatttccc 3900
   caccccagct catgccccct ttcagatgtc ttctgcctgt tataactctg cactactcct 3960
   ctgcagtgcc ttggggaatt tcctctattg atgtacagtc tgtcatgaac atgttcctga 4020
   attctatttg ctgggctttt tttttctctt tctctccttt ctttttcttc ttccctccct 4080
   atctaaccct cccatggcac cttcagactt tgcttcccat tgtggctcct atctgtgttt 4140
   tgaatggtgt tgtatgcctt taaatctgtg atgatcctca tatggcccag tgtcaagttg 4200
   tgcttgttta cagcactact ctgtgccagc cacacaaacg tttacttatc ttatgccacg 4260
   ggaagtttag agagctaaga ttatctgggg aaatcaaaac aaaaacaagc aaac 4314
<210> 2
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 2
   gagaaccatc ctcacc 16
<210> 3
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 3
   ggaccaggta gcctgt 16
<210> 4
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 4
   cccctggact cagatg 16
<210> 5
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 5
   gcacaaggag tgggac 16
<210> 6
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 6
   gctgtgaaga gagtgt 16
<210> 7
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 7
   tttgacacaa gtggga 16
<210> 8
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 8
   gtgacaccca gaagct 16
<210> 9
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 9
   catccctgct tcataa 16
<210> 10
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 10
   accaagtttc ttcagc 16
<210> 11
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 11
   cttggcccac ttgacc 16
<210> 12
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 12
   tcctggagtt gacatt 16
<210> 13
   <211> 16
   <212> DNA
   <213> artificial;
<220>
   <221> misc_feature
   <222> (1)..(16)
   <223> nucleotide or nucleotide analogues - optionally phosphorothioate
<400> 13
   cactggctgt acatcc 16
<210> 14
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 14
   catccaaact cttgag 16
<210> 15
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 15
   gctttcatgc acagga 16
<210> 16
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 16
   gaagttcatc aaagaa 16
<210> 17
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 17
   agttccttga tgtagt 16
<210> 18
   <211> 16
   <212> DNA
   <213> artificial
<220>
   <223> LNA oligomer Sequence/oligomer Sequence motif
<400> 18
   ttgcacagag atgatc 16
<210> 19
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 19
   gatgggcttg actttc 16
<210> 20
   <211> 16
   <212> DNA
   <213> artificial
<220>
   <223> LNA oligomer Sequence/oligomer Sequence motif
<400> 20
   caggcagaag acatct 16
<210> 21
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 21
   cccaaggcac tgcaga 16
<210> 22
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 22
   gctgacattc atagcc 16
<210> 23
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 23
   gagaaccatc ctcacc 16
<210> 24
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 24
   ggaccaggta gcctgt 16
<210> 25
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 25
   cccctggact cagatg 16
<210> 26
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 26
   gcacaaggag tgggac 16
<210> 27
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 27
   gctgtgaaga gagtgt 16
<210> 28
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 28
   tttgacacaa gtggga 16
<210> 29
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 29
   gtgacaccca gaagct 16
<210> 30
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 30
   catccctgct tcataa 16
<210> 31
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 31
   accaagtttc ttcagc 16
<210> 32
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 32
   cttggcccac ttgacc 16
<210> 33
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 33
   tcctggagtt gacatt 16
<210> 34
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 34
   cactggctgt acatcc 16
<210> 35
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 35
   catccaaact cttgag 16
<210> 36
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 36
   gctttcatgc acagga 16
<210> 37
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 37
   gaagttcatc aaagaa 16
<210> 38
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 38
   agttccttga tgtagt 16
<210> 39
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 39
   ttgcacagag atgatc 16
<210> 40
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 40
   gatgggcttg actttc 16
<210> 41
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 41
   caggcagaag acatct 16
<210> 42
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 42
   cccaaggcac tgcaga 16
<210> 43
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 43
   gctgacattc atagcc 16
<210> 44
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 44
   gagaaccatc ctcacc 16
<210> 45
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 45
   ggaccaggta gcctgt 16
<210> 46
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 46
   cccctggact cagatg 16
<210> 47
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 47
   gcacaaggag tgggac 16
<210> 48
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 48
   gctgtgaaga gagtgt 16
<210> 49
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 49
   ctgtgaagag agtg 14
<210> 50
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 50
   tgtgaagaga gt 12
<210> 51
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 51
   tttgacacaa gtggga 16
<210> 52
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 52
   ttgacacaag tggg 14
<210> 53
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 53
   tgacacaagt gg 12
<210> 54
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 54
   gtgacaccca gaagct 16
<210> 55
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 55
   tgacacccag aagc 14
<210> 56
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 56
   gacacccaga ag 12
<210> 57
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 57
   catccctgct tcataa 16
<210> 58
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 58
   accaagtttc ttcagc 16
<210> 59
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 59
   ccaagtttct tcag 14
<210> 60
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 60
   caagtttctt ca 12
.<210> 61
<211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 61
   cttggcccac ttgacc 16
<210> 62
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 62
   tcctggagtt gacatt 16
<210> 63
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 63
   cactggctgt acatcc 16
<210> 64
   <211> 14
   <212> DNA
   <213> artificial
<220>
   <223> LNA oligomer Sequence/oligomer Sequence motif
<220>
   <221> misc feature
   <222> (1)..(14)
   <223> 3-9-2 LNA Gapmer - fully phosphorothioate
<400> 64
   actggctgta catc 14
<210> 65
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 65
   ctggctgtac at 12
<210> 66
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 66
   catccaaact cttgag 16
<210> 67
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 67
   gctttcatgc acagga 16
<210> 68
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 68
   gaagttcatc aaagaa 16
<210> 69
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 69
   agttccttga tgtagt 16
<210> 70
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<220>
   <221> misc_feature
   <222> (1)..(14)
   <223> 3-9-2 LNA Gapmer - fully phosphorothioate
<400> 70
   gttccttgat gtag 14
<210> 71
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 71
   ttccttgatg ta 12
<210> 72
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 72
   ttgcacagag atgatc 16
<210> 73
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 73
   gatgggcttg actttc 16
<210> 74
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 74
   atgggcttga cttt 14
<210> 75
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 75
   tgggcttgac tt 12
<210> 76
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 76
   caggcagaag acatct 16
<210> 77
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 77
   cccaaggcac tgcaga 16
<210> 78
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 78
   ccaaggcact gcag 14
<210> 79
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 79
   caaggcactg ca 12
<210> 80
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 80
   gctgacattc atagcc 16
<210> 81
   <211> 2999
   <212> DNA
   <213> Mus musculus
<400> 81
   gaattcggtg gaagctacag acaagctcaa ggatggaggt gcagttaggg ctgggaaggg      60
   tctacccacg gcccccatcc aagacctatc gaggagcgtt ccagaatctg ttccagagcg     120
   tgcgcgaagc gatccagaac ccgggcccca ggcaccctga ggccgctaac atagcacctc     180
   ccggcgcctg tttacagcag aggcaggaga ctagcccccg gcggcggcgg cggcagcagc     240
   acactgagga tggttctcct caagcccaca tcagaggccc cacaggctac ctggccctgg     300
   aggaggaaca gcagccttca cagcagcagg cagcctccga gggccaccct gagagcagct     360
   gcctccccga gcctggggcg gccaccgctc ctggcaaggg gctgccgcag cagccaccag     420
   ctcctccaga tcaggatgac tcagctgccc catccacgtt gtccctgctg ggccccactt     480
   tcccaggctt aagcagctgc tccgccgaca ttaaagacat tttgaacgag gccggcacca     540
   tgcaacttct tcagcagcag caacaacagc agcagcacca acagcagcac caacagcacc     600
   aacagcagca ggaggtaatc tccgaaggca gcagcgcaag agccagggag gccacggggg     660
   ctccctcttc ctccaaggat agttacctag ggggcaattc aaccatatct gacagtgcca     720
   aggagttgtg taaagcagtg tctgtgtcca tgggattggg tgtggaagca ttggaacatc     780
   tgagtccagg ggaacagctt cggggagact gcatgtacgc gtcgctcctg ggaggtccac     840
   ccgcggtgcg tcccactcct tgtgcgccgc tgcccgaatg caaaggtctt cccctggacg     900
   aaggcccagg caaaagcact gaagagactg ctgagtattc ctctttcaag ggaggttacg     960
   ccaaaggatt ggaaggtgag agcttggggt gctctggcag cagtgaagca ggtagctctg    1020
   ggacacttga gatcccgtcc tctctgtctc tgtataaatc tggagcacta gacgaggcag    1080
   cagcatacca gaatcgcgac tactacaact ttccgctggc tctgtccggg ccgccgcacc    1140
   ccccgccccc tacccatcca cacgcccgta tcaagctgga gaacccattg gactacggca    1200
   gcgcctgggc tgcggcggca gcgcaatgcc gctatgggga cttgggtagt ctacatggag    1260
   ggagtgtagc cgggcccagc actggatcgc ccccagccac cacctcttct tcctggcata     1320
   ctctcttcac agctgaagaa ggccaattat atgggccagg aggcgggggc ggcagcagca    1380
   gcccaagcga tgccgggcct gtagccccct atggctacac tcggccccct caggggctga      1440
   caagccagga gagtgactac tctgcctccg aagtgtggta tcctggtgga gttgtgaaca    1500
   gagtacccta tcccagtccc aattgtgtca aaagtgaaat gggaccttgg atggagaact    1560
   actccggacc ttatggggac atgcgtttgg acagtaccag ggaccatgtt ttacccatcg    1620
   actattactt tccaccccag aagacctgcc tgatctgtgg agatgaagct tctggctgtc    1680
   actacggagc tctcacttgt ggcagctgca aggtcttctt caaaagagcc gctgaaggga    1740
   aacagaagta tctatgtgcc agcagaaacg attgtaccat tgataaattt cggaggaaaa    1800
   attgcccatc ttgtcgtctc cggaaatgtt atgaagcagg gatgactctg ggagctcgta    1860
   agctgaagaa acttggaaat ctaaaactac aggaggaagg agaaaactcc aatgctggca    1920
   gccccactga ggacccatcc cagaagatga ctgtatcaca cattgaaggc tatgaatgtc    1980
   agcctatctt tcttaacgtc ctggaagcca ttgagccagg agtggtgtgt gccggacatg    2040
   acaacaacca accagattcc tttgctgcct tgttatctag cctcaatgag cttggagaga    2100
   ggcagcttgt gcatgtggtc aagtgggcca aggccttgcc tggcttccgc aacttgcatg    2160
   tggatgacca gatggcggtc attcagtatt cctggatggg actgatggta tttgccatgg    2220
   gttggcggtc cttcactaat gtcaactcca ggatgctcta ctttgcacct gacttggttt    2280
   tcaatgagta ccgcatgcac aagtctcgga tgtacagcca gtgtgtgagg atgaggcacc    2340
   tgtctcaaga gtttggatgg ctccaaataa ccccccagga attcctgtgc atgaaagcac    2400
   tgctgctctt cagcattatt ccagtggatg ggctgaaaaa tcaaaaattc tttgatgaac    2460
   ttcgaatgaa ctacatcaag gaactcgatc gcatcattgc atgcaaaaga aagaatccca    2520
   catcctgctc aaggcgcttc taccagctca ccaagctcct ggattctgtg cagcctattg    2580
   caagagagct gcatcagttc acttttgacc tgctaatcaa gtcccatatg gtgagcgtgg    2640
   actttcctga aatgatggca gagatcatct ctgtgcaagt gcccaagatc ctttctggga    2700
   aagtcaagcc catctatttc cacacacagt gaagatttgg aaaccctaat acccaaaacc    2760
   caccttgttc cctttccaga tgtcttctgc ctgttatata actctgcact acttctctgc    2820
   agtgccttgg gggaaattcc tctactgatg tacagtctgt cgtgaacagg ttcctcagtt    2880
   ctatttcctg ggcttctcct tctttttttt tcttcttccc tccctctttc accctcccat     2940
   ggcacatttt gaatctgctg cgtattgtgg ctcctgcctt tgttttgatt tctgttgta     2999
<210> 82
   <211> 3175
   <212> DNA
   <213> Macaca mulatta
<400> 82
   cccaaaaaat aaaaacaaac aaaaacaaaa caaaacaaaa aaaacgaata aagaaaaagg 60
   taataactca gttcttattt gcacctactt ccagtggaca ctgaatttgg aaggtggagg 120
   attcttgttt tttcttttaa gatcgggcat cttttgaatc tacccctcaa gtgttaagag 180
   acagactgtg agcctagcag ggcagatctt gtccaccgtg tgtcttcttt tgcaggagac 240
   tttgaggctg tcagagcgct ttttgcgtgg ttgctcccgc aagtttcctt ctctggagct 300
   tcccgcaggt gggcagctag ctgcagcgac taccgcatca tcacagcctg ttgaactctt 360
   ctgagcaaga gaaggggagg cggggtaagg gaagtaggtg gaagattcag ccaagctcaa 420
   ggatggaggt gcagttaggg ctggggaggg tctaccctcg gccgccgtcc aagacctacc 480
   gaggagcttt ccagaatctg ttccagagcg tgcgcgaagt gatccagaac ccgggcccca 540
   ggcacccaga ggccgcgagc gcagcacctc ccggcgccag tttgcagcag cagcagcagc 600
   agcagcaaga aactagcccc cggcaacagc agcagcagca gcagggtgag gatggttctc 660
   cccaagccca tcgtagaggc cccacaggct acctggtcct ggatgaggaa cagcagcctt 720
   cacagcctca gtcagccccg gagtgccacc ccgagagagg ttgcgtccca gagcctggag 780
   ccgccgtggc cgccggcaag gggctgccgc agcagctgcc agcacctccg gacgaggatg 840
   actcagctgc cccatccacg ttgtctctgc tgggccccac tttccccggc ttaagcagct 900
   gctccgccga ccttaaagac atcctgagcg aggccagcac catgcaactc cttcagcaac 960
   agcagcagga agcagtatcc gaaggcagca gcagcgggag agcgagggag gcctcggggg 1020
   ctcccacttc ctccaaggac aattacttag agggcacttc gaccatttct gacagcgcca 1080
   aggagctgtg taaggcagtg tcggtgtcca tgggcttggg tgtggaggcg ttggagcatc 1140
   tgagtccagg ggaacagctt cggggggatt gcatgtacgc cccagttttg ggagttccac 1200
   ccgctgtgcg tcccactccg tgtgccccat tggccgaatg caaaggttct ctgctagacg 1260
   acagcgcagg caagagcact gaagatactg ctgagtattc ccctttcaag ggaggttaca 1320
   ccaaagggct agaaggcgag agcctaggct gctctggcag cgctgcagca gggagctccg 1380
   ggacacttga actgccgtcc accctgtctc tctacaagtc cggagcactg gacgaggcag 1440
   ctgcgtacca gagtcgcgac tactacaact ttccactggc tctggccggg ccgccgcccc 1500
   ctccaccgcc tccccatccc cacgctcgca tcaagctgga gaacccgctg gactatggca 1560
   gcgcctgggc ggctgcggcg gcgcagtgcc gctatgggga cctggcgagc ctgcatggcg 1620
   cgggtgcagc gggacccggc tctgggtcac cctcagcggc cgcttcctca tcctggcaca 1680
   ctctcttcac agccgaagaa ggccagttgt atggaccgtg tggtggtggg ggcggcggcg 1740
   gtggcggcgg cggcggcggc gcaggcgagg cgggagctgt agccccctac ggctacactc 1800
   ggccacctca ggggctggcg ggccaggaag gcgacttcac cgcacctgat gtgtggtacc 1860
   ctggcggcat ggtgagcaga gtgccctatc ccagtcccac ttgtgtcaaa agcgagatgg 1920
   gcccctggat ggatagctac tccggacctt acggggacat gcgtttggag actgccaggg 1980
   accatgtttt gccaattgac tattactttc caccccagaa gacctgcctg atctgtggag 2040
   atgaagcttc tgggtgtcac tatggagctc tcacatgtgg aagctgcaag gtcttcttca 2100
   aaagagccgc tgaagggaaa cagaagtacc tgtgtgccag cagaaatgat tgcactattg 2160
   ataaattccg aaggaaaaat tgtccatctt gccgtcttcg gaaatgttat gaagcaggga 2220
   tgactctggg agcccggaag ctgaagaaac ttggtaatct gaaactacag gaggaaggag 2280
   aggcttccag caccaccagc cccactgagg agacagccca gaagctgaca gtgtcacaca 2340
   ttgaaggcta tgaatgtcag cccatctttc tgaatgtcct ggaggccatt gagccaggtg 2400
   tggtgtgtgc tggacatgac aacaaccagc ccgactcctt cgcagccttg ctctctagcc 2460
   tcaatgaact gggagagaga cagcttgtac atgtggtcaa gtgggccaag gccttgcctg 2520
   gcttccgcaa cttacacgtg gacgaccaga tggctgtcat tcagtactcc tggatggggc 2580
   tcatggtgtt tgccatgggc tggcgatcct tcaccaatgt caactccagg atgctctact 2640
   ttgcccctga tctggttttc aatgagtacc gcatgcacaa atcccggatg tacagccagt 2700
   gtgtccgaat gaggcacctc tctcaagagt ttggatggct ccaaatcacc ccccaggaat 2760
   tcctgtgcat gaaagcgctg ctactcttca gcattattcc agtggatggg ctgaaaaatc 2820
   aaaaattctt tgatgaactt cgaatgaact acatcaagga actcgatcgt atcattgcat 2880
   gcaaaagaaa aaatcccaca tcctgctcaa ggcgtttcta ccagctcacc aagctcctgg 2940
   actccgtgca gcctattgcg agagagctgc atcagttcac ttttgacctg ctaatcaagt 3000
   cacacatggt gagcgtggac tttccggaaa tgatggcaga gatcatctct gtgcaagtgc 3060
   ccaagatcct ttctgggaaa gtcaagccca tctatttcca cacccagtga agcattggaa 3120
   atccctattt cctcacccca gctcatgccc cctttcagat gtcttctgcc tgtta 3175
<210> 83
   <211> 920
   <212> PRT
   <213> Homo sapiens
<400> 83
<210> 84
   <211> 899
   <212> PRT
   <213> Mus musculus
<400> 84
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Ala Ile Gln Asn Pro Gly Pro Arg His Pro Glu Ala Ala Asn Ile Ala
           35                  40                  45
   Pro Pro Gly Ala Cys Leu Gln Gln Arg Gln Glu Thr Ser Pro Arg Arg
       50                  55                  60
   Arg Arg Arg Gln Gln His Thr Glu Asp Gly Ser Pro Gln Ala His Ile
   65                  70                  75                  80
   Arg Gly Pro Thr Gly Tyr Leu Ala Leu Glu Glu Glu Gln Gln Pro Ser
                   85                  90                  95
   Gln Gln Gln Ala Ala Ser Glu Gly His Pro Glu Ser Ser Cys Leu Pro
               100                 105                 110
   Glu Pro Gly Ala Ala Thr Ala Pro Gly Lys Gly Leu Pro Gln Gln Pro
           115                 120                 125
   Pro Ala Pro Pro Asp Gln Asp Asp Ser Ala Ala Pro Ser Thr Leu Ser
       130                 135                 140
   Leu Leu Gly Pro Thr Phe Pro Gly Leu Ser Ser Cys Ser Ala Asp Ile
   145                 150                 155                 160
   Lys Asp Ile Leu Asn Glu Ala Gly Thr Met Gln Leu Leu Gln Gln Gln
                   165                 170                 175
   Gln Gln Gln Gln Gln His Gln Gln Gln His Gln Gln His Gln Gln Gln
               180                 185                 190
   Gln Glu Val Ile Ser Glu Gly Ser Ser Ala Arg Ala Arg Glu Ala Thr
           195                 200                 205
   Gly Ala Pro Ser Ser Ser Lys Asp Ser Tyr Leu Gly Gly Asn Ser Thr
       210                 215                 220
   Ile Ser Asp Ser Ala Lys Glu Leu Cys Lys Ala Val Ser Val Ser Met
   225                 230                 235                 240
   Gly Leu Gly Val Glu Ala Leu Glu His Leu Ser Pro Gly Glu Gln Leu
                   245                 250                 255
   Arg Gly Asp Cys Met Tyr Ala Ser Leu Leu Gly Gly Pro Pro Ala Val
               260                 265                 270
   Arg Pro Thr Pro Cys Ala Pro Leu Pro Glu Cys Lys Gly Leu Pro Leu
           275                 280                 285
   Asp Glu Gly Pro Gly Lys Ser Thr Glu Glu Thr Ala Glu Tyr Ser Ser
       290                 295                 300
   Phe Lys Gly Gly Tyr Ala Lys Gly Leu Glu Gly Glu Ser Leu Gly Cys
   305                 310                 315                 320
   Ser Gly Ser Ser Glu Ala Gly Ser Ser Gly Thr Leu Glu Ile Pro Ser
                   325                 330                 335
   Ser Leu Ser Leu Tyr Lys Ser Gly Ala Leu Asp Glu Ala Ala Ala Tyr
               340                 345                 350
   Gln Asn Arg Asp Tyr Tyr Asn Phe Pro Leu Ala Leu Ser Gly Pro Pro
           355                 360                 365
   His Pro Pro Pro Pro Thr His Pro His Ala Arg Ile Lys Leu Glu Asn
       370                 375                 380
   Pro Leu Asp Tyr Gly Ser Ala Trp Ala Ala Ala Ala Ala Gln Cys Arg
   385                 390                 395                 400
   Tyr Gly Asp Leu Gly Ser Leu His Gly Gly Ser Val Ala Gly Pro Ser
                   405                 410                 415
   Thr Gly Ser Pro Pro Ala Thr Thr Ser Ser Ser Trp His Thr Leu Phe
               420                 425                 430
   Thr Ala Glu Glu Gly Gln Leu Tyr Gly Pro Gly Gly Gly Gly Gly Ser
           435                 440                 445
   Ser Ser Pro Ser Asp Ala Gly Pro Val Ala Pro Tyr Gly Tyr Thr Arg
       450                 455                 460
   Pro Pro Gln Gly Leu Thr Ser Gln Glu Ser Asp Tyr Ser Ala Ser Glu
   465                 470                 475                 480
   Val Trp Tyr Pro Gly Gly Val Val Asn Arg Val Pro Tyr Pro Ser Pro
                   485                 490                 495
   Asn Cys Val Lys Ser Glu Met Gly Pro Trp Met Glu Asn Tyr Ser Gly
               500                 505                 510
   Pro Tyr Gly Asp Met Arg Leu Asp Ser Thr Arg Asp His Val Leu Pro
           515                 520                 525
   Ile Asp Tyr Tyr Phe Pro Pro Gln Lys Thr Cys Leu Ile Cys Gly Asp
       530                 535                 540
   Glu Ala Ser Gly Cys His Tyr Gly Ala Leu Thr Cys Gly Ser Cys Lys
   545                 550                 555                 560
   Val Phe Phe Lys Arg Ala Ala Glu Gly Lys Gln Lys Tyr Leu Cys Ala
                   565                 570                 575
   Ser Arg Asn Asp Cys Thr Ile Asp Lys Phe Arg Arg Lys Asn Cys Pro
   580 585 590
   Ser Cys Arg Leu Arg Lys Cys Tyr Glu Ala Gly Met Thr Leu Gly Ala
   595 600 605
   Arg Lys Leu Lys Lys Leu Gly Asn Leu Lys Leu Gln Glu Glu Gly Glu
   610 615 620
   Asn Ser Asn Ala Gly Ser Pro Thr Glu Asp Pro Ser Gln Lys Met Thr
   625 630 635 640
   Val Ser His Ile Glu Gly Tyr Glu Cys Gln Pro Ile Phe Leu Asn Val
   645 650 655
   Leu Glu Ala Ile Glu Pro Gly Val Val Cys Ala Gly His Asp Asn Asn
   660 665 670
   Gln Pro Asp Ser Phe Ala Ala Leu Leu Ser Ser Leu Asn Glu Leu Gly
   675 680 685
   Glu Arg Gln Leu Val His Val Val Lys Trp Ala Lys Ala Leu Pro Gly
   690 695 700
   Phe Arg Asn Leu His Val Asp Asp Gln Met Ala Val Ile Gln Tyr Ser
   705 710 715 720
   Trp Met Gly Leu Met Val Phe Ala Met Gly Trp Arg Ser Phe Thr Asn
   725 730 735
   Val Asn Ser Arg Met Leu Tyr Phe Ala Pro Asp Leu Val Phe Asn Glu
   740 745 750
   Tyr Arg Met His Lys Ser Arg Met Tyr Ser Gln Cys Val Arg Met Arg
   755 760 765
   His Leu Ser Gln Glu Phe Gly Trp Leu Gln Ile Thr Pro Gln Glu Phe
   770 775 780
   Leu Cys Met Lys Ala Leu Leu Leu Phe Ser Ile Ile Pro Val Asp Gly
   785 790 795 800
   Leu Lys Asn Gln Lys Phe Phe Asp Glu Leu Arg Met Asn Tyr Ile Lys
   805 810 815
   Glu Leu Asp Arg Ile Ile Ala Cys Lys Arg Lys Asn Pro Thr Ser Cys
   820 825 830
   Ser Arg Arg Phe Tyr Gln Leu Thr Lys Leu Leu Asp Ser Val Gln Pro
   835 840 845
   Ile Ala Arg Glu Leu His Gln Phe Thr Phe Asp Leu Leu Ile Lys Ser
   850 855 860
   His Met Val Ser Val Asp Phe Pro Glu Met Met Ala Glu Ile Ile Ser
   865 870 875 880
   Val Gln Val Pro Lys Ile Leu Ser Gly Lys Val Lys Pro Ile Tyr Phe
   885 890 895
   His Thr Gln
<210> 85
   <211> 895
   <212> PRT
   <213> Macaca mulatta
<400> 85
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Gln Gln Gln Gln Gln Gln Gln Gln Glu Thr
       50                  55                  60
   Ser Pro Arg Gln Gln Gln Gln Gln Gln Gln Gly Glu Asp Gly Ser Pro
   65                  70                  75                  80
   Gln Ala His Arg Arg Gly Pro Thr Gly Tyr Leu Val Leu Asp Glu Glu
                   85                  90                  95
   Gln Gln Pro Ser Gln Pro Gln Ser Ala Pro Glu Cys His Pro Glu Arg
               100                 105                 110
   Gly Cys Val Pro Glu Pro Gly Ala Ala Val Ala Ala Gly Lys Gly Leu
           115                 120                 125
   Pro Gln Gln Leu Pro Ala Pro Pro Asp Glu Asp Asp Ser Ala Ala Pro
       130                 135                 140
   Ser Thr Leu Ser Leu Leu Gly Pro Thr Phe Pro Gly Leu Ser Ser Cys
   145                 150                 155                 160
   Ser Ala Asp Leu Lys Asp Ile Leu Ser Glu Ala Ser Thr Met Gln Leu
                   165                 170                 175
   Leu Gln Gln Gln Gln Gln Glu Ala Val Ser Glu Gly Ser Ser Ser Gly
               180                 185                 190
   Arg Ala Arg Glu Ala Ser Gly Ala Pro Thr Ser Ser Lys Asp Asn Tyr
           195                 200                 205
   Leu Glu Gly Thr Ser Thr Ile Ser Asp Ser Ala Lys Glu Leu Cys Lys
       210                 215                 220
   Ala Val Ser Val Ser Met Gly Leu Gly Val Glu Ala Leu Glu His Leu
   225                 230                 235                 240
   Ser Pro Gly Glu Gln Leu Arg Gly Asp Cys Met Tyr Ala Pro Val Leu
   245 250 255
   Gly Val Pro Pro Ala Val Arg Pro Thr Pro Cys Ala Pro Leu Ala Glu
   260 265 270
   Cys Lys Gly Ser Leu Leu Asp Asp Ser Ala Gly Lys Ser Thr Glu Asp
   275 280 285
   Thr Ala Glu Tyr Ser Pro Phe Lys Gly Gly Tyr Thr Lys Gly Leu Glu
   290 295 300
   Gly Glu Ser Leu Gly Cys Ser Gly Ser Ala Ala Ala Gly Ser Ser Gly
   305 310 315 320
   Thr Leu Glu Leu Pro Ser Thr Leu Ser Leu Tyr Lys Ser Gly Ala Leu
   325 330 335
   Asp Glu Ala Ala Ala Tyr Gln Ser Arg Asp Tyr Tyr Asn Phe Pro Leu
   340 345 350
   Ala Leu Ala Gly Pro Pro Pro Pro Pro Pro Pro Pro His Pro His Ala
   355 360 365
   Arg Ile Lys Leu Glu Asn Pro Leu Asp Tyr Gly Ser Ala Trp Ala Ala
   370 375 380
   Ala Ala Ala Gln Cys Arg Tyr Gly Asp Leu Ala Ser Leu His Gly Ala
   385 390 395 400
   Gly Ala Ala Gly Pro Gly Ser Gly Ser Pro Ser Ala Ala Ala Ser Ser
   405 410 415
   Ser Trp His Thr Leu Phe Thr Ala Glu Glu Gly Gln Leu Tyr Gly Pro
   420 425 430
   Cys Gly Gly Gly Gly Gly Gly Gly Gly Gly Gly Gly Gly Gly Ala Gly
   435 440 445
   Glu Ala Gly Ala Val Ala Pro Tyr Gly Tyr Thr Arg Pro Pro Gln Gly
   450 455 460
   Leu Ala Gly Gln Glu Gly Asp Phe Thr Ala Pro Asp Val Trp Tyr Pro
   465 470 475 480
   Gly Gly Met Val Ser Arg Val Pro Tyr Pro Ser Pro Thr Cys Val Lys
   485 490 495
   Ser Glu Met Gly Pro Trp Met Asp Ser Tyr Ser Gly Pro Tyr Gly Asp
   500 505 510
   Met Arg Leu Glu Thr Ala Arg Asp His Val Leu Pro Ile Asp Tyr Tyr
   515 520 525
   Phe Pro Pro Gln Lys Thr Cys Leu Ile Cys Gly Asp Glu Ala Ser Gly
   530 535 540
   Cys His Tyr Gly Ala Leu Thr Cys Gly Ser Cys Lys Val Phe Phe Lys
   545 550 555 560
   Arg Ala Ala Glu Gly Lys Gln Lys Tyr Leu Cys Ala Ser Arg Asn Asp
   565 570 575
   Cys Thr Ile Asp Lys Phe Arg Arg Lys Asn Cys Pro Ser Cys Arg Leu
   580 585 590
   Arg Lys Cys Tyr Glu Ala Gly Met Thr Leu Gly Ala Arg Lys Leu Lys
   595 600 605
   Lys Leu Gly Asn Leu Lys Leu Gln Glu Glu Gly Glu Ala Ser Ser Thr
   610 615 620
   Thr Ser Pro Thr Glu Glu Thr Ala Gln Lys Leu Thr Val Ser His Ile
   625 630 635 640
   Glu Gly Tyr Glu Cys Gln Pro Ile Phe Leu Asn Val Leu Glu Ala Ile
   645 650 655
   Glu Pro Gly Val Val Cys Ala Gly His Asp Asn Asn Gln Pro Asp Ser
   660 665 670
   Phe Ala Ala Leu Leu Ser Ser Leu Asn Glu Leu Gly Glu Arg Gln Leu
   675 680 685
   Val His Val Val Lys Trp Ala Lys Ala Leu Pro Gly Phe Arg Asn Leu
   690 695 700
   His Val Asp Asp Gln Met Ala Val Ile Gln Tyr Ser Trp Met Gly Leu
   705 710 715 720
   Met Val Phe Ala Met Gly Trp Arg Ser Phe Thr Asn Val Asn Ser Arg
   725 730 735
   Met Leu Tyr Phe Ala Pro Asp Leu Val Phe Asn Glu Tyr Arg Met His
   740 745 750
   Lys Ser Arg Met Tyr Ser Gln Cys Val Arg Met Arg His Leu Ser Gln
   755 760 765
   Glu Phe Gly Trp Leu Gln Ile Thr Pro Gln Glu Phe Leu Cys Met Lys
   770 775 780
   Ala Leu Leu Leu Phe Ser Ile Ile Pro Val Asp Gly Leu Lys Asn Gln
   785 790 795 800
   Lys Phe Phe Asp Glu Leu Arg Met Asn Tyr Ile Lys Glu Leu Asp Arg
   805 810 815
   Ile Ile Ala Cys Lys Arg Lys Asn Pro Thr Ser Cys Ser Arg Arg Phe
   820 825 830
   Tyr Gln Leu Thr Lys Leu Leu Asp Ser Val Gln Pro Ile Ala Arg Glu
   835 840 845
   Leu His Gln Phe Thr Phe Asp Leu Leu Ile Lys Ser His Met Val Ser
   850 855 860
   Val Asp Phe Pro Glu Met Met Ala Glu Ile Ile Ser Val Gln Val Pro
   865 870 875 880
   Lys Ile Leu Ser Gly Lys Val Lys Pro Ile Tyr Phe His Thr Gln
   885 890 895
<210> 86
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 86
   tggggagaac catcctcacc ctgc 24
<210> 87
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 87
   tccaggacca ggtagcctgt gggg 24
<210> 88
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 88
   tgttcccctg gactcagatg ctcc 24
<210> 89
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 89
   tggggcacaa ggagtgggac gcac 24
<210> 90
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 90
   ttcggctgtg aagagagtgt gcca 24
<210> 91
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 91
   cgcttttgac acaagtggga ctgg 24
<210> 92
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 92
   catagtgaca cccagaagct tcat 24
<210> 93
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 93
   gagtcatccc tgcttcataa catt 24
<210> 94
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 94
   gattaccaag tttcttcagc ttcc 24
<210> 95
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 95
   aggccttggc ccacttgacc acgt 24
<210> 96
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 96
   agcatcctgg agttgacatt ggtg 24
<210> 97
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 97
   gacacactgg ctgtacatcc ggga 24
<210> 98
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 98
   gagccatcca aactcttgag agag 24
<210> 99
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 99
   cagtgctttc atgcacagga attc 24
<210> 100
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 100
   attcgaagtt catcaaagaa tttt 24
<210> 101
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 101
   atcgagttcc ttgatgtagt tcat 24
<210> 102
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 102
   gcacttgcac agagatgatc tctg 24
<210> 103
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 103
   aatagatggg cttgactttc ccag 24
<210> 104
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 104
   ataacaggca gaagacatct gaaa 24
<210> 105
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 105
   attccccaag gcactgcaga ggag 24
<210> 106
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 106
   atgggctgac attcatagcc ttca 24

## Description

### FIELD OF INVENTION

The present description provides compounds, compositions and methods for modulating the expression of the androgen receptor. In particular, this invention relates to oligomeric compounds (oligomers), which target the androgen receptor mRNA in a cell, leading to reduced expression of the androgen receptor. Reduction of androgen receptor expression is beneficial for a range of medical disorders, such as cancer, particularly prostate cancer or breast cancer.

### RELATED CASES

This application claims the benefit under 35 U.S.C. § 119(e) of U.S. Provisional Application Serial No. 60/990,125 filed November 26, 2007.

### BACKGROUND

The androgen receptor (AR) is a type of nuclear receptor which is activated by binding of either of the androgenic hormones testosterone or dihydrotestosterone. The main function of the androgen receptor is as a DNA binding transcription factor which regulates gene expression. However the androgen receptor also has additional functions independent of DNA binding. The androgen receptor is most closely related to the progesterone receptor, and progestins in higher dosages can block the androgen receptor.

Whilst in humans the AR gene is single copy and found on the X chromosome at position Xq11-12, the receptor itself exists in two iso-forms (A and B). AR-A is an 87kDa protein which lacks the first 187 amino acids (N-terminal truncation). Isoform AR-B is the full length 110 kDa version.

The binding of androgen to the androgen receptor induces a conformational change to the receptor, resulting in a dissociation of heat shock proteins, dimerization and transport from the cytosol to the cell nucleus where the androgen receptor dimer binds to specific DNA sequences - referred to as hormone response elements. Depending on the interaction with other nuclear proteins, the AR controls gene expression, either increasing or decreasing transcription of specific genes, such as insulin-like growth factor I (IGF-1).

Androgen receptors can also have cytoplasmic activities though with signal transduction proteins in the cytoplasm. Androgen binding to cytoplasmic androgen receptors can cause rapid changes in cell function independent of gene transcription, for example ion transport, as well as indirect influence of gene transcription, for example via mediating other signal transduction pathways, thereby influencing the activity of other transcription factors.

The over-expression of androgen receptor, or expression of mutated androgen receptor genes has been indicated in several diseases, such as cancer, including prostate cancer and breast cancer, as well as other disorders such as polyglutamate disease (Monks et al., PNAS November 2 2007, published on line) alopecia, benign prostatic hyperplasia, spinal and muscular atrophy and Kennedy disease.

WO97/11170 reports on a method of treating a patient diagnosed as having benign prostatic hyperplasia or a prostate cancer comprising administering an antisense oligonucleotide which selectively hybridises to the androgen receptor mRNA. Three antisense oligonucleotide sequences of between 27 - 29 nucleotides are disclosed.

US 6,733,776 and EP 0 692 972 report on a method for treating androgenic alopecia by applying liposomes comprising an antisense nucleic acid that hybridises to an androgen receptor gene. No antisense molecules with specific sequences and targeting the androgen receptor are provided.

US 2005/0164970 reports on a method of treating prostate cancer using siRNA complexes targeting the androgen receptor mRNA.

WO 2005/027833 reports on a method of treating prostate cancer comprising of administering a morpholino oligonucleotide of between 12-40 morpholino sub-units in length to the patient.

WO 2001/083740 reports on an antisense compound having an uncharged morpholino backbone of between 18 to 20 contiguous units which targets the human androgen receptor.

Morpholino antisense compounds work via binding to the nucleic acid target to block access to the mRNA by other molecules, such as molecules involved in mRNA splicing or translation initiation.

US 7,067,256 reports on a ribozyme which apparently mediates inactivation of the androgen receptor. A 19 nucleotide RNA molecule antisense to a corresponding region of the androgen receptor mRNA is provided.

However, despite the application of siRNA, morpholino antisense and ribozymes, none of the above androgen receptor inhibitors have been successful in efficiently down-regulating the androgen-receptor *in vivo* and at pharmacologically acceptable dosages.

In an exemplarary aspect, the present invention provides a new class of androgen receptor antagonists which have been selected based using LNA chemistry, and/or by the selection of particularly effective target sites on the androgen receptor mRNA.

### SUMMARY OF INVENTION

The present invention provides an oligomer having a contiguous nucleotide sequence of a total of 10 - 30 nucleotides, wherein said contiguous nucleotide sequence is at least 80% homologous to the reverse complement of a corresponding region of a mammalian androgen receptor gene or mRNA, wherein said oligomer comprises at least one LNA unit; and wherein the contiguous nucleotide sequence comprises at least 10 contiguous nucleotides which are 100% identical to a corresponding region of SEQ ID NO 94 or SEQ ID NO 58.

In another aspect, the present invention provides a conjugate comprising the oligomer according to the present invention, and at least one non-nucleotide or non-polynucleotide moiety covalently attached to said oligomer.

In a further aspect, the present invention provides a pharmaceutical composition comprising the oligomer according to the present invention, or the conjugate according to the present invention, and a pharmaceutically acceptable diluent, carrier, salt or adjuvant.

The present invention provides, in a further aspect, the oligomer according to the present invention, or the conjugate according to the present invention, for use as a medicament, such as for the treatment of a disease or a medical disorder as disclosed herein, such as a hyperproliferative disorder, such as cancer.

The present invention provides, in another aspect, the use of an oligomer according to the present invention, or a conjugate as defined herein, for the manufacture of a medicament for the treatment of a disease or a medical disorder as disclosed herein, such as a hyperproliferative disorder, such as cancer.

In another aspect, the present invention provides an *in vitro* method for the inhibition of Androgen Receptor in a cell which is expressing the androgen receptor, said method comprising administering an oligomer according to the present invention, or a conjugate according to the present invention to said cell so as to inhibit androgen receptor in said cell.

The description provides an oligomer of between 10 - 50, such as 10 - 30 nucleotides in length which comprises a contiguous nucleotide sequence of a total of between 10 - 50, such as 10 - 30 nucleotides, wherein said contiguous nucleotide sequence is at least 80% (e.g., 85%, 90%, 95%, 98%, or 99%) homologous to a region corresponding to the reverse complement of a nucleic acid which encodes a mammalian androgen receptor, such as a mammalian Androgen Receptor gene or mRNA, such as SEQ ID NO: 1 or naturally occurring variants thereof. Thus, for example, the oligomer hybridizes to a single stranded nucleic acid molecule having the sequence of a (corresponding) portion of SEQ ID NO: 1.

The description provides for a conjugate comprising the oligomer according to the description, and at least one non-nucleotide or non-polynucleotide moiety covalently attached to said oligomer.

The description provides for a pharmaceutical composition comprising the oligomer or the conjugate according to the description, and a pharmaceutically acceptable diluent, carrier, salt or adjuvant.

The description provides for the oligomer or the conjugate according to description, for use as a medicament, such as for the treatment of a disease or a medical disorder as disclosed herein, such as a hyperproliferative disorder, such as cancer.

The description provides for the use of an oligomer or the conjugate according to the description, for the manufacture of a medicament for the treatment of a disease or a medical disorder as disclosed herein, such as a hyperproliferative disorder, such as cancer.

The description provides for a method of treating a disease or a medical disorder as disclosed herein, such as a hyperproliferative disorder, such as cancer, said method comprising administering an oligomer, a conjugate or a pharmaceutical composition according to the description, to a patient suffering from, or likely to suffer from said a disease or a medical disorder.

The description provides for a method for the inhibition of the androgen receptor in a cell which is expressing the androgen receptor, said method comprising administering an oligomer, or a conjugate according to the description to said cell so as to effect the inhibition of the androgen receptor in said cell.

The description provides an oligomer of between 10-50 nucleobases in length which comprises a contiguous nucleobase sequence of a total of between 10-50 nucleobases, wherein said contiguous nucleobase sequence is at least 80% homologous to a corresponding region of a nucleic acid which encodes a mammalian androgen receptor.

The description provides an oligomer of between 10-50 nucleobases in length which comprises a contiguous nucleobase sequence of a total of between 10-50 nucleobases, wherein said contiguous nucleobase sequence is at least 80% identical to the reverse complement of a target region of a nucleic acid which encodes a mammalian androgen receptor.

The description further provides a conjugate comprising the oligomer according to the description, such as a conjugate which, in addition to the nucleobase sequence of the oligomer comprises at least one non-nucleotide or non-polynucleotide moiety ("conjugated moiety") covalently attached to the oligomer of the description.

The invention provides for pharmaceutical compositions comprising an oligomer or conjugate of the invention, and a pharmaceutically acceptable diluent, carrier, salt or adjuvant.

The description further provides for an oligomer according to the description, for use in medicine.

The description further provides for the use of the oligomer of the description for the manufacture of a medicament for the treatment of one or more of the diseases referred to herein, such as a disease selected from the group consisting of: cancer such as breast cancer or prostate cancer, alopecia, benign prostatic hyperplasia, spinal and muscular atrophy, Kennedy disease and polyglutamate disease.

The description further provides for an oligomer according to the description, for use for the treatment of one or more of the diseases referred to herein, such as a disease selected from the group consisting of: cancer such as breast cancer or prostate cancer, alopecia, benign prostatic hyperplasia, spinal and muscular atrophy, Kennedy disease and polyglutamate disease.

Pharmaceutical and other compositions comprising an oligomer of the description are also provided. Further provided are methods of down-regulating the expression of AR in cells or tissues comprising contacting said cells or tissues, in vitro or in vivo, with one or more of the oligomers, conjugates or compositions of the description.

Also disclosed are methods of treating a non-human animal or a human, suspected of having or being prone (susceptible) to a disease or condition, associated with expression, or over-expression, of AR, by administering to the aid animal or human a therapeutically or prophylactically effective amount of one or more of the oligomers, conjugates or pharmaceutical compositions of the description. Further, methods of using oligomers for the inhibition of expression of AR, and for treatment of diseases associated with activity of AR are provided.

The description provides for a method for treating a disease selected from the group consisting of: cancer such as breast cancer such as breast cancer or prostate cancer, alopecia, benign prostatic hyperplasia, spinal and muscular atrophy, Kennedy disease and polyglutamate disease, the method comprising administering (an effective amount of) one or more oligomers of the description, conjugates, or a pharmaceutical compositions thereof, to the description to a patient in need thereof.

The description provides for a method of inhibiting or reducing the expression of the androgen receptor in a cell or a tissue, such as by down-regulation, the method comprising the step of contacting said cell or tissue with an effective amount of one or more oligomers of the description, or conjugates, or pharmaceutical compositions, thereof, so that expression of the androgen receptor is inhibited or reduced.

The description provides, in some embodiments, an oligomer consisting of 10 to 30 contiguous monomers wherein adjacent monomers are covalently linked by a phosphate group or a phosphorothioate group, wherein said oligomer comprises a first region of at least 10 contiguous monomers;wherein at least one monomer of said first region is a nucleoside analogue; wherein the sequence of said first region is at least 80% identical to the reverse complement of the best-aligned target region of a mammalian androgen receptor gene or a mammalian androgen receptor mRNA.

In one aspect, the oligomer of the description is selected from the group consisting of
5'-A·_{S}^{Me}C·_{S}^{Me}C·_{S}a·_{S}a·_{S}g·_{S}t_{S}t_{S}t_{S}c_{S}t_{S}C·_{S}A·_{S}G·_{S}^{Me}C-3' (SEQ ID NO: 58) and,
5'-^{Me}C·_{S}^{Me}C_{S}^{Me}C_{S}a_{S}a_{S}g_{S}g_{S}c_{S}a_{S}t_{gS}c_{S}A_{S}G_{S}A-3' (SEQ.ID NO: 77),
wherein uppercase letters denote beta-D-oxy-LNA monomers and lowercase letters denote DNA monomers, the subscript "s" denotes a phosphorothioate linkage, and ^{Ma}C denotes a beta-D-oxy-LNA monomer containing a 5-methylcytosine base.

### BRIEF DESCRIPTION OF FIGURES

**Figure 1****.** Oligonucleotides presented in Table 3 were evaluated for their potential to knockdown the androgen receptor mRNA at concentrations of 1, 4 and 16 nM in MCF7 cells 24 hours after transfection using Real-time PCR. All results were normalised to GAPDH and inhibition of AR mRNA is shown as percent of untreated control. Results shown are an average of three independent experiments.
**Figure 2****.** Oligonucleotides presented in Table 3 were evaluated for their potential to knockdown the androgen receptor mRNA at concentrations of 1, 4 and 16 nM in A549 cells 24 hours after transfection using Real-time PCR. All results were normalised to GAPDH and inhibition of AR mRNA is shown as percent of untreated control. Results shown are an average of three independent experiments.

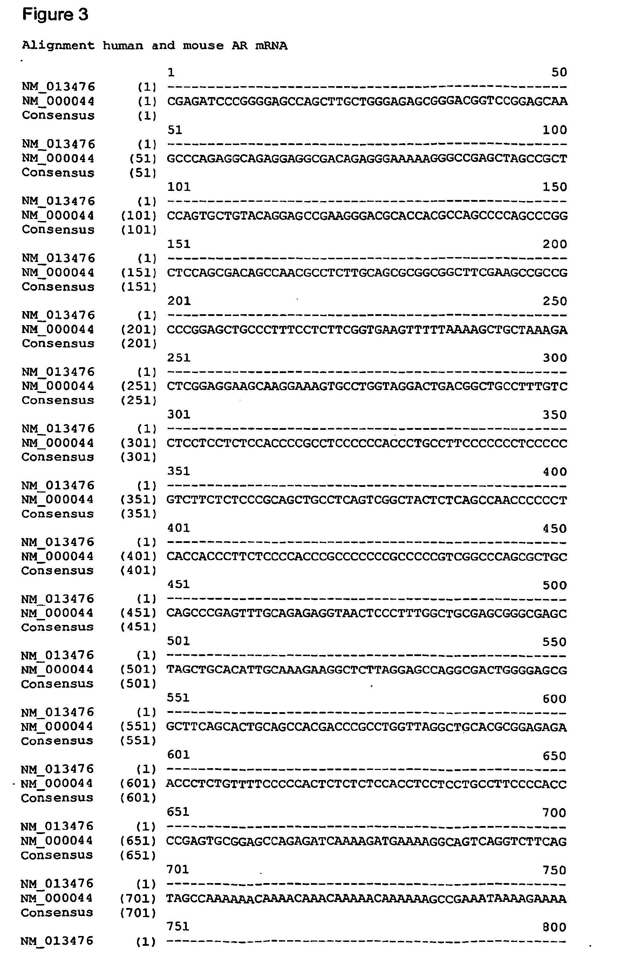

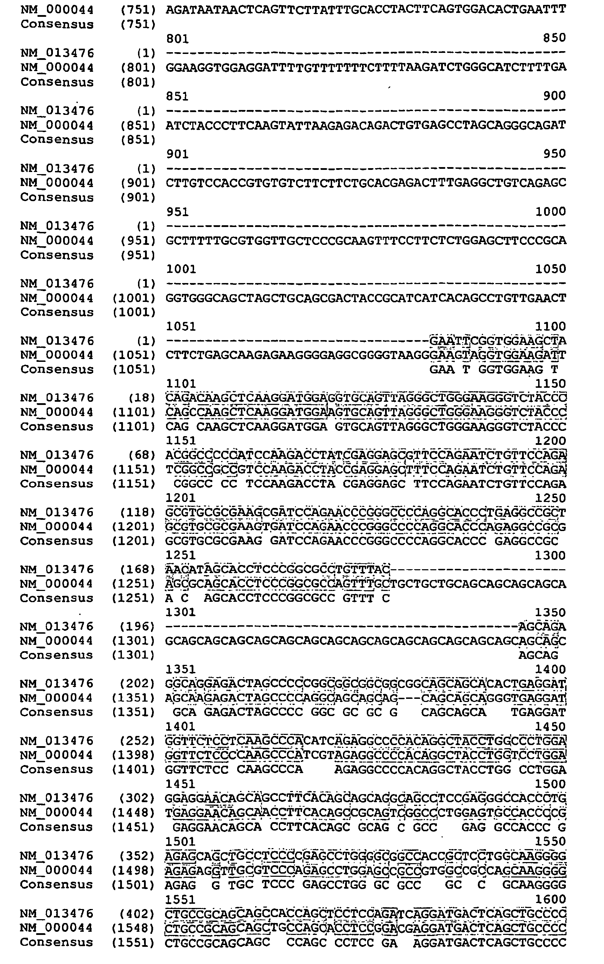

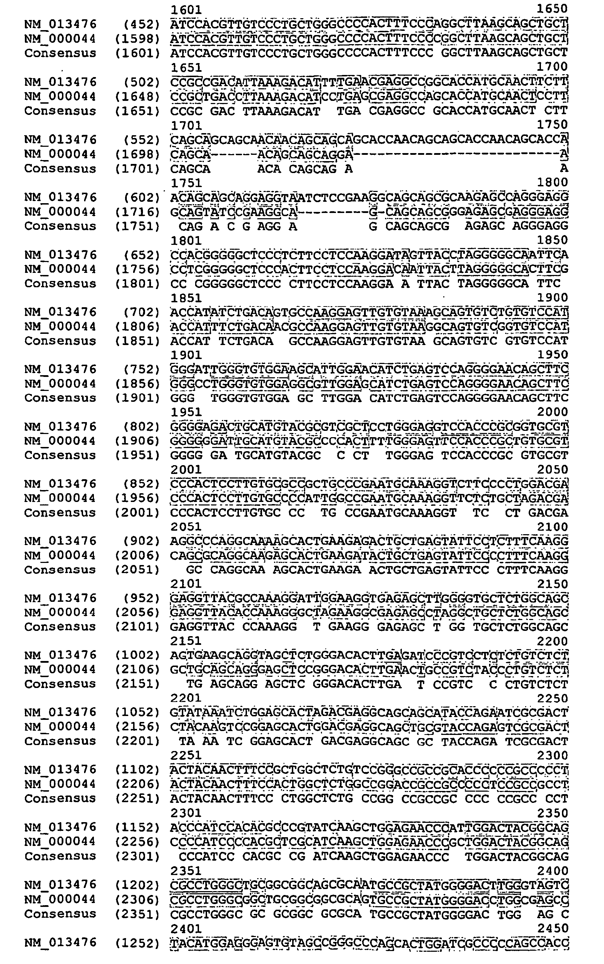

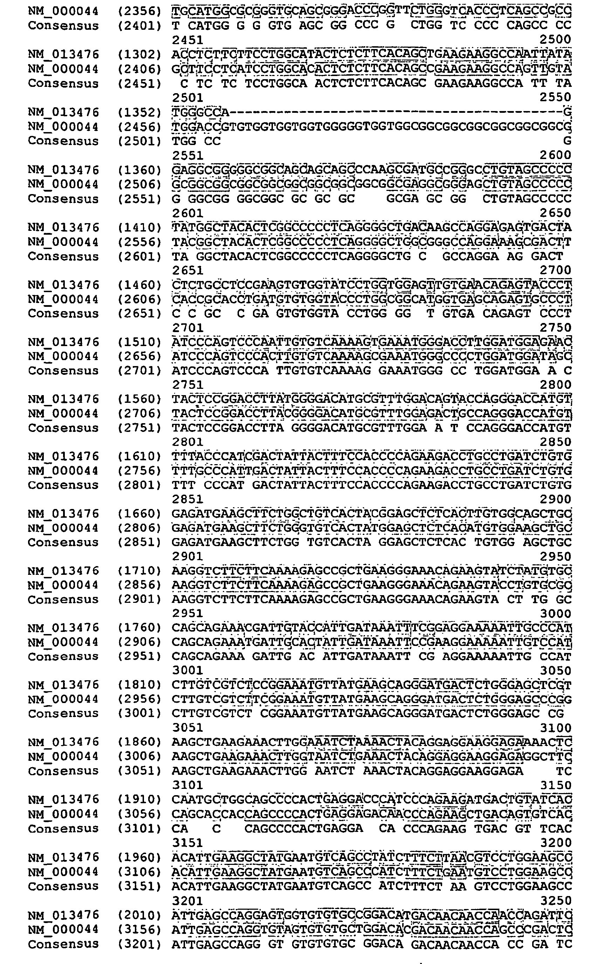

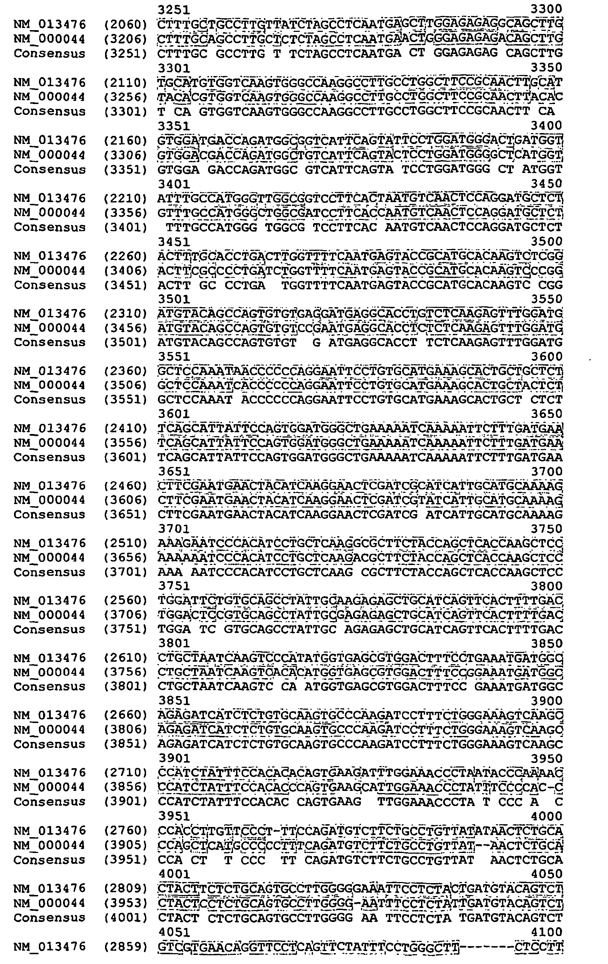

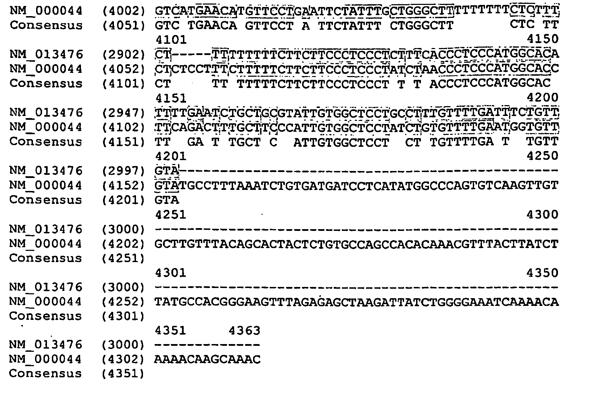

**Figure 3****.** Sequence alignment of the human Androgen receptor mRNA sequence (Genbank Accession No: NM_000044) and the mouse Androgen receptor mRNA sequence (Genbank Accessino No: NM_0134769.
**Figure 4****.** Location of preferred target regions of the human AR mRNA (cDNA) targeted by oligomers according to the description. Although 16mer target sites have been shown, , in some embodiments, these target regions may comprise an additional 4 bases 5' or 3' to the regions shown - *i.e.* are target regions of up to 24 contiguous nucleotides.

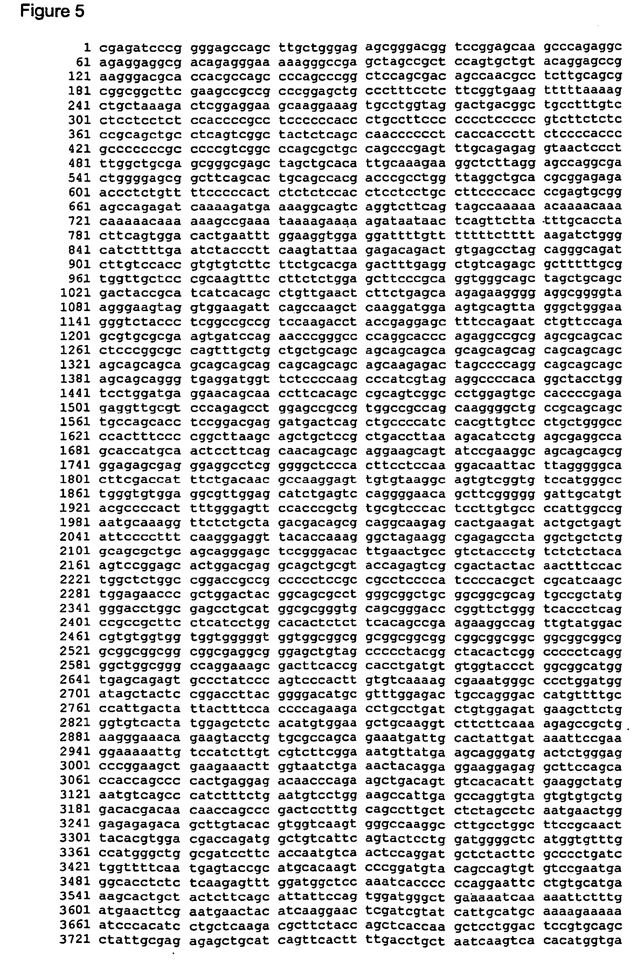

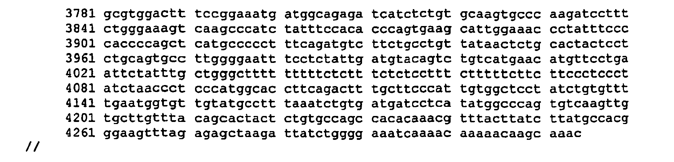

**Figure 5****.** SEQ ID NO:1 *Homo sapiens* androgen receptor (dihydrotestosterone receptor;
testicular feminization; spinal and bulbar muscular atrophy; Kennedy disease) (AR),
transcript variant 1, mRNA. (Genbank Accession number NM_000044).

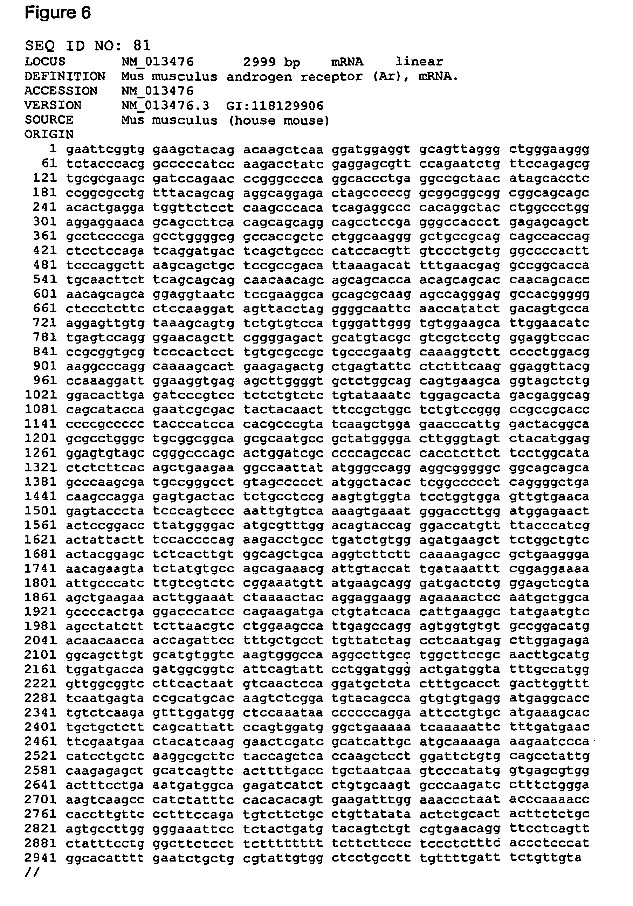

**Figure 6****.** SEQ ID NO 81: Mouse androgen receptor mRNA sequence.

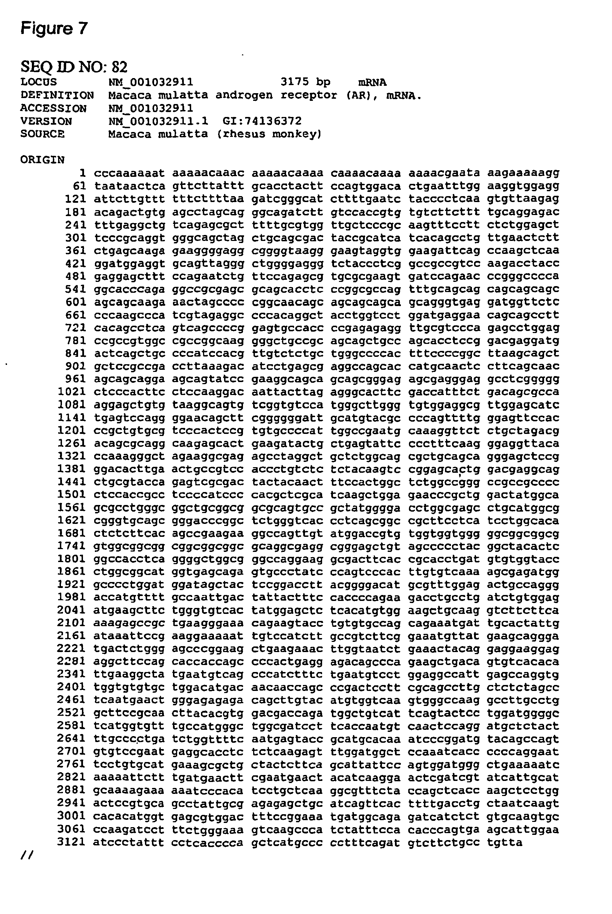

**Figure 7****.** SEQ ID NO 82: Rhesus monkey androgen receptor mRNA sequence.

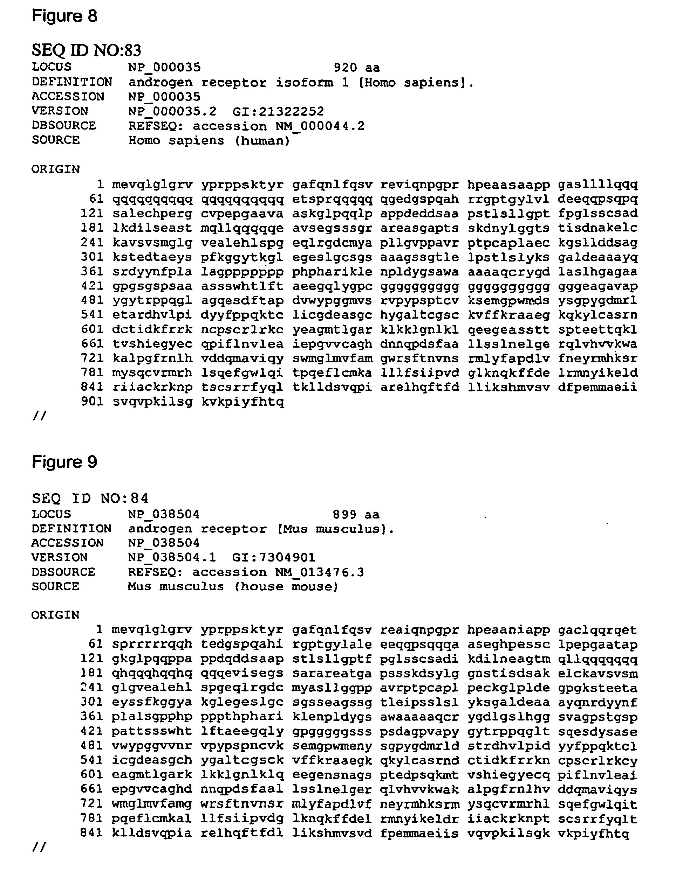

**Figure 8****.** SEQ ID NO 83: *Homo sapiens* androgen receptor protein amino acidsequence.
**Figure 9****.** SEQ ID NO 84: Mouse androgen receptor protein amino acid sequence.

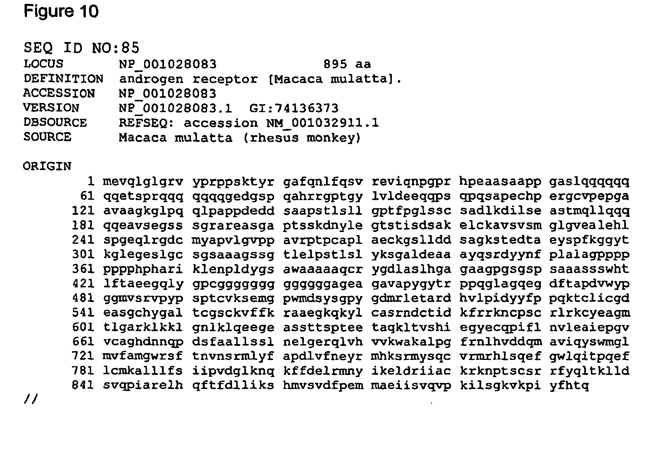

**Figure 10****.** SEQ ID NO 85: Rhesus monkey androgen receptor protein amino acid sequence.
**Figure 11****:** AR mRNA in LNCaP, 24h post-transfection
**Figure 12****:** AR mRNA in A549, 24h post-transfection
**Figure 13****:** Cell proliferation assay - A549, time course post-transfection
**Figure 14****:** Cell proliferation assay - time course post-transfection
**Figure 15****:** Caspase 3/7 activity in LNCaP cells, 24, 48 or 72 hours post-transfection.
**Figure 16****:** Caspase 3/7 activity in A549 cells, 24, 48 or 72 hours post-transfection.
**Figure 17****:** Average PSA in plasma after *in vivo* oligomer treatment.
**Figure 18****:** *In vivo* Inhibition of tumor growth

### DETAILED DESCRIPTION OF INVENTION

### The Oligomer

The present description employs oligomeric compounds (referred herein as oligomers), for use in modulating the function of nucleic acid molecules encoding mammalian Androgen Receptor, such as the Androgen Receptor nucleic acid shown in SEQ ID NO 1, and naturally occurring variants of such nucleic acid molecules encoding mammalian Androgen Receptor. The term "oligomer" in the context of the present description, refers to a molecule formed by covalent linkage of two or more nucleotides (*i.e.* an oligonudeotide). Herein, each single nucleotide, such as the nucleotides present in the oligomer of the description, may also be referred to as a "monomer" or "unit". In some embodiments, the oligomer consists or comprises of a contiguous nucleotide sequence of between 10 - 30 nucleotides in length (*i.e.* comprises or consists of from 10 - 30 covalently linked monomers).

In various embodiments, the compound of the description does not comprise RNA (units). In various embodiments, the compounds according to the description are linear molecules or are synthesised as a linear molecule. The oligomer, in such embodiments, is a single stranded molecule, and typically does not comprise short regions of, for example, at least 3, 4 or 5 contiguous nucleotides, which are complementary to another region within the same oligomer (*i.e.* duplexes) - in this regards, In some embodiments, the oligomer is not (essentially) double stranded. In some embodiments, the oligomer is essentially not double stranded, such as is not a siRNA. In various embodiments, the oligomer of the description may consist entirely of the contiguous nucleotide region. Thus, the oligomer is not substantially self-complementary. siRNAs comprise of 2 complementary short RNA (or equivalent nucleobase units) sequences, such as between 21 and 23nts long, with, typically a 2nt 3' overhang on either end. In order to enhance in vivo uptake, the siRNAs may be conjugated, such as conjugated to a sterol, such as a cholesterol group (typically at the 3' or 5' termini of one or both of the strands).

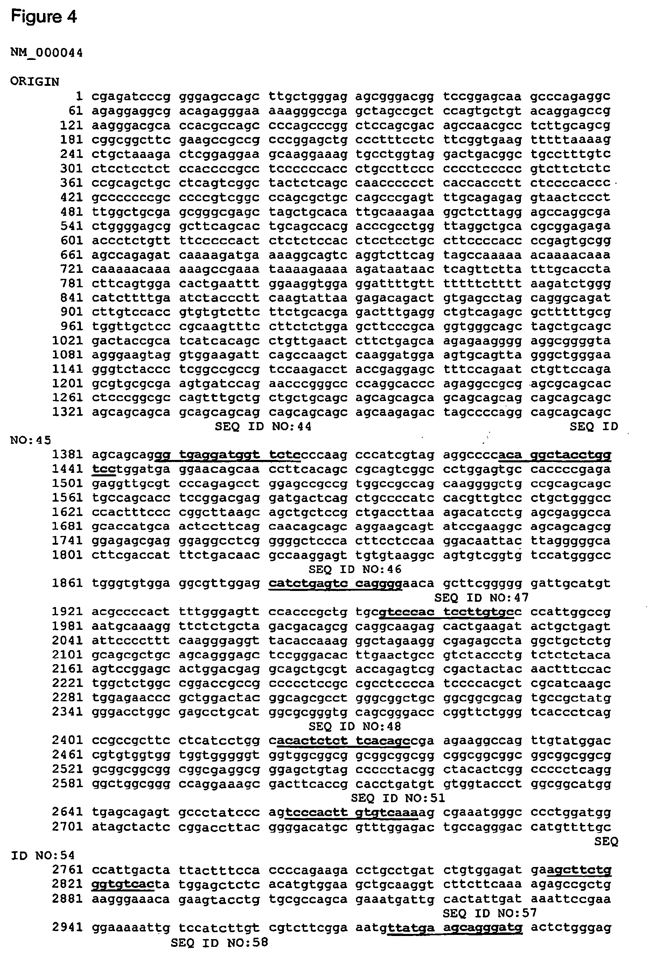

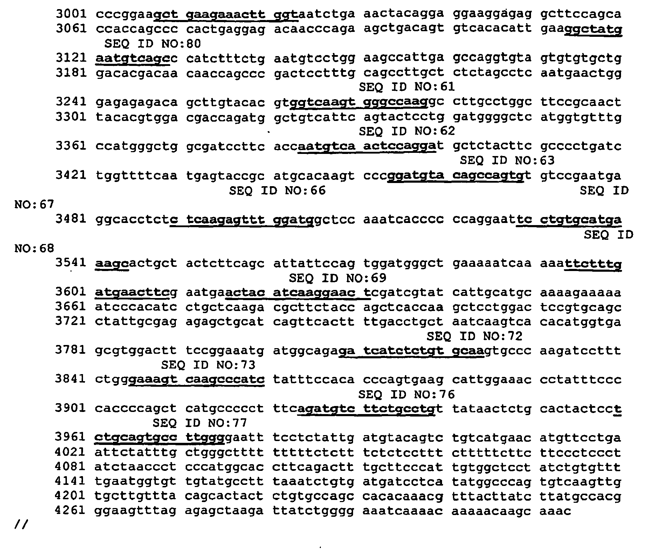

The description further provides target sequences in the AR mRNA or gene, or an allelic variant thereof, in particular those corresponding to SEQ ID NOS: 2 - 22, wherein antisense oligonucleotides corresponding to said target sequences are capable of down-regulating AR. For example, target sequences which correspond to the antisense oligonucleotide sequences SEQ ID NOS: 2 - 22, respectively, are shown in Figure 4 (bold and underlined, with the corresponding oligo SEQ ID NOS indicated above). Variant sequences, for example but not limited to allelic variants (such as a (AR) gene present at gene locus Xq11-12) of such target sequences are also within the scope of the description. A variant sequence may have at least 60%, more preferably at least 70%, more preferably at least 80%, more preferably at least 85%, more preferably at least 90%, more preferably at least 91%, at least 92%, at least 93%, at least 94%, at least 95% sequence homology to a target sequence in AR. Typically, an oligomer of the description corresponding to said variant sequences is still capable of down-regulating AR.

Specific designs of LNA oligonucleotides are also disclosed, for example those shown in SEQ ID NOS 44 - 80. The oligomers of the invention are considered to be potent inhibitors of androgen receptor mRNA and protein expression.

### The Target

The mammalian androgen receptor is preferably selected from the group consisting of human or mouse androgen receptor. Preferably the mammalian androgen receptor is human androgen receptor, such as the Androgen receptor encoded by the nucleic acid as shown in SEQ ID NO 1. Further mammalian androgen receptor genes (targets) and their corresponding proteins are shown in the following table:

| | Genbank Accession Numbers - Nucleic acid (mRNA/cDNA sequence) | Genbank Accession Numbers - Polypeptide (deduced) |
|---|---|---|
| Human | NM_000044 | NP_000035 |
| Mouse | NM_013476 | NP_038504 |
| Rhesus monkey | NM_001032911 | NP_001028083 |

It should be recognised that the Androgen Receptor gene in humans does show a degree of allelic variation, and several of the polymorphismsis recognised that the above-disclosed GenBank Accession numbers for nucleic acids refer to cDNA sequences and not to mRNA sequences per se. The sequence of a mature mRNA can be derived directly from the corresponding cDNA sequence with thymine bases (T) being replaced by uracil bases (U).

It should be recognised that the Androgen Receptor gene in humans does show a degree of allelic variation, and several of the polymorphisms are associated with disease phenotypes (Mooney et al, NAR 15; 31(8) 2003). For example, a CAG repeat expansion is associated with polyglutamine expansion disorder, other characterised allelic variants include a (GGC)n trinucleotide repeat and R726L, T887A and L710H single nucleotide polymorphisms have been identified, and the latter two have been shown to be correlated to enhanced promiscuity of the AR receptor for other steroid ligands. In one embodiment n may range from between 5 and 31. CAG repeats of less than 22 have been associated with an enhanced risk of prostate cancer in African American males, and this may therefore be a preferred allelic variant.

In some embodiments, the target nucleic acid is an AR allelic variant which comprises one or more single nucleotide polymorphisms, including R726L, T887A and L710H.

Therefore, in some embodiments, the target is an AR allelic variant which comprises a (CAG)n trinucleotide repeat, or (GGC)n trinucleotide repeat.

The nucleic acid which encodes the mammalian androgen receptor is, in a preferable embodiment, the human androgen receptor cDNA sequence is shown as SEQ ID NO 1 and/or the mouse androgen receptor cDNA sequence is shown as SEQ ID NO 81, or allelic variants thereof. The oligomer according to the invention is an antisense oligonucleotide.

Therefore, 'the target' of the oligomer according to the invention is the androgen receptor mRNA. The oligomer when introduced into the cell which is expressing the androgen receptor gene, results in reduction of the androgen receptor mRNA level, resulting in reduction in the level of expression of the androgen receptor in the cell.

The androgen receptor is known to regulate the expression of several genes, such as a gene selected from the group consisting of Protein kinase C delta (PRKCD), Glutathione S- transferase theta 2 (GSTT2), transient receptor potential cation channel subfamily V member 3 (TRPV3), Pyrroline-5-carboxylate reductase 1 (PYCR1) or ornithine aminotransferase (OAT) - such genes are referred to as androgen receptor targets herein, and as such, in some embodiments, the oligomers according to the invention may be used to modulate the expression of one or more androgen receptor targets in a cell which is expressing, or is capable of expressing (*i.e.* in the case of alleviation of repression (by the AR) of the androgen receptor target in a cell) said androgen receptor target.

The oligomers which target the androgen receptor mRNA, may hybridize to any site along the target mRNA nucleic acid, such as the 5' untranslated leader, exons, introns and 3'untranslated tail. However, it is preferred that the oligomers which target the androgen receptor mRNA hybridise to the mature mRNA form of the target nucleic acid.

Suitably the oligomer of the invention is capable of down-regulating expression of the Androgen Receptor gene. In this regards, the oligomer of the invention can effect the inhibition of Androgen Receptor, typically in a mammalian such as a human cell. In some embodiments, the oligomers of the invention bind to the target nucleic acid and effect inhibition of expression of at least 10% or 20% compared to the normal expression level, more preferably at least a 30%, 40%, 50%, 60%, 70%, 80%, 90% or 95% inhibition as compared to the normal expression level (such as immediately prior to dosing of the oligomer). In some embodiments, such modulation is seen when using between 0.04 and 25nM, such as between 0.8 and 20nM concentration of the compound of the invention. In the same or a different embodiment, the inhibition of expression is less than 100%, such as less than 98% inhibition, less than 95% inhibition, less than 90% inhibition, less than 80% inhibition, such as less than 70% inhibition. Modulation of expression level may be determined by measuring protein levels, *e.g.* by the methods such as SDS-PAGE followed by western blotting using suitable antibodies raised against the target protein. Alternatively, modulation of expression levels can be determined by measuring levels of mRNA, *e.g.* by northern blotting or quantitative RT-PCR. When measuring via mRNA levels, the level of down-regulation when using an appropriate dosage, such as between 0.04 and 25nM, such as between 0.8 and 20nM concentration, is, in some embodiments, typically to a level of between 10-20% the normal levels in the absence of the compound of the invention.

The description therefore provides a method of down-regulating or inhibiting the expression of the androgen receptor protein and/or mRNA in a cell which is expressing the androgen receptor protein and/or mRNA, said method comprising administering or contacting the oligomer or conjugate according to the invention (suitably in an effective amount) to said cell to down-regulating or inhibiting the expression of the androgen receptor protein and/or mRNA in said cell. Suitably the cell is a mammalian cell such as a human cell. The administration or contacting may occur, in some embodiments, *in vitro*. The administration or contacting may occur, in some embodiments, *in vivo*.

The term "target nucleic acid", as used herein refers to the DNA or RNA encoding mammalian androgen receptor polypeptide, such as human androgen receptor, such as SEQ ID NO: 1. Androgen receptor encoding nucleic acids or naturally occurring variants thereof, and RNA nucleic acids derived therefrom, preferably mRNA, such as pre-mRNA, although preferably mature mRNA. In some embodiments, for example when used in research or diagnostics the "target nucleic acid" may be a cDNA or a synthetic oligonucleotide derived from the above DNA or RNA nucleic acid targets. The oligomer according to the invention is preferably capable of hybridising to the target nucleic acid. It will be recognised that SEQ ID NO: 1 is a cDNA sequences, and as such, corresponds to the mature mRNA target sequence, although uracil is replaced with thymidine in the cDNA sequences.

The term "naturally occurring variant thereof" refers to variants of the androgen receptor polypeptide of nucleic acid sequence which exist naturally within the defined taxonomic group, such as mammalian, such as mouse, monkey, and preferably human. Typically, when referring to "naturally occurring variants" of a polynucleotide the term also may encompass any allelic variant of the androgen receptor encoding genomic DNA which are found at the Chromosome X: 66.68 - 66.87 Mb by chromosomal translocation or duplication, and the RNA, such as mRNA derived therefrom. "Naturally occurring variants" may also include variants derived from alternative splicing of the androgen receptor mRNA. When referenced to a specific polypeptide sequence, *e.g.*, the term also includes naturally occurring forms of the protein which may therefore be processed, *e.g.* by co- or post-translational modifications, such as signal peptide cleavage, proteolytic cleavage, glycosylation, etc.

### Oligomer Sequences

The oligomers comprise or consist of a contiguous nucleotide sequence which corresponds to the reverse complement of a nucleotide sequence present in SEQ ID NO: 1. Thus, the oligomer can comprise or consist of, or a sequence selected from the group consisting of SEQ ID NOS: 2 - 22 and 86 - 106, wherein said oligomer (or contiguous nucleotide portion thereof) may optionally have one, two, or three mismatches against said selected sequence.

The oligomer may comprise or consist of a contiguous nucleotide sequence which is fully complementary (perfectly complementary) to the equivalent region of a nucleic acid which encodes a mammalian androgen receptor (e.g., SEQ ID NO: 1). Thus, the oligomer can comprise or consist of an antisense nucleotide sequence.

However, in some embodiments, the oligomer may tolerate 1, 2, 3, or 4 (or more) mismatches, when hybridising to the target sequence and still sufficiently bind to the target to show the desired effect, *i.e.* down-regulation of the target. Mismatches may, for example, be compensated by increased length of the oligomer nucleotide sequence and/or an increased number of nucleotide analogues, such as LNA, present within the nucleotide sequence.

In some embodiments, the contiguous nucleotide sequence comprises no more than 3, such as no more than 2 mismatches when hybridizing to the target sequence, such as to the corresponding region of a nucleic acid which encodes a mammalian androgen receptor.

In some embodiments, the contiguous nucleotide sequence comprises no more than a single mismatch when hybridizing to the target sequence, such as the corresponding region of a nucleic acid which encodes a mammalian androgen receptor.

The nucleotide sequence of the oligomers of the description or the contiguous nucleotide sequence is preferably at least 80% homologous to a corresponding sequence selected from the group consisting of SEQ ID NOS: 2 - 22 and 86 - 106, such as at least 85%, at least 90%, at least 91%, at least 92%, at least 93%, at least 94%, at least 95%, at least 96% homologous, such as 100% homologous (identical).

The nucleotide sequence of the oligomers of the description or the contiguous nucleotide sequence is preferably at least 80% homologous to the reverse complement of a corresponding sequence present in SEQ ID NO: 1, such as at least 85%, at least 90%, at least 91%, at least 92%at least 93%, at least 94%, at least 95%, at least 96% homologous, at least 97% homologous, at least 98% homologous, at least 99% homologous, such as 100% homologous (identical).

The nucleotide sequence of the oligomers of the description or the contiguous nucleotide sequence is preferably at least 80% complementary to a sub-sequence present in SEQ ID NO: 1, such as at least 85%, at least 90%, at least 91%, at least 92%, at least 93%, at least 94%, at least 95%, at least 96% complementary, at least 97% complementary, at least 98% complementary, at least 99% complementary, such as 100% complementary (perfectly complementary).

In some embodiments the oligomer (or contiguous nucleotide portion thereof) is selected from, or comprises, one of the sequences selected from the group consisting of SEQ ID NOS: 2, 3, 4, 5, 6, 7, 8, 9, 10, 11, 12, 13, 14, 15, 16, 17, 18, 19, 20, 21, and 22, , or a sub-sequence of at least 10 contiguous nucleotides thereof, wherein said oligomer (or contiguous nucleotide portion thereof) may optionally comprise one, two, or three mismatches when compared to the sequence.

In some embodiments the oligomer (or contiguous nucleotide portion thereof) is selected from, or comprises, one of the sequences selected from the group consisting of SEQ ID NOS: 86, 87, 88, 89, 90, 91, 92, 93, 94, 95, 96, 97, 98, 99, 100, 101, 102, 103, 104, 105 and 106, or a sub-sequence of at least 10 contiguous nucleotides thereof, wherein said oligomer (or contiguous nucleotide portion thereof) may optionally comprise one, two, or three mismatches when compared to the sequence.

Other preferred oligomers include a (contiguous) nucleotide sequence, such as a sequence of 12, 13, 14, 15 or 16 contiguous nucleorides in length, which have a nucleotide sequence selected from a sequence from the group consisting of SEQ ID No 2, 3, 4, 5, 6, 7, 8, 9, 10, 11, 12, 13, 14, 15, 16, 17, 18, 19, 20, 21, and 22, wherein said oligomer (or contiguous nucleotide portion thereof) may optionally comprise one, two, or three mismatches against said selected sequence.

In some embodiments the sub-sequence may consist of 11, 12, 13, 14, 15, 16, 17, 18, 19, 20, 21, 22, 23, 24, 25, 26, 27, 28, or 29 contiguous nucleotides, such as between 12 -22, such as between 12-18 nucleotides. Suitably, in some embodiments, the sub-sequence is of the same length as the contiguous nucleotide sequence of the oligomer of the invention.

However, it is recognised that, in some embodiments the nucleotide sequence of the oligomer may comprise additional 5' or 3' nucleotides, such as, independently, 1, 2, 3, 4 or 5 additional nucleotides 5' and/or 3', which are non-complementary to the target sequence. In this respect the oligomer of the description, may, in some embodiments, comprise a contiguous nucleotide sequence which is flanked 5' and or 3' by additional nucleotides. In some embodiments the additional 5' or 3' nucleotides are naturally occurring nucleotides, such as DNA or RNA. In some embodiments, the additional 5' or 3' nucleotides may represent region D as referred to in the context of gapmer oligomers herein.

In some embodiments the oligomer according to the description consists or comprises of a nucleotide sequence according to SEQ ID NO 2, such as SEQ ID NO 44, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 3, such as SEQ ID 45, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 4, such as SEQ ID NO 46, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 5, such as SEQ ID NO 47, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 6, such as SEQ ID NO 48, 49 or 50, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 7, such as SEQ ID NO 51, 52, or 53, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 8, such as SEQ ID 54, 55 or 56, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 9, such as SEQ ID 57, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 10, such as SEQ ID 58, 59, or 60, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 11, such as SEQ ID 61, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 12, such as SEQ ID 62, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 13, such as SEQ ID 63, 64 or 65 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 14, such as SEQ ID 66, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 15, such as SEQ ID 67, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 16, such as SEQ ID 68, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 17, such as SEQ ID 69, 70 or 71, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 18, such as SEQ ID 72, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 19, such as SEQ ID 73, 74 or 75, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 20, such as SEQ ID 76, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 21, such as SEQ ID 77, 78 or 79, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 22, such as SEQ ID 80, or a sub-sequence of at least 10 contiguous nucleotides thereof, such as 11, 12, 13, 14, 15 or 16 contiguous nucleotides thereof.

When determining "homology" between the oligomers of the description (or contiguous nucleotide sequence) and the nucleic acid which encodes the mammalian androgen receptor or the reverse complement thereof, such as those disclosed herein, the determination of homology may be made by a simple alignment with the corresponding nucleotide sequence of the compound of the description and the corresponding region of the nucleic acid which encodes the mammalian androgen receptor (or target nucleic acid), or the reverse complement thereof, and the homology is determined by counting the number of bases which align and dividing by the total number of contiguous nucleotides in the compound of the description, and multiplying by 100. In such a comparison, if gaps exist, it is preferable that such gaps are merely mismatches rather than areas where the number of nucleotides within the gap differ between the nucleotide sequence of the description and the target nucleic acid.

The terms "corresponding to" and "corresponds to" refer to the comparison between the nucleotide sequence of the oligomer or contiguous nucleotide sequence (a first sequence) and the equivalent contiguous nucleotide sequence of a further sequence selected from either i) a sub-sequence of the reverse complement of the nucleic acid target, such as the mRNA which encodes the androgen receptor protein, such as SEQ ID NO: 1, and/or ii) the sequence of nucleotides provided herein such as the group consisting of SEQ ID NOS: 2 - 22 and 86 - 106, or sub-sequence thereof. Nucleotide analogues are compared directly to their equivalent or corresponding nucleotides. A first sequence which corresponds to a further sequence under i) or ii) typically is identicial to that sequence over the length of the first sequence (such as the contiguous nucleotide sequence) or, as described herein may, in some embodiments, is at least 80% homologous to a corresponding sequence, such as at least 85%, at least 90%, at least 91%, at least 92%at least 93%, at least 94%, at least 95%, at least 96% homologous, at least 97% homologous, at least 98% homologous, at least 99% homologous, such as 100% homologous (identical).

The terms "corresponding nucleotide analogue" and "corresponding nucleotide" are intended to indicate that the nucleotide in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

### Length

The oligomers comprise or consist of a contiguous nucleotide sequence of a total of between 10, 11, 12, 13, 14, 15, 16, 17, 18, 19, 20, 21, 22, 23, 24, 25, 26, 27, 28, 29 or 30 contiguous nucleotides in length.

In some embodiments, the oligomers comprise or consist of a contiguous nucleotide sequence of a total of between 10 - 22, such as 12 - 18, such as 13 -17 or 12 - 16, such as 13, 14, 15, 16 contiguous nucleotides in length.

In some embodiments, the oligomers comprise or consist of a contiguous nucleotide sequence of a total of 10, 11, 12, 13, or 14 contiguous nucleotides in length.

In some embodiments, the oligomer according to the invention consists of no more than 22 nucleotides, such as no more than 20 nucleotides, such as no more than 18 nucleotides, such as 15, 16 or 17 nucleotides. In some embodiments the oligomer of the invention comprises less than 20 nucleotides.

### Nucleotide analogues

The term "nucleotide" as used herein, refers to a glycoside comprising a sugar moiety, a base moiety and a covalently linked phosphate group and covers both naturally occurring nucleotides, such as DNA or RNA, preferably DNA, and non-naturally occurring nucleotides comprising modified sugar and/or base moieties, which are also referred to as "nucleotide analogues" herein.

Non-naturally occurring nucleotides include nucleotides which have modified sugar moieties, such as bicyclic nucleotides or 2' modified nucleotides, such as 2' substituted nucleotides.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligonucleotide, *i.e.* have no functional effect on the way the oligonucleotide works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell. Specific examples of nucleoside analogues are described by e.g. Freier & Altmann; Nucl. Acid Res., 1997, 25, 4429-4443 and Uhlmann; Curr. Opinion in Drug Development, 2000, 3(2), 293-213, and in Scheme 1:

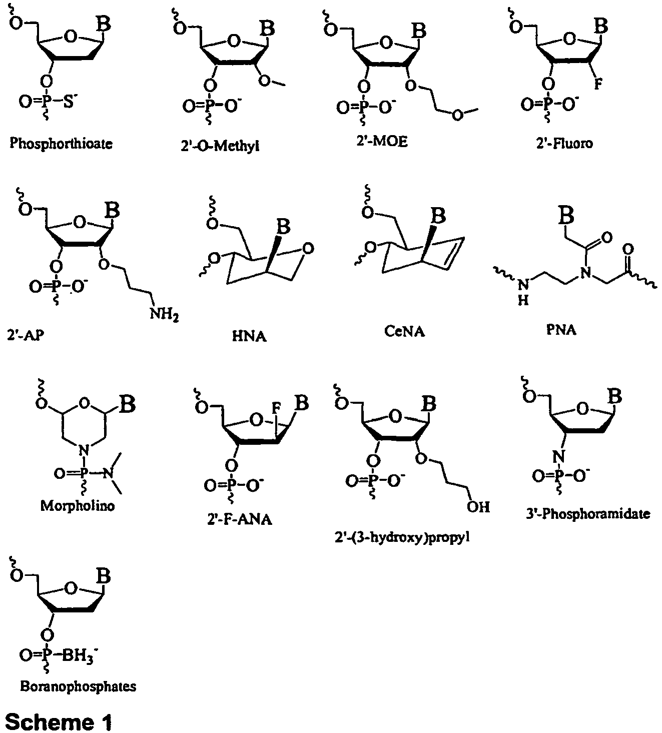

The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, *i.e.* nucleotide analogues. Such nucleotide analogues may suitably enhance the affinity of the oligomer for the target sequence.

Examples of suitable and preferred nucleotide analogues are provided by PCT/DK2006/000512 or are referenced therein.

Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

In some embodiments the oligomer comprises at least 2 nucleotide analogues. In some embodiments, the oligomer comprises from 3-8 nucleotide analogues, e.g. 6 or 7 nucleotide analogues. In the by far most preferred embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

It will be recognised that when referring to a preferred nucleotide sequence motif or nucleotide sequence, which consists of only nucleotides, the oligomers of the invention which are defined by that sequence may comprise a corresponding nucleotide analogue in place of one or more of the nucleotides present in said sequence, such as LNA units or other nucleotide analogues, which raise the duplex stability/Tₘ of the oligomer/target duplex (*i.e.* affinity enhancing nucleotide analogues).

In some embodiments, any mismatches between the nucleotide sequence of the oligomer and the target sequence are preferably found in regions outside the affinity enhancing nucleotide analogues, such as region B as referred to herein, and/or region D as referred to herein, and/or at the site of non modified such as DNA nucleotides in the oligonucleotide, and/or in regions which are 5' or 3' to the contiguous nucleotide sequence.

Examples of such modification of the nucleotide include modifying the sugar moiety to provide a 2'-substituent group or to produce a bridged (locked nucleic acid) structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments the nucleotide analogues present within the oligomer of the invention (such as in regions A and C mentioned herein) are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925,) units and 2'MOE units. In some embodiments there is only one of the above types of nucleotide analogues present in the oligomer of the invention, or contiguous nucleotide sequence thereof.

In some embodiments the nucleotide analogues are 2'-O-methoxyethyl-RNA (2'MOE), 2'-fluoro-DNA monomers or LNA nucleotide analogues, and as such the oligonucleotide of the invention may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments at least one of said nucleotide analogues is 2'-fluoro DNA, such as 2, 3, 4, 5, 6, 7, 8, 9 or 10 2'-fluoro-DNA nucleotide units.

In some embodiments, the oligomer according to the invention comprises at least one Locked Nucleic Acid (LNA) unit, such as 1, 2, 3, 4, 5, 6, 7, or 8 LNA units, such as between 3 - 7 or 4 to 8 LNA units, or 3, 4, 5, 6 or 7 LNA units. In some embodiments, all the nucleotide analogues are LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In some embodiments all LNA cytosine units are 5'methyl-Cytosine. In some embodiments of the invention, the oligomer may comprise both LNA and DNA units. Preferably the combined total of LNA and DNA units is 10-25, preferably 10-20, even more preferably 12-16. In some embodiments of the invention, the nucleotide sequence of the oligomer, such as the contiguous nucleotide sequence consists of at least one LNA and the remaining nucleotide units are DNA units. In some embodiments the oligomer comprises only LNA nucleotide analogues and naturally occurring nucleotides (such as RNA or DNA, most preferably DNA nucleotides), optionally with modified intemucleotide linkages such as phosphorothioate.

The term "nucleobase" refers to the base moiety of a nucleotide and covers both naturally occuring a well as non-naturally occurring variants. Thus, "nucleobase" covers not only the known purine and pyrimidine heterocycles but also heterocyclic analogues and tautomeres thereof.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

In some embodiments, at least one of the nucleobases present in the oligomer is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleotide analogue, known as "Locked Nucleic Acid". It may refer to an LNA monomer, or, when used in the context of an "LNA oligonucleotide" refers to an oligonucleotide containing one or more such bicyclic nucleotide analogues.

The LNA used in the oligonucleotide compounds of the invention preferably has the structure of the general formula I 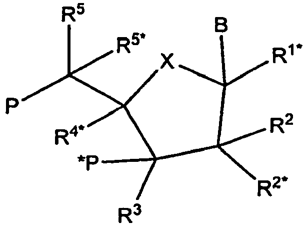
wherein X is selected from -O-, -S-, -N(R^{N*})-, -C(R⁶R^{6*})-;
B is selected from hydrogen, optionally substituted C₁₋₄-alkoxy, optionally substituted C₁₋₄-alkyl, optionally substituted C₁₋₄-acyloxy, nucleobases, DNA intercalators, photochemically active groups, thermochemically active groups, chelating groups, reporter groups, and ligands;
P designates the radical position for an internucleoside linkage to a succeeding monomer, or a 5'-terminal group, such internucleoside linkage or 5'-terminal group optionally including the substituent R⁵ or equally applicable the substituent R^{5*};
P* designates an internucleoside linkage to a preceding monomer, or a 3'-terminal group;
R^{4*} and R^{2*} together designate a biradical consisting of 1-4 groups/atoms selected from -C(R^{a}R^{b})-, -C(R^{a})=C(R^{b})-, -C(R^{a})=N-, -O-, -Si(R^{a})₂-, -S-, -SO₂-, -N(R^{a})-, and >C=Z,
wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂), and each of the substituents R^{1*}, R², R³, R⁵, R^{5*}, R⁶ and R^{6*}, which are present is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof;

In some embodiments R^{5*} is selected from H, -CH₃, -CH₂-CH₃,- CH₂-O-CH₃, and - CH=CH₂.

In some embodiments, R^{4*} and R^{2*} together designate a biradical selected from - C(R^{a}R^{b})-O-, -C(R^{a}R^{b})-C(R^{c}R^{d})-O-, -C(R^{a}R^{b})-C(R^{c}R^{d})-C(R^{c}R^{f})-O-, -C(R^{a}R^{b})-O-C(R^{c}R^{d})-, - C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-, - C(R^{a})=C(R^{b})-C(R^{C}R^{d})-, -C(R^{a}R^{b})-N(R^{c}), -C(R^{a}R^{b})-C(R^{c}R^{d})- N(R^{e}), -C(R^{a}R^{b})-N(R^{c})-O-, and - C(R^{a}R^{b})-S-, -C(R^{a}R^{b})-C(R^{C}R^{d})-S-, wherein R^{a}, R^{b}, R^{c}, R^{d}, R^{e}, and R^{f} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂₋alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂),

In a further embodiment R^{4*} and R^{2*} together designate a biradical (bivalent group) selected from -CH₂-O-, -CH₂-S-, -CH₂-NH-, -CH₂-N(CH₃)-, -CH₂-CH₂-O-, -CH₂-CH(CH₃)-, - CH₂-CH₂-S-, -CH₂-CH₂-NH-, -CH₂-CH₂-CH₂-, -CH₂-CH₂-CH₂-O, -CH₂-CH₂-CH(CH₃)-, - CH=CH-CH₂-, -CH₂-O-CH₂-O-, -CH₂-NH-O-, -CH₂-N(CH₃)-O-, -CH₂-O-CH₂-, -CH(CH₃)-O-, - CH(CH₂-O-CH₃)-O-.

For all chiral centers, asymmetric groups may be found in either *R* or *S* orientation.

Preferably, the LNA used in the oligomer of the invention comprises at least one LNA unit according to any of the formulas 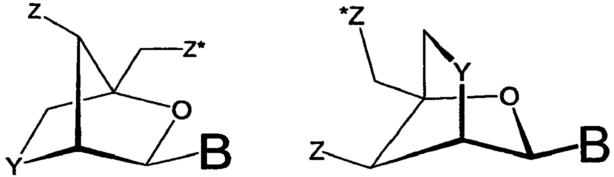 wherein Y is -O-, -O-CH₂- ,-S-, -NH-, or N(R^{H}); Z and Z* are independently selected among an internucleotide linkage, a terminal group or a protecting group; B constitutes a natural or non-natural nucleotide base moiety, and R^{H} is selected from hydrogen and C₁₋₄-alkyl.

Specifically preferred LNA units are shown in scheme 2:

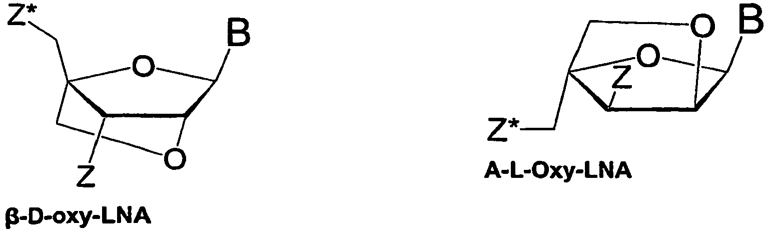

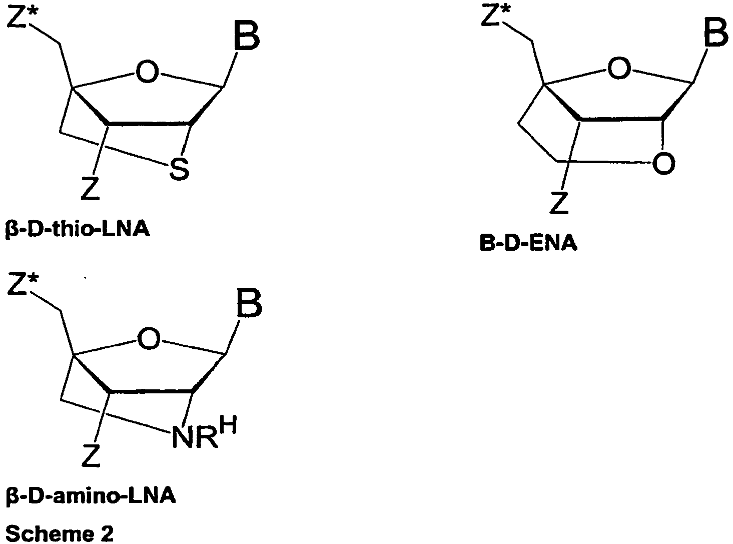

The term "thio-LNA" comprises a locked nucleotide in which Y in the general formula above is selected from S or -CH₂-S-. Thio-LNA can be in either the beta-D and alpha-L-configuration.

The term "amino-LNA" refers to a locked nucleotide in which Y in the general formula above is selected from -N(H)-, N(R)-, CH₂-N(H)-, and -CH2-N(R)- where R is selected from hydrogen and C₁-₄-alkyl. Amino-LNA can be in either the beta-D and alpha-L-configuration.

The term "oxy-LNA" refers to a locked nucleotide in which Y in the general formula above represents -0- or -CH₂-O-. Oxy-LNA can be in either the beta-D and alpha-L-configuration.

The term "ENA" refers to a locked nucleotide in which Y in the general formula above is -CH₂-O- (where the oxygen atom of -CH₂-O- is attached to the 2'-position relative to the base B).

In a preferred embodiment LNA is selected from beta-D-oxy-LNA, alpha-L-oxy-LNA, beta-D-amino-LNA and beta-D-thio-LNA, in particular beta-D-oxy-LNA.

### RNAse recruitment

It is recognised that an oligomeric compound may function via non RNase mediated degradation of target mRNA, such as by steric hindrance of translation, or other methods, however, the preferred oligomers of the invention are capable of recruiting an endoribonuclease (RNase), such as RNase H.

It is preferable that the oligomer, or contiguous nucleotide sequence, comprises of a region of at least 6, such as at least 7 consecutive nucleotide units, such as at least 8 or at least 9 consecutive nucleotide units (residues), including 7, 8, 9, 10, 11, 12, 13, 14, 15 or 16 consecutive nucleotides, which, when formed in a duplex with the complementary target RNA is capable of recruiting RNase. The contiguous sequence which is capable of recruiting RNAse may be region B as referred to in the context of a gapmer as described herein. In some embodiments the size of the contiguous sequence which is capable of recruiting RNAse, such as region B, may be higher, such as 10, 11, 12, 13, 14, 15, 16, 17, 18, 19 or 20 nucleotide units.

EP 1 222 309 provides *in vitro* methods for determining RNaseH activity, which may be used to determine the ability to recruit RNaseH. A oligomer is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

In some embodiments, an oligomer is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

In other embodiments, an oligomer is deemed capable of recruiting RNaseH if, when provided with the complementary RNA target, and RNaseH, the RNaseH initial rate, as measured in pmol/l/min, is at least 20%, such as at least 40 %, such as at least 60 %, such as at least 80 % of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

Typically the region of the oligomer which forms the consecutive nucleotide units which, when formed in a duplex with the complementary target RNA is capable of recruiting RNase consists of nucleotide units which form a DNA/RNA like duplex with the RNA target - and include both DNA units and LNA units which are in the alpha-L configuration, particularly preferred being alpha-L-oxy LNA.

The oligomer of the invention may comprise a nucleotide sequence which comprises both nucleotides and nucleotide analogues, and may be in the form of a gapmer, a headmer or a mixmer.

A headmer is defined by a contiguous stretch of non-RNase recruiting nucleotide analogues at the 5'-end followed by a contiguous stretch of DNA or modified nucleotide units recognizable and cleavable by the RNase towards the 3'-end (such as at least 7 such nucleotides), and a tailmer is defined by a contiguous stretch of DNA or modified nucleotides recognizable and cleavable by the RNase at the 5'-end (such as at least 7 such nucleotides), followed by a contiguous stretch of non-RNase recruiting nucleotide analogues towards the 3'-end. Other chimeras according to the invention, called mixmers consisting of an alternate composition of DNA or modified nucleotides recognizable and cleavable by RNase and non-RNase recruiting nucleotide analogues. Some nucleotide analogues may also be able to mediate RNaseH binding and cleavage. Since α-L-LNA recruits RNaseH activity to a certain extent, smaller gaps of DNA or modified nucleotides recognizable and cleavable by the RNaseH for the gapmer construct might be required, and more flexibility in the mixmer construction might be introduced.

### Gapmer Design

Preferably, the oligomer of the invention is a gapmer. A gapmer oligomer is an oligomer which comprises a contiguous stretch of nucleotides which is capable of recruiting an RNAse, such as RNAseH, such as a region of at least 6 or 7 DNA nucleotides, referred to herein in as region B, wherein region B is flanked both 5' and 3' by regions of affinity enhancing nucleotide analogues, such as between 1 - 6 nucleotide analogues 5' and 3' to the contiguous stretch of nucleotides which is capable of recruiting RNAse - these regions are referred to as regions A and C respectively.

Preferably the gapmer comprises a (poly)nucleotide sequence of formula (5' to 3'), A-B-C, or optionally A-B-C-D or D-A-B-C, wherein; region A (5' region) consists or comprises of at least one nucleotide analogue, such as at least one LNA unit, such as between 1-6 nucleotide analogues, such as LNA units, and; region B consists or comprises of at least five consecutive nucleotides which are capable of recruiting RNAse (when formed in a duplex with a complementary RNA molecule, such as the mRNA target), such as DNA nucleotides, and; region C (3'region) consists or comprises of at least one nucleotide analogue, such as at least one LNA unit, such as between 1-6 nucleotide analogues, such as LNA units, and; region D, when present consists or comprises of 1, 2 or 3 nucleotide units, such as DNA nucleotides.

In some embodiments, region A consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units; and/or region C consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units.

In some embodiments B consists or comprises of 5, 6, 7, 8, 9, 10, 11 or 12 consecutive nucleotides which are capable of recruiting RNAse, or between 6-10, or between 7-9, such as 8 consecutive nucleotides which are capable of recruiting RNAse. In some embodiments region B consists or comprises at least one DNA nucleotide unit, such as 1-12 DNA units, preferably between 4-12 DNA units, more preferably between 6-10 DNA units, such as between 7-10 DNA units, most preferably 8, 9 or 10 DNA units.

In some embodiments region A consist of 3 or 4 nucleotide analogues, such as LNA, region B consists of 7, 8, 9 or 10 DNA units, and region C consists of 3 or 4 nucleotide analogues, such as LNA. Such designs include (A-B-C) 3-10-3, 3-10-4, 4-10-3, 3-9-3, 3-9-4, 4-9-3, 3-8-3, 3-8-4, 4-8-3, 3-7-3, 3-7-4, 4-7-3, and may further include region D, which may have one or 2 nucleotide units, such as DNA units.

Further gapmer designs are disclosed in WO2004/046160.

US provisional application, 60/977409, refers to 'shortmer' gapmer oligomers, which, in some embodiments may be the gapmer oligomer according to the present invention.

In some embodiments the oligomer is consisting of a contiguous nucleotide sequence of a total of 10, 11, 12, 13 or 14 nucleotide units, wherein the contiguous nucleotide sequence is of formula (5' - 3'), A-B-C, or optionally A-B-C-D or D-A-B-C, wherein; A consists of 1, 2 or 3 nucleotide analogue units, such as LNA units; B consists of 7, 8 or 9 contiguous nucleotide units which are capable of recruiting RNAse when formed in a duplex with a complementary RNA molecule (such as a mRNA target); and C consists of 1, 2 or 3 nucleotide analogue units, such as LNA units. When present, D consists of a single DNA unit.

In some embodiments A consists of 1 LNA unit. In some embodiments A consists of 2 LNA units. In some embodiments A consists of 3 LNA units. In some embodiments C consists of 1 LNA unit. In some embodiments C consists of 2 LNA units. In some embodiments C consists of 3 LNA units. In some embodiments B consists of 7 nucleotide units. In some embodiments B consists of 8 nucleotide units. In some embodiments B consists of 9 nucleotide units. In some embodiments B comprises of between 1 - 9 DNA units, such as 2, 3, 4, 5, 6, 7 or 8 DNA units. In some embodiments B consists of DNA units. In some embodiments B comprises of at least one LNA unit which is in the alpha-L configuration, such as 2, 3, 4, 5, 6, 7, 8 or 9 LNA units in the alpha-L-configuration. In some embodiments B comprises of at least one alpha-L-oxy LNA unit or wherein all the LNA units in the alpha-L- configuration are alpha-L-oxy LNA units. In some embodiments the number of nucleotides present in A-B-C are selected from the group consisting of (nucleotide analogue units - region B - nucleotide analogue units): 1-8-1, 1-8-2, 2-8-1, 2-8-2, 3-8-3, 2-8-3, 3-8-2, 4-8-1, 4-8-2, 1-8-4, 2-8-4, or;1-9-1, 1-9-2, 2-9-1, 2-9-2, 2-9-3, 3-9-2, 1-9-3, 3-9-1, 4-9-1, 1-9-4, or, 1-10-1, 1-10-2, 2-10-1, 2-10-2, 1-10-3, 3-10-1. In some embodiments the number of nucleotides in A-B-C are selected from the group consisting of: 2-7-1, 1-7-2, 2-7-2, 3-7-3, 2-7-3, 3-7-2, 3-7-4, and 4-7-3. In some embodiments both A and C consists of two LNA units each, and B consists of 8 or 9 nucleotide units, preferably DNA units.

### Internucleodde Linkages

The terms "linkage group" or "internucleotide linkage" are intended to mean a group capable of covalently coupling together two nucleotides, two nucleotide analogues, and a nucleotide and a nucleotide analogue, etc. Specific and preferred examples include phosphate groups and phosphorothioate groups.

The nucleotide of the oligomer of the invention or contiguous nucleotides sequence thereof are coupled together via linkage groups. Suitably each nucleotide is linked to the 3' adjacent nucleotide via a linkage group.

Suitable internucleotide linkages include those listed within PCT/DK2006/000512. for example the internucleotide linkages listed on the first paragraph of page 34 of PCT/DK2006/000512.

It is, in some embodiments, preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

Suitable sulphur (S) containing internucleotide linkages as provided herein may be preferred. Phosphorothioate internucleotide linkages are also preferred, particularly for the gap region (B) of gapmers. Phosphorothioate linkages may also be used for the flanking regions (A and C, and for linking A or C to D, and within region D, as appropriate).

Regions A, B and C, may however comprise internucleotide linkages other than phosphorothioate, such as phosphodiester linkages, particularly, for instance when the use of nucleotide analogues protects the internucleotide linkages within regions A and C from endo-nuclease degradation - such as when regions A and C comprise LNA nucleotides:

The internucleotide linkages in the oligomer may be phosphodiester, phosphorothioate or boranophosphate so as to allow RNase H cleavage of targeted RNA. Phosphorothioate is preferred, for improved nuclease resistance and other reasons, such as ease of manufacture.

In one aspect of the oligomer of the invention, the nucleotides and/or nucleotide analogues are linked to each other by means of phosphorothioate groups.

It is recognised that the inclusion of phosphodiester linkages, such as one or two linkages, into an otherwise phosphorothioate oligomer, particularly between or adjacent to nucleotide analogue units (typically in region A and or C) can modify the bioavailability and/or bio-distribution of an oligomer - see WO2008/053314.

In some embodiments, such as the embodiments referred to above, where suitable and not specifically indicated, all remaining linkage groups are either phosphodiester or phosphorothioate, or a mixture thereof.

In some embodiments all the internucleotide linkage groups are phosphorothioate. When referring to specific gapmer oligonucleotide sequences, such as those provided herein it will be understood that, in various embodiments, when the linkages are phosphorothioate linkages, alternative linkages, such as those disclosed herein may be used, for example phosphate (phosphodiester) linkages may be used, particularly for linkages between nucleotides analogues, such as LNA, units. Likewise, when referring to specific gapmer oligonucleotide sequences, such as those provided herein, when the C residues are annotated as 5'methyl modified cytosine, in various embodiments, one or more of the Cs present in the oligomer may be unmodified C residues.in some embodimentsin some embodiments

### Oligomeric Compounds

The oligomers of the description may, for example, be selected from the group consisting of: 22 - 43 and 44 to 80.

### Conjugates

In the context of this disclosure, the term "conjugate" indicates a heterogenous molecule formed by the covalent attachment ("conjugation") of the oligomer as described herein to one or more non-nucleotide, or non-polynucleotide moieties. Examples of non-nucleotide or non- polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol.

Therefore, in various embodiments, the oligomer of the invention may comprise both a polynucleotide region which typically consists of a contiguous sequence of nucleotides, and a further non-nucleotide region. When referring to the oligomer of the invention consisting of a contiguous nucleotide sequence, the compound may comprise non-nucleotide components, such as a conjugate component.

In various embodiments of the invention the oligomeric compound is linked to ligands/conjugates, which may be used, e.g. to increase the cellular uptake of oligomeric compounds. WO2007/031091 provides suitable ligands and conjugates.

The invention also provides for a conjugate comprising the compound according to the invention as herein described, and at least one non-nucleotide or non-polynucleotide moiety covalently attached to said compound. Therefore, in various embodiments where the compound of the invention consists of a specified nucleic acid or nucleotide sequence, as herein disclosed, the compound may also comprise at least one non-nucleotide or non-polynucleotide moiety (e.g. not comprising one or more nucleotides or nucleotide analogues) covalently attached to said compound.

Conjugation (to a conjugate moiety) may enhance the activity, cellular distribution or cellular uptake of the oligomer of the invention. Such moieties include, but are not limited to, antibodies, polypeptides, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In certain embodiments the conjugated moiety is a sterol, such as cholesterol.

In various embodiments, the conjugated moiety comprises or consists of a positively charged polymer, such as a positively charged peptides of, for example between 1 -50, such as 2 - 20 such as 3 -10 amino acid residues in length, and/or polyalkylene oxide such as polyethylglycol(PEG) or polypropylene glycol - see WO 2008/034123. Suitably the positively charged polymer, such as a polyalkylene oxide may be attached to the oligomer of the invention via a linker such as the releasable inker described in WO 2008/034123.

By way of example, the following conjugate moieties may be used in the conjugates of the invention:

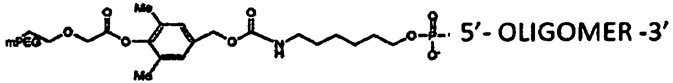

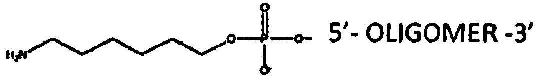

### Activated oligomers

The term "activated oligomer," as used herein, refers to an oligomer of the invention that is covalently linked (i.e., functionalized) to at least one functional moiety that permits covalent linkage of the oligomer to one or more conjugated moieties, i.e., moieties that are not themselves nucleic acids or monomers, to form the conjugates herein described. Typically, a functional moiety will comprise a chemical group that is capable of covalently bonding to the oligomer via, e.g., a 3'-hydroxyl group or the exocyclic NH₂ group of the adenine base, a spacer that is preferably hydrophilic and a terminal group that is capable of binding to a conjugated moiety (e.g., an amino, sulfhydryl or hydroxyl group). In some embodiments, this terminal group is not protected, e.g., is an NH₂ group. In other embodiments, the terminal group is protected, for example, by any suitable protecting group such as those described in "Protective Groups in Organic Synthesis" by Theodora W Greene and Peter G M Wuts, 3rd edition (John Wiley & Sons, 1999). Examples of suitable hydroxyl protecting groups include esters such as acetate ester, aralkyl groups such as benzyl, diphenylmethyl, or triphenylmethyl, and tetrahydropyranyl. Examples of suitable amino protecting groups include benzyl, alpha-methylbenzyl, diphenylmethyl, triphenylmethyl, benzyloxycarbonyl, tert-butoxycarbonyl, and acyl groups such as trichloroacetyl or trifluoroacetyl. In some embodiments, the functional moiety is self-cleaving. In other embodiments, the functional moiety is biodegradable. See e.g., U.S. Patent No. 7,087,229.

In some embodiments, oligomers of the invention are functionalized at the 5' end in order to allow covalent attachment of the conjugated moiety to the 5' end of the oligomer. In other embodiments, oligomers of the invention can be functionalized at the 3' end. In still other embodiments, oligomers of the invention can be functionalized along the backbone or on the heterocyclic base moiety. In yet other embodiments, oligomers of the invention can be functionalized at more than one position independently selected from the 5' end, the 3' end, the backbone and the base.

In some embodiments, activated oligomers of the invention are synthesized by incorporating during the synthesis one or more monomers that is covalently attached to a functional moiety. In other embodiments, activated oligomers of the invention are synthesized with monomers that have not been functionalized, and the oligomer is functionalized upon completion of synthesis. In some embodiments, the oligomers are functionalized with a hindered ester containing an aminoalkyl linker, wherein the alkyl portion has the formula (CH₂)_{w}, wherein w is an integer ranging from 1 to 10, preferably about 6, wherein the alkyl portion of the alkylamino group can be straight chain or branched chain, and wherein the functional group is attached to the oligomer via an ester group (-O-C(O)-(CH₂)_{w}NH).

In other embodiments, the oligomers are functionalized with a hindered ester containing a (CH₂)_{w}-sulfhydryl (SH) linker, wherein w is an integer ranging from 1 to 10, preferably about 6, wherein the alkyl portion of the alkylamino group can be straight chain or branched chain, and wherein the functional group attached to the oligomer via an ester group (-O-C(O)-(CH₂)_{w}SH)

In some embodiments, sulfhydryl-activated oligonucleotides are conjugated with polymer moieties such as polyethylene glycol or peptides (via formation of a disulfide bond).

Activated oligomers containing hindered esters as described above can be synthesized by any method known in the art, and in particular by methods disclosed in PCT Publication No. WO 2008/034122 and the examples therein.

In still other embodiments, the oligomers of the invention are functionalized by introducing sulfhydryl, amino or hydroxyl groups into the oligomer by means of a functionalizing reagent substantially as described in U.S. Patent Nos. 4,962,029 and 4,914,210, i.e., a substantially linear reagent having a phosphoramidite at one end linked through a hydrophilic spacer chain to the opposing end which comprises a protected or unprotected sulfhydryl, amino or hydroxyl group. Such reagents primarily react with hydroxyl groups of the oligomer. In some embodiments, such activated oligomers have a functionalizing reagent coupled to a 5'-hydroxyl group of the oligomer. In other embodiments, the activated oligomers have a functionalizing reagent coupled to a 3'-hydroxyl group. In still other embodiments, the activated oligomers of the invention have a functionalizing reagent coupled to a hydroxyl group on the backbone of the oligomer. In yet further embodiments, the oligomer of the invention is functionalized with more than one of the functionalizing reagents as described in U.S. Patent Nos. 4,962,029 and 4,914,210. Methods of synthesizing such functionalizing reagents and incorporating them into monomers or oligomers are disclosed in U.S. Patent Nos. 4,962,029 and 4,914,210.

In some embodiments, the 5'-terminus of a solid-phase bound oligomer is functionalized with a dienyl phosphoramidite derivative, followed by conjugation of the deprotected oligomer with, e.g., an amino acid or peptide via a Diels-Alder cycloaddition reaction.

In various embodiments, the incorporation of monomers containing 2'-sugar modifications, such as a 2'-carbamate substituted sugar or a 2'-(O-pentyl-N-phthalimido)-deoxyribose sugar into the oligomer facilitates covalent attachment of conjugated moieties to the sugars of the oligomer. In other embodiments, an oligomer with an amino-containing linker at the 2'-position of one or more monomers is prepared using a reagent such as, for example, 5'-dimethoxytrityl-2'-O-(e-phthalimidylaminopentyl)-2'-deoxyadenosine-3'- N,N-diisopropyl-cyanoethoxy phosphoramidite. See, e.g., Manoharan, et al., Tetrahedron Letters, 1991, 34, 7171.

In still further embodiments, the oligomers of the invention may have amine-containing functional moieties on the nucleobase, including on the N6 purine amino groups, on the exocyclic N2 of guanine, or on the N4 or 5 positions of cytosine. In various embodiments, such functionalization may be achieved by using a commercial reagent that is already functionalized in the oligomer synthesis.

Some functional moieties are commercially available, for example, heterobifunctional and homobifunctional linking moieties are available from the Pierce Co. (Rockford, III.). Other commercially available linking groups are 5'-Amino-Modifier C6 and 3'-Amino-Modifier reagents, both available from Glen Research Corporation (Sterling, Va.). 5'-Amino-Modifier C6 is also available from ABI (Applied Biosystems Inc., Foster City, Calif.) as Aminolink-2, and 3'-Amino-Modifier is also available from Clontech Laboratories Inc. (Palo Alto, Calif.).

### Compositions

The oligomer of the invention may be used in pharmaceutical formulations and compositions. Suitably, such compositions comprise a pharmaceutically acceptable diluent, carrier, salt or adjuvant. PCT/DK2006/000512 provides suitable and preferred pharmaceutically acceptable diluent, carrier and adjuvants. Suitable dosages, formulations, administration routes, compositions, dosage forms, combinations with other therapeutic agents, pro-drug formulations are also provided in PCT/DK2006/000512.

### Applications

The oligomers of the invention may be utilized as research reagents for, for example, diagnostics, therapeutics and prophylaxis.

In research, such oligomers may be used to specifically inhibit the synthesis of androgen receptor protein (typically by degrading or inhibiting the mRNA and thereby prevent protein formation) in cells and experimental animals thereby facilitating functional analysis of the target or an appraisal of its usefulness as a target for therapeutic intervention.

In diagnostics the oligomers may be used to detect and quantitate androgen receptor expression in cell and tissues by Northern blotting, in-situ hybridisation or similar techniques.

For therapeutics, an animal or a human, suspected of having a disease or disorder, which can be treated by modulating the expression of androgen receptor is treated by administering antisense compounds in accordance with this invention, suitably in an effective ampount. Further provided are methods of treating a mammal, such as treating a human, suspected of having or being prone to a disease or condition, associated with expression of androgen receptor by administering a therapeutically or prophylactically effective amount of one or more of the oligomers or compositions of the invention.

The pharmaceutical composition according to the invention may be used for the treatment of conditions associated with abnormal levels of androgen receptor, such as cancer, such as prostate or breast cancer,

The pharmaceutical composition according to the invention may be used for the treatment of alopecia, benign prostatic hyperplasia, spinal and muscular atrophy and Kennedy disease, or polyglutamate disease.

It will be recognised that treatment as referred to herein may be prophylactic.

Suitable dosages, formulations, administration routes, compositions, dosage forms, combinations with other therapeutic agents, pro-drug formulations are also provided in PCT/DK2006/000512, although it should be recognised that the aspects of PCT/DK2006/000512 which are only specifically applicable to the treatment of cancer may not be appropriate in the therapeutic/pharmaceutical compositions and methods of the present description.

The description also provides for a pharmaceutical composition comprising a compound or a conjugate as herein described or a conjugate, and a pharmaceutically acceptable diluent, carrier or adjuvant. PCT/DK2006/000512 provides suitable and preferred pharmaceutically acceptable diluent, carrier and adjuvants.

The oligomer, a conjugate or a pharmaceutical composition according to the invention is typically administered in an effective amount.

The invention also provides for the use of the compound or conjugate of the description as described for the manufacture of a medicament for the treatment of a disorder as referred to herein, or for a method of the treatment of as a disorder as referred to herein.

The description also provides for a method for treating a disorder as referred to herein said method comprising administering a compound according to the invention as herein described, and/or a conjugate according to the invention, and/or a pharmaceutical composition according to the invention to a patient in need thereof.

### Pharmaceutical composition comprising more than one active ingredient

The pharmaceutical composition according to the invention may further comprise other active ingredients, including those which are indicated as being useful for the treatment of cancer such as prostate cancer or breast cancer, particularly agents used in conventional antiandrogen therapy.

One such class of compounds are Nonsteroidal antiandrogens (NSAAs), which block the binding of androgens at the receptor site.

Luteinizing hormone-releasing hormone analogs (LHRH-As) suppress testicular production of androgens to castrate levels.

NSAAs, such as CASODEX when used with an LHRH-A as part of Combined Androgen Blocade therapy, help inhibit the growth of prostate cancer cells. In one embodiment, the present description provides for a combined androgen blocade therapy, characterised in that the therapy comprises administering the pharmaceutical compostion according to the invention, and an NSAA and/or LHRH-A agent, which may be administered prior to, during or subsequent to the administering of the pharmaceutical composition of the invention.

The description also provides a kit of parts wherein a first part comprises the oligomer, the conjugate and/or the pharmaceutical composition according to the invention and a further part comprises a Nonsteroidal antiandrogen and/or a Luteinizing hormone-releasing hormone analog. It is therefore envisaged that the kit of parts may be used in a method of treatment, as referred to herein, where the method comprises administering both the first part and the further part, either simultaneously or one after the other.

### Medical indications

The oligomers and other compositions according to the invention can be used for the treatment of conditions associated with over expression or expression of mutate version of the AR. It has been suggested by leading scientists in the field that pharmaceutical intervention with AR will result in therapeutic options against prostate or breast cancer.

Further conditions which may be associated with abnormal levels of androgen receptor, and which, therefore may be treated using the compositions, conjugates and compounds according to the invention include disorders selected form the group consisting of cancer such as breast cancer or prostate cancer, alopecia, benign prostatic hyperplasia, spinal and muscular atrophy, Kennedy disease and polyglutamate disease.

In one embodiment the conditions which may be associated with abnormal levels of androgen receptor, and which, therefore may be treated using the compositions, conjugates and compounds according to the invention include disorders selected form the group consisting of cancer such as breast cancer or prostate cancer.

The description further provides use of a compound of the invention in the manufacture of a medicament for the treatment of any and all conditions disclosed herein.

Generally stated, one aspect of the description is directed to a method of treating a mammal suffering from or susceptible to conditions associated with abnormal levels of androgen receptor, comprising administering to the mammal a therapeutically effective amount of an oligomer targeted to AR that comprises one or more LNA units.

An interesting aspect of the description is directed to the use of an oligomer (compound) as defined herein or as conjugate as defined herein for the preparation of a medicament for the treatment of a condition according to above.

The methods of the description are preferably employed for treatment or prophylaxis against diseases caused by abnormal levels of androgen receptor.

Furthermore, the description encompasses a method of preventing or treating a disease comprising a therapeutically effective amount of an androgen receptor modulating oligomer to a human in need of such therapy. The description further encompasses the use of a short period of administration of an androgen receptor modulating oligonucleotide compound.

In one embodiment of the invention the oligomer (compound) is linked to a ligand or conjugate. For example in order to increase the cellular uptake of the oligomer. in some embodiments the conjugate is a sterol, such as cholesterol.

The oligomers of the invention may also be conjugated to active drug substances, for example, aspirin, ibuprofen, a sulfa drug, an antidiabetic, an antibacterial or an antibiotic.

Alternatively stated, the description is furthermore directed to a method for treating abnormal levels of androgen receptor, said method comprising administering a oligomer of the invention, or a conjugate of the invention or a pharmaceutical composition of the invention to a patient in need thereof and further comprising the administration of a further chemotherapeutic agent. Said further administration may be such that the further chemotherapeutic agent is conjugated to the compound of the invention, is present in the pharmaceutical composition, or is administered in a separate formulation.

The invention also relates to an oligomer, a composition or a conjugate as defined herein for use as a medicament.

The description further relates to use of a compound, composition, or a conjugate as defined herein for the manufacture of a medicament for the treatment of abnormal levels of androgen receptor or expression of mutant forms of AR (such as allelic variants, such as those associated with one of the diseases referred to herein).

Moreover, the description relates to a method of treating a subject suffering from a disease or condition selected from cancer such as breast cancer or prostate cancer, alopecia, benign prostatic hyperplasia, spinal and muscular atrophy, Kennedy disease and polyglutamate disease the method comprising the step of administering a pharmaceutical composition as defined herein to the subject in need thereof.

Furthermore the description refers to methods of regulating genes, and their respective mRNA and protein products, which are modulated by the androgen receptor (i.e androgen receptor targets) such as genes, mRNA and/or proteins selected form the group consisting of: Protein kinase C delta (PRKCD), Glutathione S- transferase theta 2 (GSTT2), transient receptor potential cation channel subfamily V member 3 (TRPV3), Pyrroline-5-carboxylate reductase 1 (PYCR1) or ornithine aminotransferase (OAT). Depedning on the androgen receptor target, the modulation may result in increased expression or activity of the androgen receptor target, or decreased expression or activity.

### EMBODIMENTS

The following embodiments of the present description may be used in combination with the other embodiments described herein.
1. An oligomer of between 10-50 nucleobases in length which comprises a contiguous nucleobase sequence of a total of between 10-50 nucleobases, wherein said contiguous nucleobase sequence is at least 80% homologous to a corresponding region of a nucleic acid which encodes a mammalian androgen receptor.
2. The oligomer according to embodiment 1, wherein said oligomer comprises at least one LNA unit.
3. The oligomer according to embodiment 1 or 2, wherein the contiguous nucleobase sequence comprises no more than 3, such as no more than 2 mismatches to the corresponding region of a nucleic acid which encodes a mammalian androgen receptor.
4. The oligomer according to embodiment 3, wherein said contiguous nucleobase sequence comprises no more than a single mismatch to the corresponding region of a nucleic acid which encodes a mammalian androgen receptor.
5. The oligomer according to embodiment 4, wherein said contiguous nucleobase sequence comprises no mismatches, (*i.e.* is complementary to) the corresponding region of a nucleic acid which encodes a mammalian androgen receptor.
6. The oligomer according to any one of embodiments 1 - 5, wherein the nucleobase sequence of the oligomer consists of the contiguous nucleobase sequence.
7. The oligomer according to any one of embodiments 1- 6, wherein the nucleic acid which, encodes a mammalian androgen receptor is the human androgen receptor nucleotide sequence such as SEQ ID No 1, or a naturally occurring allelic variant thereof.
8. The oligomer according to any one of embodiments 1 - 7, wherein the contiguous nucleobase sequence is complementary to a corresponding region of both the human androgen receptor nucleic acid sequence and a non-human mammalian androgen receptor nucleic acid sequence, such as the mouse androgen receptor nucleic acid sequence.
9. The oligomer according to any one of embodiments 1 to 8, wherein the contiguous nucleobase sequence comprises a contiguous subsequence of at least 7, nucleobase residues which, when formed in a duplex with the complementary androgen receptor target RNA is capable of recruiting RNaseH.
10.The oligomer according to embodiment 9, wherein the contiguous nucleobase sequence comprises of a contiguous subsequence of at least 8, at least 9 or at least 10 nucleobase residues which, when formed in a duplex with the complementary androgen receptor target RNA is capable of recruiting RNaseH.
11.The oligomer according to any one of embodiments 9 or 10 wherein said contiguous subsequence is at least 9 or at least 10 nucleobases in length, such as at least 12 nucleobases or at least 14 nucleobases in length, such as 14, 15 or 16 nucleobases residues which, when formed in a duplex with the complementary androgen receptor target RNA is capable of recruiting RNaseH.
12.The oligomer according to embodiment any one of embodiments 1 - 11 wherein said oligomer is conjugated with one or more non-nucleobase compounds.
13.The oligomer according to any one of embodiments 1 -12, wherein said oligomer has a length of between 10 - 22 nucleobases.
14.The oligomer according to any one of embodiments 1 - 13, wherein said oligomer has a length of between 12 -18 nucleobases.
15.The oligomer according to any one of embodiments 1 - 14, wherein said oligomer has a length of 14, 15 or 16 nucleobases.
16.The oligomer according to any one of embodiments 1 - 15, wherein said continuous nucleobase sequence corresponds to a contiguous nucleotide sequence present in a nucleic acid sequence selected from the group consisting of SEQ ID NO 86 -106.
17. The oligomer according to any one of embodiments 1-16, wherein the oligomer or contiguous nucleobase sequence comprises, or is selected from a corresponding nucleobase sequence present in a nucleotide sequence selected from the group consisting of SEQ ID NO 2 - 22.
18.The oligomer according to any one of embodiments 1 -17, wherein said contiguous nucleobase sequence comprises at least one affinity enhancing nucleotide analogue.
19.The oligomer according to embodiment 18, wherein said contiguous nucleobase sequence comprises a total of 2, 3, 4, 5, 6, 7, 8, 9 or 10 affinity enhancing nucleotide analogues, such as between 5 and 8 affinity enhancing nucleotide analogues.
20. The oligomer according to any one of embodiments 1 -19 which comprises at least one affinity enhancing nucleotide analogue, wherein the remaining nucleobases are selected from the group consisting of DNA nucleotides and RNA nucleotides, preferably DNA nucleotides.
21. The oligomer according to any one of embodiments 1 - 20, wherein the oligomer comprises of a sequence of nucleobases of formula, in 5' to 3' direction, A-B-C, and optionally of formula A-B-C-D, wherein:
   A consists or comprises of at least one nucleotide analogue, such as 1, 2, 3, 4, 5 or 6 nucleotide analogues, preferably between 2-5 nucleotide analogues, preferably 2, 3 or 4 nucleotide analogues, most preferably 2, 3 or 4 consecutive nucleotide analogues and;
   B consists or comprises at least five consecutive nucleobases which are capable of recruiting RNAseH (when formed in a duplex with a complementary RNA molecule, such as the AR mRNA target), such as DNA nucleobases, such as 5, 6, 7, 8, 9, 10, 11 or 12 consecutive nucleobases which are capable of recruiting RNAseH, or between 6-10, orbetween 7-9, such as 8 consecutive nucleobases which are capable of recruiting RNAseH, and;
   C consists or comprises of at least one nucleotide analogue, such as 1, 2, 3, 4, 5, or 6 nucleotide analogues, preferably between 2-5 nucleotide analogues, such as 2, 3 or 4 nucleotide analogues, most preferably 2, 3 or 4 consecutive nucleotide analogues, and;
   D when present, consists or comprises, preferably consists, of one or more DNA nucleotide, such as between 1-3 or 1-2 DNA nucleotides.
22. The oligomers according to embodiment 21, wherein region A consists or comprises of 2, 3 or 4 consecutive nucleotide analogues.
23. The oligomer according to any one of embodiments 21 - 22, wherein region B consists or comprises of 7, 8, 9 or 10 consecutive DNA nucleotides or equivalent nucleobases which are capable of recruiting RNAseH when formed in a duplex with a complementary RNA ,such as the androgen receptor mRNA target.
24. The oligomer according to any one of embodiments 21 - 23, wherein region C consists or comprises of 2, 3 or 4 consecutive nucleotide analogues.
25. The oligomer according to any one of embodiments 21 - 24, wherein region D consists, where present, of one or two DNA nucleotides.
26. The oligomer according to any one of embodiments 21 - 25, wherein:
   A Consists or comprises of 3 contiguous nucleotide analogues;
   B Consists or comprises of 7, 8, 9 or 10 contiguous DNA nucleotides or equivalent nucleobases which are capable of recruiting RNAseH when formed in a duplex with a complementary RNA ,such as the androgen receptor mRNA target;
   C Consists or comprises of 3 contiguous nucleotide analogues;
   D Consists, where present, of one or two DNA nucleotides.
27. The oligomer according to embodiment 26, wherein the contiguous nucleobase sequence consists of 10, 11, 12, 13 or 14 nucleobases, and wherein;
   A Consists of 1 ,2 or 3 contiguous nucleotide analogues;
   B Consists of 7, 8, or 9 consecutive DNA nucleotides or equivalent nucleobases which are capable of recruiting RNAseH when formed in a duplex with a complementary RNA ,such as the androgen receptor mRNA target;
   C Consists of 1 ,2 or 3 contiguous nucleotide analogues;
   D Consists, where present, of one DNA nucleotide.
28. The oligomer according to anyone of embodiments 21 - 27, wherein B comprises at least one LNA nucleobase which is in the alpha-L configuration, such as alpha-L-oxy LNA.
29. The oligomer according to any one of embodiments 1 - 28, wherein the nucleotide analogue(s) are independently or collectively selected from the group consisting of: Locked Nucleic Acid (LNA) units; 2'-O-alkyl-RNA units, 2'-OMe-RNA units, 2'-amino-DNA units, 2'-fluoro-DNA units, PNA units, HNA units, and INA units.
30.The oligomer according to embodiment 29 wherein all the nucleotide analogues(s) are LNA units.
31.The oligomer according to any one of embodiments 1 - 30, which comprises 1, 2, 3, 4, 5, 6, 7. 8. 9 or 10 LNA units such as between 2 and 8 nucleotide LNA units.
32. The oligomer according to any one of the embodiments 29 - 31, wherein the LNAs are independently selected from oxy-LNA, thio-LNA, and amino-LNA, in either of the beta-D and alpha-L configurations or combinations thereof.
33. The oligomer according to embodiment 32, wherein the LNAs are all beta-D-oxy-LNA.
34.The oligomer according to any one of embodiments 21-33, wherein the nucleotide analogues or nucleobases of regions A and C are beta-D-oxy-LNA.
35.The oligomer according to any one of embodiments 1 - 34, wherein at least one of the nucleobases present in the oligomeris a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.
36. The oligomer according to any one of embodiments 1 - 35, wherein said oligomer, hybridises with a corresponding mammalian androgen receptor mRNA with a Tₘ of at least 50°C.
37.The oligomer according to any one of embodiments 1 - 36, wherein said oligomer hybridises with a corresponding mammalian androgen receptor mRNA with a Tₘ of no greater than 80°C.
38. The oligomer according to any one of embodiments 1 - 37, wherein the internucleoside linkages are independently selected from the group consisting of: phosphodiester, phosphorothioate and boranophosphate.
39. The oligomer according to embodiment 38, wherein the oligomer comprises at least one phosphorothioate internucleoside linkage.
40.The oligomer according to embodiment 39, wherein the internucleoside linkages adjacent to or between DNA or RNA units, or within region B are phosphorothioate linkages.
41.The oligomer according to embodiment 39 or 40, wherein the linkages between at least one pair of consecutive nucleotide analogues is a phosphodiester linkage.
42. The oligomer according to embodiment 39 or 40, wherein all the linkages between consecutive nucleotide analogues are phosphodiester linkages.
43. The oligomer according to embodiment 42 wherein all the internucleoside linkages are phosphorothioate linkages.
44. A conjugate comprising the oligomer according to any one of the embodiments 1-43 and at least one non-nucleotide or non-polynucleotide moiety covalently attached to said compound.
45. A pharmaceutical composition comprising an oligomer as defined in any of embodiments 1-43 or a conjugate as defined in embodiment 44, and a pharmaceutically acceptable diluent, carrier, salt or adjuvant.
46. A pharmaceutical composition according to 45, wherein the oligomer is constituted as a pro-drug.
47. A pharmaceutical composition according to embodiment 45 or 46, which further comprises a further therapeutic agent selected from the group consisting of: Nonsteroidal Antiandrogens and Luteinizing hormone -releasing hormone analogs.
48. Use of an oligomer as defined in any one of the embodiments 1-43, or a conjugate as defined in embodiment 44, for the manufacture of a medicament for the treatment of a disease or disorder selected from the group consisting of: Cancer such as breast cancer or prostate cancer, alopecia, benign prostatic hyperplasia, spinal and muscular atrophy, Kennedy disease and polyglutamate disease.
49.An oligomer as defined in any one of the embodiments 1-43, or a conjugate as defined in embodiment 44, for use in the treatment of a disease or disorder selected from the group consisting of: Cancer such as breast cancer or prostate cancer, alopecia, benign prostatic hyperplasia, spinal and muscular atrophy, Kennedy disease and polyglutamate disease.
50. A method for treating a disease or disorder selected from the group consisting of:
   Cancer such as breast cancer or prostate cancer, alopecia, benign prostatic hyperplasia, spinal and muscular atrophy, Kennedy disease and polyglutamate disease, said method comprising administering an oligomer as defined in one of the embodiments 1-43, or a conjugate as defined in embodiment 44, or a pharmaceutical composition as defined in any one of the embodiments 45 - 47, to a patient in need thereof.
51.A method for treating an cancer such as prostate cancer or breast cancer, said method comprising administering an oligomer as defined in one of the embodiments 1-43, or a conjugate as defined in embodiment 44, or a pharmaceutical composition as defined in any one of the embodiments 45 - 47, to a patient in need thereof.
52. A method of reducing or inhibiting the expression of androgen receptor in a cell or a tissue, the method comprising the step of contacting said cell or tissue with a compound as defined in one of the embodiments 1-43, or a conjugate as defined in embodiment 44, or a pharmaceutical composition as defined in any one of the embodiments 45 - 47, so that expression of androgen receptor is reduce or inhibited.

A method for modulating the expression of a gene which is regulated by the androgen receptor (i.e. an androgen receptor target) in a cell which is expressing said gene, said method comprising the step of contacting said cell or tissue with a compound as defined in one of the embodiments 1-43, or a conjugate as defined in embodiment 44, or a pharmaceutical composition as defined in any one of the embodiments 45 - 47, so that expression of androgen receptor target is modulated.

### EXAMPLES

### Example 1: Monomer synthesis

The LNA monomer building blocks and derivatives were prepared following published procedures and references cited therein - see WO07/031081 and the references cited therein.

### Example 2: Oligonucleotide synthesis

Oligonucleotides were synthesized according to the method described in WO07/031081. Table 1 shows examples of antisense oligonucleotide sequences of the description. Tables 2 and 3 show examples of antisense oligonucleotides (oligos) of the description.

### Example 3: Design of the oligonucleotides

In accordance with the present invention, a series of different oligonucleotides were designed to target different regions of human Androgen receptor mRNA(GenBank accession number NM_000044, SEQ ID NO: 1).

**Table 1 Antisense oligonucleotide sequences of the description: SEQ ID NOS: 2-22 are oligo sequences designed to target human Androgen receptor mRNA.**

| **SEQ ID NO** | **Sequence (5'-3')** | **Length (bases)** | **Target site NM_000044** |
|---|---|---|---|
| SEQ ID NO: 2 | GAGAACCATCCTCACC | 16 | 1389-1404 |
| SEQ ID NO: 3 | GGACCAGGTAGCCTGT | 16 | 1428-1443 |
| SEQ ID NO: 4 | CCCCTGGACTCAGATG | 16 | 1881-1896 |
| SEQ ID NO: 5 | GCACAAGGAGTGGGAC | 16 | 1954-1969 |
| SEQ ID NO: 6 | GCTGTGAAGAGAGTGT | 16 | 2422-2437 |
| SEQ ID NO: 7 | TTTGACACAAGTGGGA | 16 | 2663-2678 |
| SEQ ID NO: 8 | GTGACACCCAGAAGCT | 16 | 2813-2828 |
| SEQ ID NO: 9 | CATCCCTGCTTCATAA | 16 | 2975-2990 |
| SEQ ID NO: 10 | ACCAAGTTTCTTCAGC | 16 | 3008-3023 |
| SEQ ID NO: 11 | CTTGGCCCACTTGACC | 16 | 3263-3278 |
| SEQ ID NO: 12 | TCCTGGAGTTGACATT | 16 | 3384-3399 |
| SEQ ID NO: 13 | CACTGGCTGTACATCC | 16 | 3454-3469 |
| SEQ ID NO: 14 | CATCCAAACTCTTGAG | 16 | 3490-3505 |
| SEQ ID NO: 15 | GCTTTCATGCACAGGA | 16 | 3529-3544 |
| SEQ ID NO: 16 | GAAGTTCATCAAAGAA | 16 | 3594-3609 |
| SEQ ID NO: 17 | AGTTCCTTGATGTAGT | 16 | 3616-3631 |
| SEQ ID NO: 18 | TTGCACAGAGATGATC | 16 | 3809-3824 |
| SEQ ID NO: 19 | GATGGGCTTGACTTTC | 16 | 3845-3860 |
| SEQ ID NO: 20 | CAGGCAGAAGACATCT | 16 | 3924-3939 |
| SEQ ID NO: 21 | CCCAAGGCACTGCAGA | 16 | 3960-3975 |
| SEQ ID NO: 22 | GCTGACATTCATAGCC | 16 | 3114-3129 |
| SEQ ID NO: 86 | TGGGGAGAACCATCCTCACCCTGC | 24 | 1385-1408 |
| SEQ ID NO: 87 | TCCAGGACCAGGTAGCCTGTGGGG | 24 | 1424-1447 |
| SEQ ID NO: 88 | TGTTCCCCTGGACTCAGATGCTCC | 24 | 1877-1990 |
| SEQ ID NO: 89 | TGGGGCACAAGGAGTGGGACGCAC | 24 | 1950-1973 |
| SEQ ID NO: 90 | TTCGGCTGTGAAGAGAGTGTGCCA | 24 | 2418-2441 |
| SEQ ID NO: 91 | CGCTTTTGACACAAGTGGGACTGG | 24 | 2659-2682 |
| SEQ ID NO: 92 | CATAGTGACACCCAGAAGCTTCAT | 24 | 2809-2832 |
| SEQ ID NO: 93 | GAGTCATCCCTGCTTCATAACATT | 24 | 2971-2994 |
| SEQ ID NO: 94 | GATTACCAAGTTTCTTCAGCTTCC | 24 | 3004-3027 |
| SEQ ID NO: 95 | AGGCCTTGGCCCACTTGACCACGT | 24 | 3259-3282 |
| SEQ ID NO: 96 | AGCATCCTGGAGTTGACATTGGTG | 24 | 3380-3403 |
| SEQ ID NO: 97 | GACACACTGGCTGTACATCCGGGA | 24 | 3450-3473 |
| SEQ ID NO: 98 | GAGCCATCCAAACTCTTGAGAGAG | 24 | 3486-3509 |
| SEQ ID NO: 99 | CAGTGCTTTCATGCACAGGAATTC | 24 | 3525-3548 |
| SEQ ID NO: 100 | ATTCGAAGTTCATCAAAGAATTTT | 24 | 3590-3613 |
| SEQ ID NO: 101 | ATCGAGTTCCTTGATGTAGTTCAT | 24 | 3612-3635 |
| SEQ ID NO: 102 | GCACTTGCACAGAGATGATCTCTG | 24 | 3805-3828 |
| SEQ ID NO: 103 | AATAGATGGGCTTGACTTTCCCAG | 24 | 3841-3864 |
| SEQ ID NO: 104 | ATAACAGGCAGAAGACATCTGAAA | 24 | 3920-3943 |
| SEQ ID NO: 105 | ATTCCCCAAGGCACTGCAGAGGAG | 24 | 3956-3979 |
| SEQ ID NO: 106 | ATGGGCTGACATTCATAGCCTTCA | 24 | 3110-3133 |

**Table 2: Oligonucleotide designs of the description - In SEQ ID NOs: 23-43 upper case letters indicates nucleotide analogue units and subscript "s" represents phosphorothiote linkage. Lower case letters represent nucleotide units. Absence of "s" (if any) indicates phosphodiester linkage.**

| **SEQ ID NO** | **Sequence (5'-3')** |
|---|---|
| SEQ ID NO: 23 | 5'-**G·_{S}A·_{S}G·_{S}**a·_{S}a·_{S}c·_{S}c·_{S}a·_{S}t·_{S}c·_{S}c·_{S}t·_{S}c·_{S}T·_{S}**A·_{S}C**·**_{S}**C-3' |
| SEQ ID NO: 24 | 5'-**G·_{S}G·_{S}A·_{S}**c·_{S}c·_{S}a·_{S}g·_{S}g·_{S}t·_{S}a·_{S}g·_{S}c·_{S}c·_{S}**T·_{S}G·_{S}**T-3' |
| SEQ ID NO: 25 | 5'-**C**·**_{S}C_{S}C·_{S}**C·_{S}t·_{S}g·_{S}g·_{S}a·_{S}c·_{S}t·_{S}c·_{S}a·_{S}g·_{S}**A·_{S}T·_{S}G**-3' |
| SEQ ID NO: 26 | 5'-**G·_{S}C·_{S}A·_{S}**c·_{S}a·_{S}a·_{S}g·_{S}g·_{S}a·_{S}g·_{S}t·_{S}g·_{S}g·_{S}**G·_{S}A·_{S}C**-3' |
| SEQ ID NO: 27 | 5'-**G**·**_{S}C·_{S}T·_{S}**g·_{S}t·_{S}g·_{S}a·_{S}a·_{S}g·_{S}a·_{S}g·_{S}a·_{S}g·_{S}**T·_{S}G·_{S}T**-3' |
| SEQ ID NO: 28 | 5'-**T·_{S}T·_{S}T·_{S}**g·_{S}a·_{S}c·_{S}a·_{S}c·_{S}a·_{S}a·_{S}g·_{S}t·_{S}g·_{S}**G·_{S}G·_{S}A**-3' |
| SEQ ID NO: 29 | 5'-**G·_{S}T·_{S}G·_{S}**a·_{S}c·_{S}a·_{S}c·_{S}c·_{S}c·_{S}a·_{S}g·_{S}a·_{S}a·_{S}**G·_{S}G·_{S}T**-3' |
| SEQ ID NO: 30 | 5'-**C·_{S}A·_{S}T·_{S}**c·_{S}c·_{S}c·_{S}t·_{S}g·_{S}c·_{S}t·_{S}t·_{S}c·_{S}a·_{S}**T·_{S}A·_{S}A**-3' |
| SEQ ID NO: 31 | **5'-A·_{S}C·_{S}C·_{S}a·_{S}a·_{S}g·_{S}t·_{S}t·_{S}t·_{S}c·_{S}t·_{S}t·_{S}c·_{S}A·_{S}G·_{S}C-3'** |
| SEQ ID NO: 32 | 5'-**C·_{S}T·_{S}T·_{S}**g·_{S}g·_{S}c·_{S}c·_{S}c·_{S}a·_{S}c·_{S}t·_{S}t·_{S}g·_{S}**A·_{S}C·_{S}C**-3' |
| SEQ ID NO: 33 | 5'-**T·_{S}C·_{S}C·_{S}**t·_{S}g·_{S}g·_{S}a·_{S}g·_{S}t·_{S}t·_{S}g·_{S}a·_{S}c·_{S}**A·_{S}T·_{S}T**-3' |
| SEQ ID NO: 34 | 5'-**C·_{S}A·_{S}C·_{S}**t·_{S}g·_{S}g·_{S}c·_{S}t_{S}g·_{S}t·_{S}a·_{S}**T·_{S}C·_{S}C**-3' |
| SEQ ID NO: 35 | 5'-**C**·**_{S}A·_{S}T·_{S}**c·_{S}c·_{S}a·_{S}a·_{S}a·_{S}t·_{S}c·_{S}t·_{S}t·_{S}**G·_{S}A·_{S}G**-3' |
| SEQ ID NO: 36 | 5'-**G·_{S}C·_{S}T·_{S}**t·_{S}t·_{S}c·_{S}a·_{S}t·_{S}g·_{S}c·_{S}a·_{S}c·_{S}a·_{S}**G·_{S}G·_{S}A**-3' |
| SEQ ID NO: 37 | 5'-**G**·**_{S}A·_{S}A·_{S}**g·_{S}t·_{S}t·_{S}c·_{S}a·_{S}t·_{S}c·_{S}a·_{S}a·_{S}a·_{S}**G·_{S}A·_{S}A**-3' |
| SEQ ID NO: 38 | 5'-**A·_{S}G·_{S}T·_{S}**t·_{S}c·_{S}c·_{S}t·_{S}t·_{S}g·_{S}a·_{S}t·_{S}g·_{S}t·_{S}**A·_{S}G·_{S}T**-3' |
| SEQ ID NO: 39 | 5'-**T·_{S}T·_{S}**G·_{S}c·_{S}a·_{S}c·_{S}a·_{S}g·_{S}a·_{S}g·_{S}a·_{S}t·_{S}g·_{S}**A·_{S}T·_{S}C**-3' |
| SEQ ID NO: 40 | 5'-**G·_{S}A·_{S}T·_{S}**g·_{S}g·_{S}g·_{S}c·_{S}t·_{S}t·_{S}g·_{S}a·_{S}c·_{S}t·_{S}**T·_{S}T·_{S}C**-3' |
| SEQ ID NO: 41 | 5'-**C·_{S}A·_{S}**G·_{S}g·_{S}c·_{S}a·_{S}g·_{S}a·_{S}a·_{S}g·_{S}a·_{S}c·_{S}a·_{S}**T·_{S}C·_{S}T·_{S}**T-3' |
| SEQ ID NO: 42 | 5'-**C·_{S}C·_{S}**C·_{S}a·_{S}a·_{S}g·_{S}g·_{S}c·_{S}a·_{S}c·_{S}t·_{S}g·_{S}c·_{S}**A·_{S}G·_{S}A**-3' |
| SEQ ID NO: 43 | 5'-**G_{S}C_{S} T_{S}**g_{S} a_{S}c_{S} a_{S} t_{S} t_{S} c_{S} a_{S} t_{S} a_{S} G_{S} **G_{S} C_{S}** C-3' |

### Example 4: In vitro model: Cell culture

The effect of antisense oligonucleotides on target nucleic acid expression can be tested in any of a variety of cell types provided that the target nucleic acid is present at measurable levels. The target can be expressed endogenously or by transient or stable transfection of a nucleic acid encoding said target. The expression level of target nucleic acid can be routinely determined using, for example, Northern blot analysis, Real-Time PCR, Ribonuclease protection assays. The following cell types are provided for illustrative purposes, but other cell types can be routinely used, provided that the target is expressed in the cell type chosen.

Cells were cultured in the appropriate medium as described below and maintained at 37°C at 95-98% humidity and 5% CO₂. Cells were routinely passaged 2-3 times weekly.

A549 The human lung cancer cell line A5439 was cultured in DMEM (Sigma) + 10% fetal bovine serum (FBS) + 2 mM Glutamax I + gentamicin (25µg/ml).

MCF7 The human breast cancer cell line MCF7 was cultured in EagleMEM (Sigma) + 10% fetal bovine serum (FBS) + 2 mM Glutamax I + 1xNEAA + gentamicin (25µg/ml).

### Example 5: In vitro model: Treatment with antisense oligonucleotide+

The cell lines listed in example 4 were treated with oligonucleotide using the cationic liposome formulation LipofectAMINE 2000 (Gibco) as transfection vehicle. Cells were seeded in 6-well cell culture plates (NUNC) and treated when 80-90% confluent. Oligo concentrations used ranged from 1 nM to 16 nM final concentration. Formulation of oligo-lipid complexes were carried out essentially as described by the manufacturer using serum-free OptiMEM (Gibco) and a final lipid concentration of 5 µg/mL LipofectAMINE 2000. Cells were incubated at 37°C for 4 hours and treatment was stopped by removal of oligo-containing culture medium. Cells were washed and serum-containing media was added. After oligo treatment cells were allowed to recover for 20 hours before they were harvested for RNA analysis.

### Example 6: In vitro model: Extraction of RNA and cDNA synthesis

### Total RNA Isolation and First strand synthesis

Total RNA was extracted from cells transfected as described above and using the Qiagen RNeasy kit (Qiagen cat. no. 74104) according to the manufacturer's instructions. First strand synthesis was performed using Reverse Transcriptase reagents from Ambion according to the manufacturer's instructions.

For each sample 0.5 µg total RNA was adjusted to (10.8 µl) with RNase free H₂O and mixed with 2 µl random decamers (50 µM) and 4 µl dNTP mix (2.5 mM each dNTP) and heated to 70 °C for 3 min after which the samples were rapidly cooled on ice. After cooling the samples on ice, 2 µl 10x Buffer RT, 1 µl MMLV Reverse Transcriptase (100 U/µl) and 0.25 µl RNase inhibitor (10 U/µl) was added to each sample, followed by incubation at 42 °C for 60 min, heat inactivation of the enzyme at 95°C for 10 min and then the sample was cooled to 4 °C.

### Example 7: In vitro model: Analysis of Oligonucleotide Inhibition of Androgen receptor Expression by Real-time PCR

Antisense modulation of Androgen receptor expression can be assayed in a variety of ways known in the art. For example, Androgen receptor mRNA levels can be quantitated by, e.g., Northern blot analysis, competitive polymerase chain reaction (PCR), or real-time PCR. Real-time quantitative PCR is presently preferred. RNA analysis can be performed on total cellular RNA or mRNA.

Methods of RNA isolation and RNA analysis such as Northern blot analysis is routine in the art and is taught in, for example, Current Protocols in Molecular Biology, John Wiley and Sons.

Real-time quantitative (PCR) can be conveniently accomplished using the commercially available Multi-Color Real Time PCR Detection System, available from Applied Biosystem.

### Real-time Quantitative PCR Analysis of Androgen receptor mRNA Levels

The sample content of human Androgen receptor mRNA was quantified using the human Androgen receptor ABI Prism Pre-Developed TaqMan Assay Reagents (Applied Biosystems cat. no. Hs00171172_m1) according to the manufacturer's instructions. Glyceraldehyde-3-phosphate dehydrogenase (GAPDH) mRNA quantity was used as an endogenous control for normalizing any variance in sample preparation.

The sample content of human GAPDH mRNA was quantified using the human GAPDH ABI Prism Pre-Developed TaqMan Assay Reagent (Applied Biosystems cat. no. 4310884E) according to the manufacturer's instructions.

Real-time Quantitative PCR is a technique well known in the art and is taught in for example Heid et al. Real time quantitative PCR, Genome Research (1996), 6: 986-994.

Real time PCR: The cDNA from the first strand synthesis performed as described in example 6 was diluted 2-20 times, and analyzed by real time quantitative PCR using Taqman 7500 FAST or 7900 FAST from Applied Biosystems. The primers and probe were mixed with 2 x Taqman Fast Universal PCR master mix (2x) (Applied Biosystems Cat.# 4364103) and added to 4 µl cDNA to a final volume of 10µl. Each sample was analysed in duplicate. Assaying 2 fold dilutions of a cDNA that had been prepared on material purified from a cell line expressing the RNA of interest generated standard curves for the assays. Sterile H₂O was used instead of cDNA for the no template control. PCR program: 95° C for 30 seconds, followed by 40 cycles of 95° C, 3 seconds, 60° C, 20-30 seconds. Relative quantities of target mRNA sequence were determined from the calculated Threshold cycle using the Applied Biosystems Fast System SDS Software Version 1.3.1.21. or SDS Software Version 2.3.

### Example 8: In vitro analysis: Antisense Inhibition of Human Androgen receptor mRNA Expression by oligonucleotide compounds

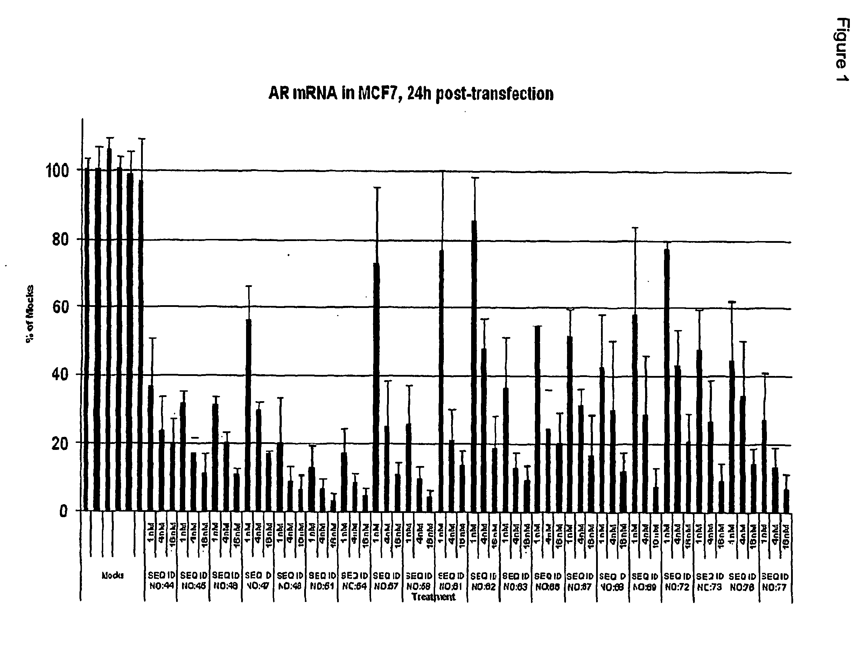

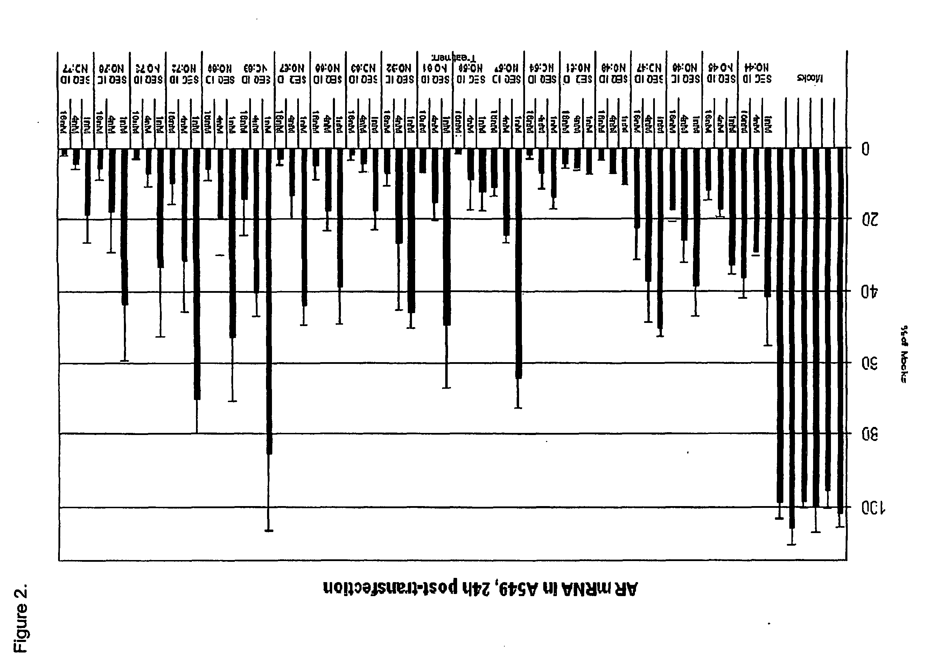

Oligonucleotides presented in Table 3 were evaluated for their potential to knockdown of the androgen receptor mRNA at concentrations of 1, 4 and 16 nM (see Figures 1 and 2).

**Table 3: Antisense Inhibition of Human Androgen receptor mRNA expression by oligonucleotides.**

| | | | |
|---|---|---|---|
| The data in Table 3 are presented as percentage down-regulation relative to mock transfected cells at 16 nM. Lower case letters represent DNA units, bold upper case letters represent β-D-oxy-LNA units. All LNA C are 5'methyl C. Subscript "s" represents phosphorothioate linkage. | | | |

| **Test substance** | **Sequence (5'-3')** | Percent inhibition of Androgen receptor-MCF7 | Percent inhibition of Androgen receptor-A549 |
|---|---|---|---|
| SEQ ID NO: 44 | 5'-**G·_{S}A·_{S}G·_{S}**a·_{S}a·_{S}c·_{S}c·_{S}a·_{S}t·_{S}c·_{S}c·_{S}t·_{S}c·_{S}**A·_{S}C·_{S}C**-3' | 80-1 | 63.8 |
| SEQ ID NO: 45 | 5'-**G·_{S}G·_{S}A·_{S}**c·_{S}c·_{S}a·_{S}g·_{S}g·_{S}t·_{S}a·_{S}g·_{S}c·_{S}c·_{S}**T·_{S}G·_{S}T**-3' | 89.0 | 88.2 |
| SEQ ID NO: 46 | 5'-**C·_{S}C·_{S}C·_{S}**c·_{S}t·_{S}g·_{S}g·_{S}a·_{S}c·_{S}t·_{S}c·_{S}a·_{S}g·_{S}**A·_{S}T·_{S}G**-3' | 89.4 | 82.8 |
| SEQ ID NO: 47 | 5'-**G·_{S}C·_{S}A·_{S}**c·_{S}a·_{S}a·_{S}g·_{S}g·_{S}a·_{S}g·_{S}t·_{S}g·_{S}g·_{S}**G·_{S}A·_{S}C**-3' | 83.1 | 77.7 |
| SEQ ID NO: 48 | 5'-**G·_{S}C·_{S}T·_{S}**g·_{S}t·_{S}g·_{S}a·_{S}a·_{S}g·_{S}a·_{S}g·_{S}a·_{S}g·_{S}**T·_{S}G·_{S}T**-3' | 93.8 | 96.7 |
| SEQ ID NO: 49 | 5'-**C·_{S}T·_{S}G·_{S}**t·_{S}g·_{S}a·_{S}a·_{S}g·_{S}a·_{S}g·_{S}a·_{S}g·_{S}**T·_{S}G**-3 | n.d. | n.d. |
| SEQ ID NO: 50 | 5'-**T·_{S}G·_{S}t·_{S}**g·_{S}a·_{S}a·_{S}g·_{S}a·_{S}g·_{S}a·_{S}**G·_{S}T·_{S}**-3' | n.d. | n.d. |
| SEQ ID NO: 51 | 5'-**T·_{S}T·_{S}T·**_{S}g·_{S}a·_{S}c·_{S}a·_{S}c·_{S}a·_{S}a·_{S}g·_{S}t·_{S}g·_{S}**G·_{S}sG·_{S}A**-3' | 96.9 | 95.5 |
| SEQ ID NO: 52 | 5'-**T·_{S}T·_{S}G·_{S}**a·_{S}c·_{S}a·_{S}c·_{S's}a·_{Ss}a·_{S}sg·_{Ss}t·_{Ss}g·_{S}**G·_{S}G**-3' | n.d. | n.d. |
| SEQ ID NO: 53 | **5'-T·_{S}G·_{S}**a·_{S}c·_{S}a·_{S}c·_{S}a·_{S}a·_{S}g·_{S}t·_{S}**G·_{S}G**-3' | n.d. | n.d. |
| SEQ ID NO: 54 | 5'-**G·_{S}T·_{S}G·_{S}**a·_{S}c·_{S}a·_{S}c·_{S}c·_{S}c·_{S}a·_{S}g·_{S}a·_{S}a·_{S}**G·_{S}C·_{S}T**-3' | 95.4 | 98.3 |
| SEQ ID NO: 55 | 5'-**T·_{S}G·_{S}A·_{S}**c·_{S}a·_{S}c·_{S}c·_{S}c·_{S}a·_{S}g·_{S}a·_{S}a·_{S}**G·_{S}C**-3' | n.d. | n.d. |
| SEQ ID NO: 56 | 5'- **G·_{S}A·_{S}**c·_{S}a·_{S}c·_{S}c·_{S}c·_{S}a·_{S}g·_{S}a·_{S}**A·_{S}G**-3' | n.d. | n.d. |
| SEQ ID NO: 57 | 5'-**C·_{S}A·_{S}T·_{S}**c·_{S}c·_{S}c·_{S}t·_{S}g·_{S}c·_{S}t·_{S}t·_{S}c·_{S}a·_{S}**T·_{S}A·_{S}A**-3' | 89.5 | 88.9 |
| SEQ ID NO: 58 | 5'-**A**·**_{S}C·_{S}C·_{S}**a·_{S}a·_{S}g·_{S}t·_{S}t·_{S}t·_{S}c·_{S}t·_{S}t·_{S}c·_{S}**A·_{S}G·_{S}C**-3' | 95.6 | 98.9 |
| SEQ ID NO: 59 | 5'-**C·_{S}C·_{S}A·_{S}**a·_{S}g·_{S}t·_{S}t·_{S}t·_{S}c·_{S}t·_{S}t·_{S}c·_{S}**A·_{S}G**-3' | n.d. | n.d. |
| SEQ ID NO: 60 | 5'-**C**·_{S}A·_{S}a·_{S}g·_{S}t·_{S}t·_{S}t·_{S}c·_{S}t·_{S}t·_{S}**C·_{S}A**-3' | n.d. | n.d. |
| SEQ ID NO: 61 | 5'-**C**·_{S}T·_{S}T·_{S}g·_{S}g·_{S}c·_{S}c·_{S}c·_{S}a·_{S}c·_{S}t·_{S}t·_{S}g·_{S}**A·_{S}C·_{S}C**-3' | 86.7 | 93.3 |
| SEQ ID NO: 62 | 5'-**T·_{S}C·_{S}C·_{S}**t·_{S}g·_{S}g·_{S}a·_{S}g·_{S}t·_{S}t·_{S}g·_{S}a·_{S}c·_{S}**A·_{S}T·_{S}T**-3' | 81.3 | 93.0 |
| SEQ ID NO: 63 | 5'-**C·_{S}A·_{S}C·_{S}**t·_{S}g·_{S}g·_{S}c·_{S}t·_{S}g·_{S}t·_{S}a·_{S}c·_{S}a·_{S}**T·_{S}C·_{S}C**-3' | 90.9 | 98.4 |
| SEQ ID NO: 64 | 5'-**A·_{S}C·_{S}T·_{S}**g·_{S}g·_{S}c·_{S}t·_{S}g·_{S}t·_{S}a·_{S}c·_{S}a·_{S}**T·_{S}C**-3' | n.d. | n.d. |
| SEQ ID NO: 65 | 5'-**C·_{S}T·_{S}**g·_{S}g·_{S}c·_{S}t·_{S}g·_{S}t·_{S}a·_{S}c·_{S}**A·_{S}T-**3' | n.d. | n.d. |
| SEQ ID NO: 66 | 5'-**C·_{S}A·_{S}T·_{S}**c·_{S}c·_{S}a·_{S}a·_{S}a·_{S}c·_{S}t·_{S}c·_{S}t·_{S}t·_{S}**G·_{S}A·_{S}G**-3' | 79.8 | 95.3 |
| SEQ ID NO_{:} 67 | 5'-**G·_{S}C·_{S}T·_{S}**t·_{S}t·_{S}c·_{S}a·_{S}t·_{S}g·_{S}c·_{S}a·_{S}**G·_{S}G·_{S}A**-3' | 83.5 | 97.0 |
| SEQ ID NO: 68 | 5'-**G**·_{S}A·_{S}A**·_{S}**g·_{S}t·_{S}t·_{S}c·_{S}a·_{S}t·_{S}c·_{S}a·_{S}a·_{S}a·_{S}**G·_{S}A·_{S}A**-3' | 88.2 | 85.6 |
| SEQ ID NO: 69 | 5'-**A**·_{S}**G·_{S}T·_{S}**t·_{S}c·_{S}c·_{S}t·_{S}t·_{S}g·_{S}a·_{S}t·_{S}g·_{S}t·_{S}**A·_{S}G·_{S}T**-3' | 92.7 | 94.0 |
| SEQ ID NO: 70 | 5'-**G·_{S}T·_{S}T·_{S}**c·_{S}c·_{S}t·_{S}t·_{S}g·_{S}a·_{S}t·_{S}g·_{S}**T·_{S}A·_{S}G**-3' | n.d. | n.d. |
| SEQ ID NO: 71 | 5'-**T·_{S}T·_{S}**c·_{S}c·_{S}t·_{S}t·_{S}g·_{S}a·_{S}t·_{S}g·_{S}**T·_{S}A**-3' | n.d. | n.d. |
| SEQ ID NO: 72 | 5'**-T·_{S}T·_{S}G·_{S}**c·_{S}a**·**_{S}c·_{S}a·_{S}g·_{S}a·_{S}g·_{S}a·_{S}t·_{S}g·_{S}**A·_{S}T·_{S}**c-3' | 79.2 | 90.4 |
| SEQ ID NO: 73 | 5'**-G·_{S}A·_{S}T·_{S}**g·_{S}g·_{S}g·_{S}c·_{S}t·_{S}t·_{S}g·_{S}a·_{S}c·_{S}t·_{S}**T**·_{S}**T**·_{S}**C**-3' | 91.1 | 97.3 |
| SEQ ID NO: 74 | 5'**-A·_{S}T·_{S}**g·_{S}g·_{S}g·_{S}c·_{S}t·_{S}t·_{S}g·_{S}a·_{S}c·_{S}t·_{S}**T**·_{S}**T**-3' | n.d. | n.d**.** |
| SEQ ID NO: 75 | 5'**-T·_{S}G·_{S}**g·_{S}g·_{S}c·_{S}t·_{S}t·_{S}g·_{S}a·_{S}c·_{S}**T·_{S}T-**3' | n.d. | n.d. |
| SEQ ID NO: 76 | 5'**-C·_{S}A·_{S}G·_{S}**g·_{S}c·_{S}a·_{S}g·_{S}a·_{S}a·_{S}g·_{S}a·_{S}c·_{S}a·_{S}**T·_{S}C·_{S}T-**3' | 85.9 | 94.3 |
| SEQ ID NO: 77 | 5'-**C·_{S}C·_{S}C·_{S}**a·_{S}a·_{S}g·_{S}g·_{S}c·_{S}a·_{S}c·_{S}t·_{S}g·_{S}c·_{S}**A·_{S}G·_{S}A**-3' | 93.0 | 98.5 |
| SEQ ID NO: 78 | 5'-**C·_{S}C·_{S}A·_{S}**a·_{S}g·_{S}g·_{S}c·_{S}a·_{S}c·_{S}t·_{S}g·_{S}c·_{S}**A·_{S}G**-3' | n.d. | n.d. |
| SEQ ID NO: 79 | 5'-**C·_{S}A·_{S}a**·**_{S}**g·_{S}g·_{S}c·_{S}a·_{S}c·_{S}t·_{S}g·_{S}**C·_{S}A**-3' | n.d. | n.d. |
| SEQ ID NO: 80 | 5'- **G_{S} C_{S} T_{S}** g_{S} a_{S} c_{S} a_{S} t_{S} t_{S} c_{S} a_{S} t_{S} a_{S} **G_{S}** C_{S} **C**-3' | n.d. | n.d. |

As shown in Table 3, oligonucleotides of SEQ ID NOs: 48, 51, 54, 58, 63, 69, 73 and 77 at 16 nM demonstrated greater than 90% inhibition of Androgen receptor mRNA expression in A549 and MCF7 cells in these experiments and are therefore preferred. Also preferred are oligonucleotides based on the illustrated antisense oligo sequences, for example varying the length (shorter or longer) and/or nucleobase content (e.g. the type and/or proportion of analogue units), which also provide good inhibition of Androgen receptor expression.

### Example 9: In vivo analysis: Antisense Inhibition of mouse Androgen receptor mRNA liver Expression by oligonucleotide compounds

Nude mice were dosed i.v. q3dx4 with 100 mg/kg oligonucleotide (group size of 5 mice). The antisense oligonucleotides (SEQ ID:48, SEQ ID:51, SEQ ID:58, SEQ ID:63, SEQ ID:77) were dissolved in 0.9% phosphate buffered saline. Animals were sacrificed 24h after last dosing and liver tissue was sampled and stored in RNA later until RNA extraction and QPCR analysis. Total RNA was extracted and AR mRNA expression in liver samples was measured by QPCR as described in example 7 using a mouse AR QPCR assay (cat. Mm01238475_m1, Applied Biosystems). Results were normalised to mouse GAPDH (cat. no. 4352339E, Applied Biosystems) and Knockdown was quantitated relative to saline treated controls. The data in Table 4 are presented as percentage down-regulation relative to saline treated animals.

| **Table 4** | |
|---|---|
| **In vivo knock-down of AR mRNA expression** | |
| Compound | Liver (% KD) |
| Saline | 0 |
| SEQ ID: 51 100 mg/kg | 65.0+/-12.6 |
| SEQ ID: 58 100 mg/kg | 95.2+/-1.0 |
| SEQ ID: 77 100 mg/kg | 91.9+/-3.9 |

As shown in Table 4, oligonucleotides of SEQ ID NOs: 58 and 77 at 100 mg/kg demonstrated greater than 90% inhibition of Androgen receptor mRNA expression in mouse liver cells in these experiments and are therefore preferred.

### Example 10: In vitro analysis: Antisense Inhibition of Human Androgen receptor mRNA

### Measurement of proliferating viable cells (MTS assay)

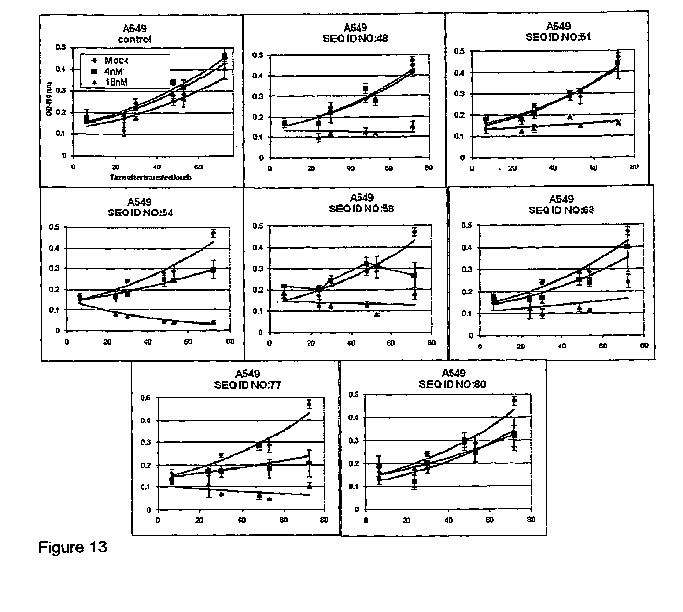

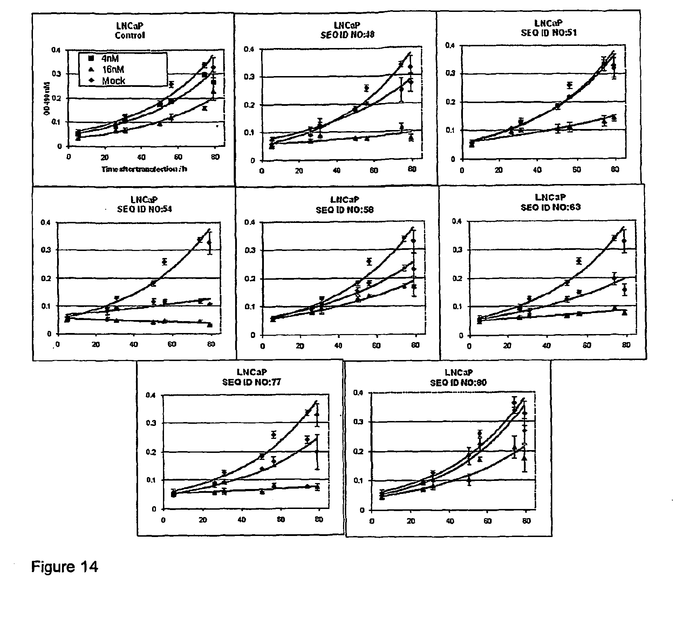

LNCaP prostate cancer and A549 lung cancer cells were seeded to a density of 150000 cells per well in a 6-well plate the day prior to transfection. A549 cells were cultured in DMEM (Sigma) +10% fetal bovine serum (FBS) + 2 mM Glutamax I + gentamicin (25µg/ml) wheras LNCaP cells were cultured in RPMI 1640 Medium (Sigma) + 10% fetal bovine serum (FBS) + 2 mM Glutamax I + gentamicin (25µg/ml). The next day medium was removed followed by addition of 1.2 ml OptiMEM containing 5 µg/ml Lipofectamine2000 (Invitrogen). Cells were incubated for 7 min before adding 0.3 ml oligonucleotides diluted in OptiMEM. The final oligonucleotide concentrations were 4 and 16 nM. After 4 hours of treatment, media was removed and cells were trypsinized and seeded to a density of 5000 cells per well in clear 96 well plate (Scientific Orange no. 1472030100) in 100 µl media. Viable cells were measured at the times indicated by adding 10 µl the tetrazolium compound [3-(4,5-dimethyl-2-yl)-5-(3-carboxymethoxyphenyl)-2-(4-sulfophenyl)-2H-tetrazolium, inner salt; MTS] and an electron coupling reagent (phenazine ethosulfate; PES) (CellTiter 96® AQueous One Solution Cell Proliferation Assay, Promega). Viable cells were measured at 490 nm in a Powerwave (Biotek Instruments). The OD490 nm were plotted against time/h. *(See* *Figure 13* *and* *Figure 14**).* As shown in figure 13 and figure 14, oligonucleotides of SEQ ID NOs: 58 and 77 inhibits growth of both LNCaP prostate and A549 lung cancer cells and are therefore preferred.

### Example 11: In vitro analysis: Caspase 3/7 activity by Antisense Inhibition of Human Androgen receptor mRNA

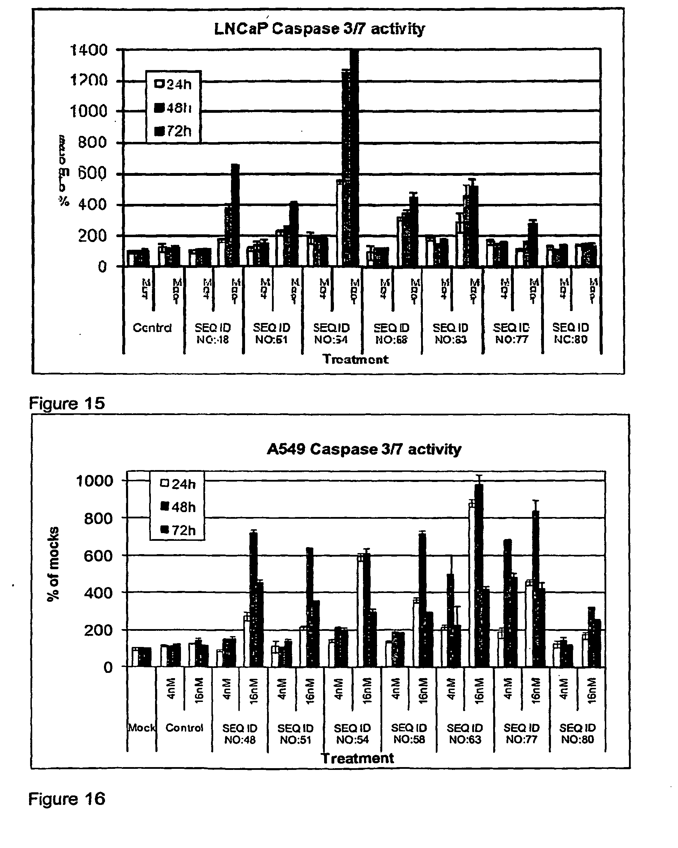

LNCaP prostate cancer and A549 lung cancer cells were seeded to a density of 150000 cells per well in a 6-well plate the day prior to transfection. A549 cells were cultured in DMEM (Sigma) + 10% fetal bovine serum (FBS) + 2 mM Glutamax I + gentamicin (25µg/ml) wheras LNCaP cells were cultured in RPMI 1640 Medium (Sigma) + 10% fetal bovine serum (FBS) + 2 mM Glutamax I + gentamicin (25µg/ml). The next day medium was removed followed by addition of 1.2 ml OptiMEM containing 5 µg/ml Lipofectamine2000 (Invitrogen). Cells were incubated for 7 min before adding 0.3 ml oligonucleotides diluted in OptiMEM. The final oligonucleotide concentrations were 4 and 16 nM. After 4 hours of treatment, media was removed and cells were trypsinized and seeded to a density of 5000 cells per well in white 96 well plate (Nunc) in 100 µl media. Caspase 3/7 activity was measured at the times indicated by adding 100 µl Caspase-Glo 3/7 assay (promega). Caspase 3/7 activity was measured using a luminometer. The the caspase 3/7 activities were measuered at three different time points 14h, 48h and 72h *(See* *Figure 15 and Figure 16**).* As shown in figure 15 and figure 16, oligonucleotides of SEQ ID NOs: 58 and 77 induce caspase 3/7 activity in both LNCaP prostate and A549 lung cancer cells and are therefore preferred.

### Example 12: In vitro analysis: Antisense Inhibition of Human Androgen receptor mRNA Expression by oligonucleotide compounds in prostate cancer cells LNCaP and the lung cancer cell line A549.

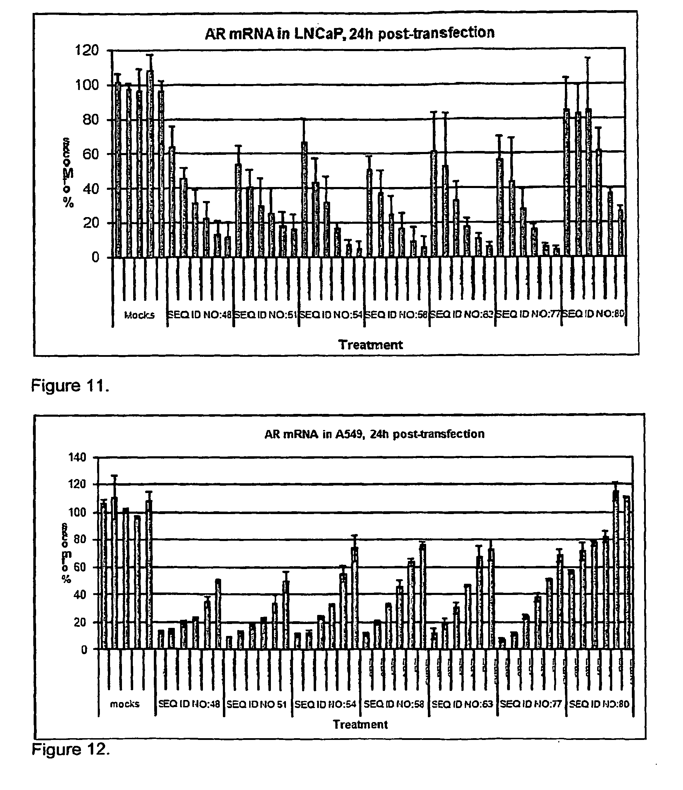

Oligonucleotides were evaluated for their potential to knockdown of the androgen receptor mRNA at concentrations of 0.5, 1, 2, 4, 8 and 16 nM (see Figure 11). LNCaP prostate cancer and A549 lung cancer cells were seeded to a density of 150000 cells per well in a 6-well plate the day prior to transfection. A549 cells were cultured in DMEM (Sigma) +10% fetal bovine serum (FBS) + 2 mM Glutamax I + gentamicin (25µg/ml) wheras LNCaP cells were cultured in RPMI 1640 Medium (Sigma) + 10% fetal bovine serum (FBS) + 2 mM Glutamax I + gentamicin (25µg/ml). The next day medium was removed followed by addition of 1.2 ml OptiMEM containing 5 µg/ml Lipofectamine2000 (Invitrogen). Cells were incubated for 7 min before adding 0.3 ml oligonucleotides diluted in OptiMEM. The final oligonucleotide concentrations were 0.5, 1, 2, 4, 8 and 16 nM. Cells were washed and serum-containing media was added. After oligo treatment cells were allowed to recover for 20 hours before they were harvested for RNA analysis. The procedure for RNA isolation, cDNA synthesis and qPCR were as descriped in example 5, 6 and 7. As shown in Figures 11 and 12 oligonucleotides of SEQ ID NOs: 58 and 77 were potent in knocking down AR mRNA expression in both the lung cancer cell line A549 and in the androgen receptor-dependent 22RV1 prostate cancer cell line.

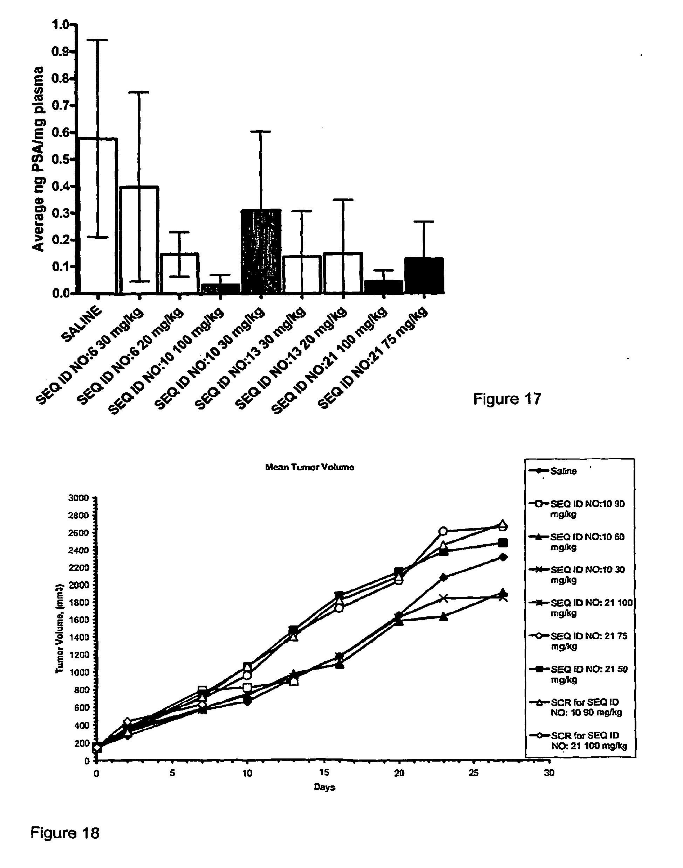

*Example 13: Effect of antisense oligonucleotide on PSA (**Figure 17**):* Six to seven week old male athymic nu/nu mice (Harlan Sprague Dawley) weighing an average of 27.3±2.4g were used in the study. Ten million cells of 22RV1 (androgen-independent prostate cancer line) were suspended in PBS (Gibco#14190) and Matrigel (BD#356234) with a ratio of 1:1 were injected subcutaneously into each mouse. When tumors reach an average volume of 150-200 mm³, the mice were divided into nine experimental groups. Two hundred µl of oligo was injected intravenously when the average tumor size reached 152.66±27.97 mm³. Oligos were given every 3 days for a total of 5 times. The control vehicles were given the same regime as the oligos. On day 16, mice were sacrificed and blood collected in EDTA laced tubes and spun for 5 min. 50 µl of the supernatants were then subjected to PSA assay using the ELISA kit from ALPCO Diagnostics in Salem (PSAHU-L01).

Effect of antisense oligonucleotide on tumor growth (Figure 18): Six to seven week old male athymic nu/nu mice (Harlan Sprague Dawley) weighing an average of 27.3±2.4g were used in the study. Ten million cells of 22RV1 (androgen-independent prostate cancer line) were suspended in PBS (Gibco#14190) and Matrigel (BD#356234) with a ratio of 1:1 were injected subcutaneously into each mouse. When tumors reach an average volume of 150-200 mm³, the mice were divided into nine experimental groups. Two hundred µl of oligo was injected intravenously when the average tumor size reached 152.66±27.97 mm³. Oligos were given every 3 days for a total of 5 times. The control vehicles were given the same regime as the oligos. The tumor volumes for each mouse were determined by measuring two dimensions with calipers and calculated using the formula: tumor volume = (length x width²)/2)_{.}

### Example 14: Preparation of conjugates of oligomers with polyethylene glycol

The oligomers having sequences shown as SEQ ID NO: 48 or SEQ ID NO: 63 are functionalized on the 5' terminus by attaching an aminoalkyl group, such as hexan-1-amine blocked with a blocking group such as Fmoc to the 5' phosphate groups of the oligomers using routine phosphoramidite chemistry, oxidizing the resultant compounds, deprotecting them and purifying them to achieve the functionalized oligomers, respectively, having the formulas (IA) and (IB): 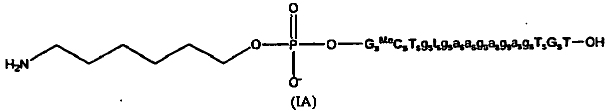 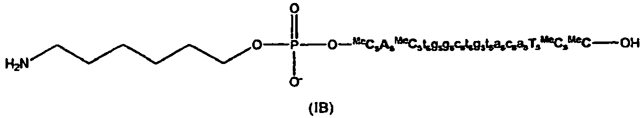 wherein the bold uppercase letters represent nucleoside analogue monomers, lowercase letters represent DNA monomers, the subscript "s" represents a phosphorothioate linkage, and ^{Me}C represents 5-methylcytosine.

A solution of activated PEG, such as the one shown in formula (II): 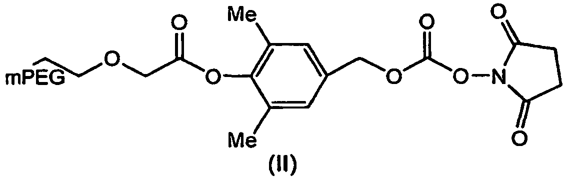 wherein the PEG moiety has an average molecular weight of 12,000, and each of the compounds of formulas (IA) and (IB) in PBS buffer are stirred in separate vessels at room temperature for 12 hours. The reaction solutions are extracted three times with methylene chloride and the combined organic layers are dried over magnesium sulphate and filtered and the solvent is evaporated under reduced pressure. The resulting residues are dissolved in double distilled water and loaded onto an anion exchange column. Unreacted PEG linker is eluted with water and the products are eluted with NH₄HCO₃ solution. Fractions containing pure products are pooled and lypophilized to yield the conjugates SEQ ID NOs: 48 and 63, respectively as show in formulas (IIIA) and (IIIB): 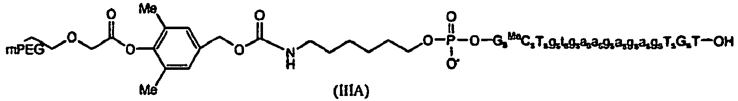 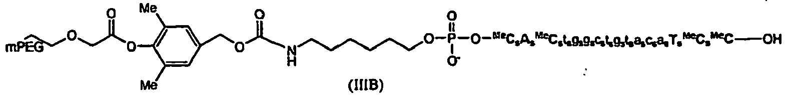 wherein each of the oligomers of SEQ ID NOs: 48 and 63 is attached to a PEG polymer having average molecular weight of 12,000 via a releasable linker.

Chemical structures of PEG polymer conjugates that can be made with oligomers having sequences shown in SEQ ID NOs: 51, 58 and 77 using the process described above are respectively shown in formulas (IVA), (IVB) and (IVC): 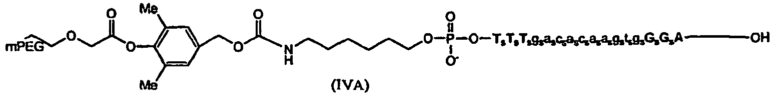 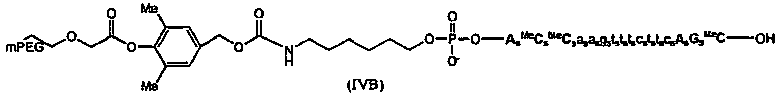 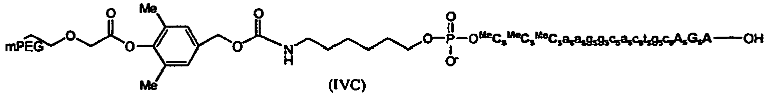 wherein bold uppercase letters represent beta-D-oxy-LNA monomers, lowercase letters represent DNA monomers, the subscript "s" represents a phosphorothioate linkage and ^{Me}C represent 5-methylcytosine.

Activated oligomers that can be used in this process to respectively make the conjugates shown in formulas (IVA), (IVB) and (IVC) have the chemical structures shown in formulas (VA), (VB) and (VC):

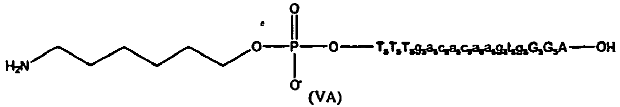

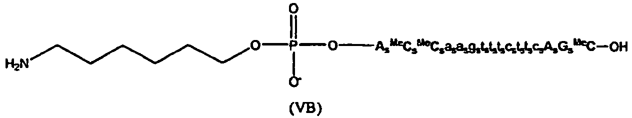

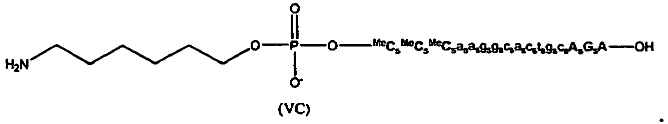

## Claims

1. An oligomer having a contiguous nucleotide sequence of a total of 10 - 30 nucleotides, wherein said contiguous nucleotide sequence is at least 80% homologous to the reverse complement of a corresponding region of a mammalian androgen receptor gene or mRNA, wherein said oligomer comprises at least one LNA unit; and wherein the contiguous nucleotide sequence comprises at least 10 contiguous nucleotides which are 100% identical to a corresponding region of SEQ ID NO 94 or SEQ ID NO 58.

2. The oligomer according to claim 1 wherein the contiguous nucleotide sequence comprises SEQ ID No 94 or SEQ ID No 58.

3. The oligomer according to claim 1 or 2, wherein the contiguous nucleotide sequence is 10 - 22 nucleotides in length.

4. The oligomer according to any one of claims 1 to 3, wherein the contiguous nucleotide sequence is 10 - 18 nucleotides in length.

5. The oligomer according to any one of claims 1 to 4, wherein the contiguous nucleotide sequence comprises other nucleotide analogues.

6. The oligomer according to claim 5, wherein the nucleotide analogues are sugar modified nucleotides, such as sugar modified nucleotides selected from the group consisting of: Locked Nucleic Acid (LNA) units; 2'-O-alkyl-RNA units, 2'-OMe-RNA units, 2'-amino-DNA units, and 2'-fluoro-DNA units.

7. The oligomer according to claim 5, wherein the nucleotide analogues are LNA.

8. The oligomer according to any one of claims 5 to 7 which is a gapmer.

9. The oligomer according to any one of claims 1 to 8, which is capable of down-regulating the expression of androgen receptor gene or mRNA in a cell which is expressing androgen receptor gene or mRNA.

10. The oligomer according to any one of claims 1 to 9, wherein the oligomer consists of
**5'-A·_{S}^{Me}C_{S}^{Me}C·_{S}a·_{S}a·_{S}g·_{S}t·_{S}t·_{S}t·_{S}c·_{S}t_{S·}t_{·S}c·_{S}A·_{S}G·_{S}^{Me}C-3' (SEQ ID NO: 58),**
wherein uppercase letters denote beta-D-oxy-LNA monomers and lowercase letters denote DNA monomers, the subscript "s" denotes a phosphorothioate linkage, and ^{Me}C denotes a beta-D-oxy-LNA monomer containing a 5-methylcytosine base.

11. A conjugate comprising the oligomer according to any one of claims 1 to 10, and at least one non-nucleotide or non-polynucleotide moiety covalently attached to said oligomer.

12. A pharmaceutical composition comprising the oligomer according to any one of claims 1 to 10, or the conjugate according to claim 11, and a pharmaceutically acceptable diluent, carrier, salt or adjuvant.

13. The oligomer according to any one of claims 1 to 10, or the conjugate according to claim 11, for use as a medicament, such as for the treatment of a disease or a medical disorder as disclosed herein, such as a hyperproliferative disorder, such as cancer.

14. The use of an oligomer according to any one of the claims 1 to 10, or a conjugate as defined in claim 11, for the manufacture of a medicament for the treatment of a disease or a medical disorder as disclosed herein, such as a hyperproliferative disorder, such as cancer.

15. An *in vitro* method for the inhibition of Androgen Receptor in a cell which is expressing the androgen receptor, said method comprising administering an oligomer according to any one of the claims 1 to 10, or a conjugate according to claim 11 to said cell so as to inhibit androgen receptor in said cell.

## Patentansprüche

1. Oligomer mit einer zusammenhängenden Nukleotidsequenz von insgesamt 10 - 30 Nukleotiden, wobei die zusammenhängende Nukleotidsequenz eine Homologie von wenigstens 80% zu dem reversen Komplement eines entsprechenden Bereichs eines Gens oder einer mRNA eines Säuger-Androgenrezeptors aufweist, wobei das Oligomer wenigstens eine LNA-Einheit umfasst; und wobei die zusammenhängende Nukleotidsequenz wenigstens 10 zusammenhängende Nukleotide umfasst, die mit einem entsprechenden Bereich der SEQ ID NO 94 oder SEQ ID NO 58 zu 100% identisch sind.

2. Oligomer nach Anspruch 1, wobei die zusammenhängende Nukleotidsequenz SEQ ID Nr. 94 oder SEQ ID Nr. 58 umfasst.

3. Oligomer nach Anspruch 1 oder 2, wobei die zusammenhängende Nukleotidsequenz eine Länge von 10 - 22 Nukleotiden aufweist.

4. Oligomer nach einem der Ansprüche 1 bis 3, wobei die zusammenhängende Nukleotidsequenz eine Länge von 10 - 18 Nukleotiden aufweist.

5. Oligomer nach einem der Ansprüche 1 bis 4, wobei die zusammenhängende Nukleotidsequenz andere Nukleotidanaloge umfasst.

6. Oligomer nach Anspruch 5, wobei es sich bei den Nukleotidanalogen um zucker-modifizierte Nukleotide handelt, wie etwa zucker-modifizierte Nukleotide, die aus der aus LNA(Locked Nucleic Acid)-Einheiten; 2'-O-Alkyl-RNA-Einheiten, 2'-OMe-RNA-Einheiten, 2'-Amino-DNA-Einheiten und 2'-Fluor-DNA-Einheiten bestehenden Gruppe ausgewählt sind.

7. Oligomer nach Anspruch 5, wobei es sich bei den Nukleotidanalogen um LNA handelt.

8. Oligomer nach einem der Ansprüche 5 bis 7, bei dem es sich um ein Gapmer handelt.

9. Oligomer nach einem der Ansprüche 1 bis 8, das zur Herunterregulation der Expression von Androgenrezeptor-Gen oder -mRNA in einer Zelle, die Androgenrezeptor-Gen bzw. -mRNA exprimiert, fähig ist.

10. Oligomer nach einem der Ansprüche 1 bis 9, wobei das Oligomer aus
**5'-A·_{S}^{Me}C·_{S}^{Me}C·_{S}a·_{S}a·_{S}t·_{S}t_{S}·t_{S}·c·_{S}t·_{S}t·_{S}c·_{S}A·_{S}G·_{S}^{Me}C-3' (SEQ ID NO:58),**
besteht, wobei Großbuchstaben Beta-D-oxy-LNA-Monomere und Kleinbuchstaben DNA-Monomere bezeichnen, das tiefgestellte "s" eine Phosphorothioat-Verknüpfung und ^{Me}C ein Beta-D-oxy-LNA-Monomer mit einer 5-Methylcytosin-Base bezeichnet.

11. Konjugat, umfassend das Oligomer nach einem der Ansprüche 1 bis 10 und wenigstens eine kovalent an das Oligomer gebundene Nicht-Nukleotid- oder Nicht-Polynukleotid-Gruppierung.

12. Pharmazeutische Zusammensetzung, umfassend das Oligomer nach einem der Ansprüche 1 bis 10 oder das Konjugat nach Anspruch 11 sowie ein pharmazeutisch unbedenkliches Verdünnungsmittel, Trägermittel, Salz oder Hilfsmittel.

13. Oligomer nach einem der Ansprüche 1 bis 10 oder Konjugat nach Anspruch 11 zur Verwendung als Arzneimittel, wie etwa zur Behandlung einer Krankheit oder einer medizinischen Störung, wie hier offenbart, wie etwa einer Hyperproliferationsstörung, wie etwa Krebs.

14. Verwendung eines Oligomers nach einem der Ansprüche 1 bis 10 oder eines Konjugats nach Anspruch 11 zur Herstellung eines Arzneimittels zur Behandlung einer Krankheit oder einer medizinischen Störung, wie hier offenbart, wie etwa einer Hyperproliferationsstörung, wie etwa Krebs.

15. In-vitro-Verfahren zur Hemmung von Androgenrezeptor in einer Zelle, die den Androgenrezeptor exprimiert, wobei man bei dem Verfahren der Zelle ein Oligomer nach einem der Ansprüche 1 bis 10 oder ein Konjugat nach Anspruch 11 verabreicht, um Androgenrezeptor in der Zelle zu hemmen.

## Revendications

1. Oligomère ayant une séquence nucléotidique contiguë d'un total de 10 à 30 nucléotides, dans lequel ladite séquence nucléotidique contiguë est au moins 80 % homologue au complément inverse d'une région correspondante d'un gène ou ARNm de récepteur d'androgène de mammifère, ledit oligomère comprenant au moins un motif de LNA ; et la séquence nucléotidique contiguë comprenant au moins 10 nucléotides contigus qui sont à 100 % identiques à une région correspondante de SEQ ID NO 94 ou SEQ ID NO 58.

2. Oligomère selon la revendication 1 dans lequel la séquence nucléotidique contiguë comprend SEQ ID NO 94 ou SEQ ID NO 58.

3. Oligomère selon la revendication 1 ou 2, dans lequel la séquence nucléotidique contiguë est de 10 à 22 nucléotides de longueur.

4. Oligomère selon l'une quelconque des revendications 1 à 3, dans lequel la séquence nucléotidique contiguë est de 10 à 18 nucléotides de longueur.

5. Oligomère selon l'une quelconque des revendications 1 à 4, dans lequel la séquence nucléotidique contiguë comprend d'autres analogues de nucléotide.

6. Oligomère selon la revendication 5, dans lequel les analogues de nucléotide sont des nucléotides à glucide modifié, tels que les nucléotides à glucide modifié choisis dans le groupe constitué de : motifs d'acide nucléique bloqué (LNA) ; motifs 2'-O-alkyl-ARN, motifs 2'-OMe-ARN, motifs 2'-amino-ADN, et motifs 2'-fluoro-ADN.

7. Oligomère selon la revendication 5, dans lequel les analogues de nucléotide sont des LNA.

8. Oligomère selon l'une quelconque des revendications 5 à 7 qui est un gapmère.

9. Oligomère selon l'une quelconque des revendications 1 à 8, qui est capable de réguler à la baisse l'expression d'un gène ou ARNm de récepteur d'androgène dans une cellule qui exprime un gène ou ARNm de récepteur d'androgène.

10. Oligomère selon l'une quelconque des revendications 1 à 9, l'oligomère étant constitué de
**5'-A·ₛ^{Me}C·ₛ^{Me}C·ₛa·ₛa·ₛg·ₛt·ₛt·ₛt·ₛc·ₛt·ₛt·ₛt·ₛcₛA·ₛG·ₛ^{Me}C-3' (SEQ ID NO:58),**
où les lettres en majuscules désignent des monomères bêta-D-oxy-LNA et les lettres en minuscules désignent des monomères d'ADN, l'indice « s » désigne un lieur phosphorothioate, et ^{Me}C désigne un monomère bêta-D-oxy-LNA contenant une base 5-méthylcytosine.

11. Conjugué comprenant l'oligomère selon l'une quelconque des revendications 1 à 10, et au moins un fragment non-nucléotide ou non-polynucléotide lié de façon covalente audit oligomère.

12. Composition pharmaceutique comprenant l'oligomère selon l'une quelconque des revendications 1 à 10, ou le conjugué selon la revendication 11, et un diluant, véhicule, sel ou adjuvant pharmaceutiquement acceptable.

13. Oligomère selon l'une quelconque des revendications 1 à 10, ou conjugué selon la revendication 11, pour utilisation en tant que médicament, par exemple pour le traitement d'une maladie ou un trouble médical tel que décrit dans la présente, comme un trouble hyperprolifératif, tel que le cancer.

14. Utilisation d'un oligomère selon l'une quelconque des revendications 1 à 10, ou d'un conjugué tel que défini dans la revendication 11, pour la fabrication d'un médicament pour le traitement d'une maladie ou un trouble médical tel que décrit dans la présente, comme un trouble hyperprolifératif, tel que le cancer.

15. Procédé *in vitro* pour l'inhibition de récepteur d'androgène dans une cellule qui exprime le récepteur d'androgène, ledit procédé comprenant l'administration d'un oligomère selon l'une quelconque des revendications 1 à 10, ou un conjugué selon la revendication 11, à ladite cellule de manière à inhiber le récepteur d'androgène dans ladite cellule.
